# EUROPEAN PATENT APPLICATION

(11) **EP 2 520 648 A1**
(43) Date of publication of application: **07.11.2012**
(21) Application number: 10841044.0
(22) Date of filing: 28.12.2010
(51) Int. Cl.: C12N 15/09, A61K 39/395, A61P 13/12, A61P 37/02, C07K 16/42, C07K 16/46, C12N 1/15, C12N 1/19, C12N 1/21, C12N 5/10, C12N 15/02, C12P 21/08, G01N 33/53

(54) **ANTI-IGA1 ANTIBODY**

(30) Priority: 28.12.2009 JP 2009296706
(71) Applicant: Kyowa Hakko Kirin Co., Ltd., Tokyo 100-8185 (JP)
(72) Inventor: KANEKO, Etsuji, Tokyo 194-8533 (JP); SASAKI, Yuka, Tokyo 100-8185 (JP); MORI, Katsuhiro, Tokyo 100-8185 (JP); KANDA, Yutaka, La Jolla, CA 92037 (US); SATOH, Mitsuo, Tokyo 100-8185 (JP)
(74) Representative: HOFFMANN EITLE
(86) International application number: PCT/JP2010/073737
(87) International publication number: WO 2011/081189

(57) **Abstract**

An object of the present invention is to provide a monoclonal antibody which is effective for diagnosing IgA nephropathy and specifically recognizes and binds to a hinge region of a polypeptide encoded by a heavy chain gene of immunoglobulin A1 that comprises a serine/threonine-linked sugar chain to which galactose is not bound. According to the present invention, it is possible to provide a monoclonal antibody or an antibody fragment thereof that specifically recognizes and binds to a hinge region of a polypeptide encoded by a heavy chain gene of immunoglobulin A1 that comprises a serine/threonine-linked sugar chain to which galactose is not bound, to provide a diagnostic agent using the antibody or the antibody fragment thereof, and to provide a therapeutic agent comprising the antibody or the antibody fragment thereof as an active ingredient.

## Description

### Field of the Invention

The present invention relates to a monoclonal antibody or an antibody fragment thereof, which specifically recognizes and binds to a hinge region of polypeptide encoded by a heavy chain gene of immunoglobulin A1 comprising an serine/threonine-linked sugar chain to which galactose is not bound; a hybridoma which produces the antibody; a DNA which encodes the antibody; a vector which comprises the DNA; a transformant obtainable by transformation of the vector; a process for producing an antibody or an antibody fragment thereof using the hybridoma or the transformant; a diagnostic agent using the antibody or the antibody fragment thereof, and a therapeutic agent comprising the antibody or the antibody fragment thereof as an active ingredient.

### Background Art

In recent years, there have been reported some cases in which the onset of various diseases or the progression of pathology is accompanied by structural changes in sugar chains attached to the protein which is expressed by cells involved in the disease or pathology thereof. Representative ones among these cases are an expression of Tn antigen (Thomsen antigen, C175 antigen) which is one of the O-linked (serine/threonine type) sugar chain antigens whose expression is found in more than 80% of human cancer types, and an expression of a sialyl Tn antigen (CD175s antigen) which mean the Tn antigen with addition of sialic acid (Non-Patent Literature 2). It is known that the expressions of these sugar chain antigens are hardly found in normal cells, and researches for applying them as target molecules of cancer-specific vaccine therapies to medical care has been carried out (Non-Patent Literature 1). The expression of these cancer-specific sugar chain antigens are regulated by the activity of enzymes constituting the complicated biosynthetic pathway of sugar chains and the complicated metabolic pathway of sugar chains in living organisms. For example, it is known that, in cancer cells, changes in the expression pattern of genes encoding the proteins responsible for the biosynthetic pathway of sugar chains leads to blockage of the biosynthetic pathway of sugar chains. The Tn antigen is known as an intermediate of the biosynthetic pathway of an O-linked sugar chain in normal cells, and has a structure (GalNAc α-Ser/Thr) in which N-acetylgalactosamine (GalNAc) is α-bound to a hydroxyl group on a side chain of a certain serine (Ser) or threonine (Thr) residue of an amino acid sequence of a protein. Biosynthesis of a normal-type O-linked sugar chain such as TF antigen (Thomsen-Friedenreich antigen, CD 176 antigen)takes place by the addition of one molecule of galactose to the non-reducing terminal of the Tn antigen by the activity of core 1β3 galactosyltransferase (core 1β3Gal-T, T-synthetase). It is considered that the biosynthetic pathway of sugar chains is blocked as a result of decrease in the activity of intracellular core 1β3 galactosyltransferase, and thereby the Tn antigen or the sialyl Tn antigen is expressed in many types of cancer cell lines. The mechanism of the decrease in the activity of core 1β3 galactosyltransferase in cancer cells is complicated and has not yet been fully elucidated. However, as one possible mechanism, it has been supposed that the intracellular core 1β3 galactosyltransferase activity is greatly decreased due to a mutation in a gene encoding a certain chaperone protein (Cosmc) which is necessary for the activity expression of core 1β3 galactosyltransferase (Non-Patent Literature 6). Based on the fact that expression of the Tn antigen is commonly found among plural cancer types, it is considered that aberration in the biosynthetic pathway of sugar chains or the metabolic pathway of sugar chains in cells is a main cause of common changes in structures of sugar chains attached to many different glycoproteins expressed in the cells.

Cancer is a representative disease which is known to have a close relationship between the structural change of a sugar chain and the progression of pathology. Other than cancer, IgA nephropathy is known as another disease which is known to have a close linkage between the structural change of sugar chains and the pathological progression. IgA nephropathy is chronic glomerular nephritis which is pathologically characterized by showing granular deposition of one of the immune globulin, immunoglobulin A (IgA), in the glomerular mesangium, and was first reported by Berger in 1968 (Non-Patent Literature 2). This disease is representative nephritis accounting for about half of chronic glomerular nephritis patients in Japan. It is said that about 40% of patients who have been diagnosed with IgA nephropathy will undergo a transition of the disease to late-stage renal failure within 20 years, and who will inevitably receive hemodialysis or renal transplantation. As described above, even though IgA nephropathy has been generally recognized as a poor-prognosis disease, a clinically-validated therapy has not yet been established. There is known that IgA1, out of two different IgA isotypes (IgA1 and IgA2), is mainly deposited in the kidney in the bodies of patients with IgA nephropathy. In addition, as a cause of IgA1 deposition, it has been reported that a structure of an O-linked sugar chain attached to a hinge region specifically present on the human IgA1 molecule changed from a normal type to a Tn or sialyl Tn antigen (Non-Patent Literatures 3 and 4). It was demonstrated that once the deficiency of galactose from a O-linked type sugar chain added to the IgA1 hinge region has resulted in conversion of the sugar chain into a Tn or sialyl Tn antigen, self-agglutination ability of the IgA1 molecule is enhanced, immuno complex is formed by binding to an autoantibody which specifically recognizes this sugar chain-deficient IgA1, and the IgA1 moleculs which are formed into aggregate or immuno complex circumvent normal clearance mechanism in circulating blood and deposition of the IgA1 molecule into the renal mesangial areas occurs (Non-Patent Literature 5). Further, a decline of the core 1β3 galactosyltransferase activity due to a decreased expression level of Cosmc has been reported in IgA-producing cells isolated from IgA nephropathy patients (Non-Patent Literature 6). In other words, the biosynthetic pathway of sugar chains is blocked halfway through in IgA-producing cells in the bodies of IgA nephropathy patients and as a result, sugar chain-deficient IgA 1 is produced instead of IgA1 having a normal type sugar chain. As one of the pathogenic mechanisms of IgA nephropathy, it is advocated that the inflammation in renal tissue is induced as a result of the deposition of complex comprising this sugar chain-deficient IgA1 in the renal glomerulus.

Generally, IgA is produced by B cells in blood or tissue, or plasma cells (PCs) differentiated from B cells. The plasma cell is the final stage of B-cell differentiation. The plasma cells are distributed in secondary lymphoid tissues, systemic mucosal tissues, bone marrow, etc., and produce large quantities of antibodies. It is known that IgA-producing plasma cells are distributed mainly in mucosal tissues. On the other hand, it is known that, in the germinal center of secondary lymphoid tissues, memory B cells or plasma cells are differentiated from B cell clones which have acquired an ability to produce high-affinity IgA antibodies, and the thus differentiated cells are distributed throughout target organs in whole-body and continuously produce antibodies over an extended period of time. However, it is unclear at which stage of the B cell differentiation process, the cells which produce the sugar chain-deficient IgA involved in the pathogenesis of IgA nephropathy are developed, and to which body tissues the B cells or plasma cells which produce the sugar chain-deficient IgA are distributed.

It is known that about half of patients with IgA nephropathy show an increased level of IgA in blood (Non-Patent Literature 7). It is also known that sugar chain-deficient IgA1 which is generally rarely observed in healthy people is detected in body fluid such as peripheral blood and urine as well as renal glomeruli of patients with IgA nephropathy, which is a phenomenon commonly found in IgA nephropathy patients (Non-Patent Literature 8). As described above, this sugar chain-deficient IgA1, galactose-deficient IgA1, is a factor contributing to the progress of pathological conditions of IgA nephropathy. Moreover, in recent years, it has been proved that emergence and renal accumulation of the sugar chain-deficient IgA 1 also induces renal malfunction in several human diseases other than IgA nephropathy, for example, in Henoch-Schonlein purpura as an allergic disease, systemic lupus erythematosus as an autoimmune disease, or in a portion of IgA1-type myelomas as cancer (Non-Patent Literature 8). Due to this background, the sugar chain-deficient IgA1 is being recognized as a biomarker in specific human diseases including IgA nephropathy, such as a diagnostic biomarker, a predictive biomarker, or a pharmacodynamic biomarker.

It is difficult to strictly define a hinge region of human IgA1 by an amino acid sequence number of an IgA1 heavy chain polypeptide. In general, it means a region positioned between a CH1 domain and a CH2 domain in a heavy chain polypeptide constituting an IgA1 molecule. In a heavy chain polypeptide (SEQ ID NO:2) constituting a common human secretory IgA1 molecule, the hinge region frequently means a region from proline at position 223 to serine at position 240 or to cysteine at position 241 from an N-terminal. This region is also called an IgA1 hinge region core peptide. By the previous researches, amino acid residues to which an O-linked sugar chain is attached were identified in this region, and as the residues, 5 positions including threonine at position 225, threonine at position 228, serine at position 230, serine at position 232, and threonine at position 236 are known. In addition, it is known that an N-linked sugar chain does not attach to the hinge region but attaches to asparagine at position 263 and asparagine at position 459 in a heavy chain polypeptide constituting the IgA1 molecule.

Biopsy, a current definitive diagnosis method of IgA nephropathy, causes patients mental suffering, a risk of perinephric hemorrhage, and a financial burden due to hospitalization for several days. Several experimental techniques for directly detecting and analyzing sugar chain-deficient IgA1 have been considered. Immunological techniques such as ELISA or Western blotting using lectin recognizing and binding to a Tn type sugar chain or sialyl Tn type sugar chain, such as Vicia villosa-derived lectin, Vatairea macrocarpa-derived lectin, soy-derived lectin, Helix aspersa-derived lectin, Caragana arborescens-derived lectin, or the like is one of the simple techniques (Non-Patent Literature 9). However, since these lectins recognize and bind to only sugar chain structures, there is a problem that the lectin also nonselectively binds to glycoproteins (mucins, complement C1 inhibitor, and the like) that are contained in a sample such as a human-derived sample and have an O-linked sugar chain, other than IgA1. Although an immunological technique such as ELISA or Western blotting using an anti-Tn monoclonal antibody, such as MLS128, 22-1-1, HBTn1, or Bric111 is also one of simple techniques, low specificity of this technique is a problem similarly to the above method using lectin (Non-Patent Literature 10). On the other hand, a technique in which a mixture of sugar chains and glycopeptides obtained by treating IgA1, which is purified and extracted from a sample such as a human-derived sample, with various enzymes such as glycanase or peptidase is analyzed using matrix-assisted laser desorption/ionization-time of flight mass spectrometry (MALDI-TOF MS) or the like, has an advantage in relatively high specificity and detection sensitivity (Non-Patent Literature 11). However, this technique is not necessarily simple due to requiring steps of protein purification, enzymatic treatment, and instrumental analysis, and has problem of poor quantitativeness. In this way, a technique capable of detecting or measuring sugar chain-deficient IgA1 that is contained in a sample such as a human-derived sample in a specific, simple, and quantitative manner is not yet known. In addition, though it is considered that cells producing the sugar chain-deficient IgA1 express or accumulate the sugar chain-deficient IgA1 inside the cells or on the cell surface, a technique capable of specifically and simply detecting such cells is not yet known.

In the method according to Hiki et al. (Patent Literature 1), first, an ELISA plate is prepared in which normal IgA1 is captured on an ELISA plate where jacalin as a plant lectin which recognizes a TF antigen (Thomsen-Friedenreich antigen, a CD 176 antigen) as a normal O-linked sugar chain is immobilized. Subsequently, IgA1 is purified from a patient-derived sample, and the IgA1 is labeled with biotin or the like and added to the ELISA plate. In this manner, sugar chain-deficient IgA1 is bound onto the plate by a self-agglutination reaction with the normal IgA1 previously captured on the plate. A problem of this method is poor quantitativeness, since correlation between the degree of sugar chain deficiency in the IgA1 hinge region and the intensity of self-agglutination is unclear, and since the influence of the denaturation caused by labeling of patient-derived IgA1 on the agglutination properties cannot be excluded. Furthermore, since it is necessary to purifying IgA1 from a sample, this method also has a problem in terms of simplicity.

The method according to Narita et al. (Patent Literature 2) is a simpler method compared to the above method, since it is not necessary to purify and isolate the patient-derived IgA1. In this method, an ELISA plate where SAP which is a Streptococcus-derived IgA-bound peptide is immobilized is prepared, and a patient-derived sample is added thereto, thereby causing IgA to be captured on the plate. Subsequently, an antibody labeled with plant lectin (Vicia Villosa B4 lectin; VVL) which recognizes N-acetylgalactosamine is added thereto, thereby detecting sugar chain-deficient IgA1. However, VVL binds not only to N-acetylgalactosamine α-binding to serine/threonine but also to N-acetylgalactosamine β-binding to a non-reducing terminal of galactose included in an N-linked sugar chain. Accordingly, the method of Narita et al. is not a method that specifically detects structural change of the O-linked sugar chain in the IgA1 hinge region. Another method according to Hiki et al. (Patent Literature 3) is a method in which a patient-derived serum is passed through a column filled with jacalin agarose so as to isolate IgA1, the IgA1 is immobilized on an ELISA plate, and subsequently a rabbit-derived polyclonal antibody against a synthetic peptide (PVPSTPPTPSPSTPPTPSPS) having an amino acid sequence of the IgA1 hinge region is added to the ELISA plate, thereby finally detecting a labeled anti-rabbit IgG antibody. This method is a method of detecting IgA1 which is contained in the patient-derived serum and completely deficient in the O-linked sugar chain of the hinge region. This method is not a method specifically detecting the sugar chain-deficient IgA1 having a Tn-type sugar chain in the hinge region. Moreover, since jacalin, a lectin specific to a TF antigen for purifying IgA1, is used for this method, the sugar chain-deficient IgA1 contained in the patient's serum is incompletely recovered.

Attempts to diagnose IgA nephropathy by analyzing a patient-derived sample using the ELISA method have also been considered, though this is not a method of directly detecting the sugar chain-deficient IgA1. The method disclosed in Japanese Patent No. 4197393 is a method in which an ELISA plate is prepared where a protein prepared by conjugating a synthetic peptide (PVPSTPPTPSPSTPPTPSPSC) having an amino acid sequence of the IgA1 hinge region to a bovine serum albumin is immobilized, and subsequently a patient-derived sample is added to the plate. In this method, an autoantibody (IgG type) specifically binding to the IgA 1 hinge region is captured on the plate, and a labeled anti-human IgG antibody is finally added thereto, whereby the autoantibody can be detected. However, this method detects not just the IgA1 having the Tn-type sugar chain in the hinge region but just the IgG-type autoantibody binding to the IgA1 completely deficient in the O-linked sugar chain of the hinge region.

As an antibody which specifically recognizes IgA1, a B3506B4 antibody obtained by immunizing mice with human IgA1 heavy chain protein or 3C10 antibody obtained by immunizing mice with human milk-derived IgA1 (Non-Patent Document 12) and the like has been reported.
The B3506B4 antibody and 3C10 antibody are also shown to have an affinity with normal sugar chain IgA1. Up to date, an antibody which specifically recognizes IgA1 molecule comprising an O-linked sugar chain to which galactose is not bound has not been known.

It is generally known that, when a non-human antibody such as a mouse antibody is administered to human, it is recognized as a foreign substance so that a human antibody for mouse antibody [human anti mouse antibody (HAMA)] is induced in the human body. It is known that HAMA reacts with the administered mouse antibody to thereby induce side effects (Non-patent Documents 13 to 1516), enhances disappearance of the mouse antibody from the body (Non-patent Documents 17 to 19) and decreases therapeutic effect of the mouse antibody (Non-patent Documents 20 and 21).

In order to solve these problems, attempts have been made to prepare a human chimeric antibody or a humanized antibody from a non-human antibody using gene recombination techniques.
A humanized antibody has various advantages in administration to human in comparison with a non-human antibody such as a mouse antibody. For example, it has been reported that the immunogenicity was decreased and the blood half-life was prolonged in a test using monkey, in comparison with a mouse antibody (Non-patent Documents 22 and 23). That is, the humanized antibody is expected to cause fewer side effects in human than non-human antibodies and have sustained therapeutic effect for a long time.

Also, since a humanized antibody is prepared using gene recombination techniques, it can be prepared as various forms of molecules. For example, when γ1 subclass is used as a heavy chain (hereinafter referred to as "H chain") constant region (hereinafter referred to as "C region") of a human antibody (H chain C region is referred to as "CH"), a humanized antibody having high effector functions such as antibody-dependent cellular cytotoxicity (hereinafter referred to as "ADCC activity") can be prepared (Non-patent Document 24), and prolongation of the blood half life in comparison with mouse antibodies can be expected (Non-patent Document 25). Particularly, in the case of treatment for removing Tn antigen-type IgA1-producing cells, which express Tn antigen-type IgA1 on its cell surface, from human body, cytotoxic activities such as complement-dependent cytotoxicity (hereinafter referred to as "CDC activity") and ADCC activity via the Fc region (the region after the antibody heavy chain hinge region) of an antibody are important, in order to specifically damage the target cells by accumulating effector cells near a tumor tissue via the antibody. In the treatment of humans, a human chimeric antibody, a humanized antibody or a human antibody is preferably used for exhibiting the cytotoxic activities (Non-patent Documents 26 and 27).

In addition, with recent advance in protein engineering and genetic engineering, the humanized antibody can also be prepared as an antibody fragment having small molecular weight, such as Fab, Fab', F(ab')₂, a single chain antibody (hereinafter referred to as "scFv") (Non-patent Document 28), a dimerized V region fragment (hereinafter referred to as "Diabody") (Non-patent Document 29), a disulfide stabilized V region fragment (hereinafter referred to as "dsFv") (Non-patent Document 30), or a peptide comprising a complementarity determining region (hereinafter referred to as "CDR") (Non-patent Document 31), and these antibody fragments are more excellent in transitivity to target tissues than complete antibody molecules (Non-patent Document 32).

### Citation List

### Patent Literature

[Patent Literature 1] JP-A-9-311132
[Patent Literature 2] JP-A-2007-24661
[Patent Literature 3] JP-A-10-111290

### Non Patent Literature

[Non Patent Literature 1] Crit Rev Oncog., 6, 57 (1995)
[Non Patent Literature 2] J Urol Nephrol., 74, 694 (1968)
[Non Patent Literature 3] Clin Exp Immunol., 100, 470 (1995)
[Non Patent Literature 4] J Am Soc Neph., 7, 955 (1996)
[Non Patent Literature 5] Nephrol Dial Transplant., 17, 50 (2002)
[Non Patent Literature 6] J Intern Med., 258, 467 (2005)
[Non Patent Literature 7] Journal of Japanese Society of Nephrology 44(7), 514-523(2002)
[Non Patent Literature 8] Seminars in Nephrology 28(1), 78 (2008)
[Non Patent Literature 9] JBC 282, 28256, 2007; Kidney Int. 1997 52:509
[Non Patent Literature 10] Chem Bio Chem 6, 22292005
[Non Patent Literature 11] Carbohydrate Research 339(13), 2329-2355 (2004)
[Non Patent Literature 12] Clinical & Experimental Immunology 79(1), 35-40(1990)
[Non Patent Literature 13] Hum. Pathol., 38, 564 (2007)
[Non Patent Literature 14] Hum. Pathol., 36, 886 (2005)
[Non Patent Literature 15] FEBS Lett., 579, 6179 (2005)
[Non Patent Literature 16] Cancer Res., 65, 7378 (2005)
[Non Patent Literature 17] Hum. Pathol., 36, 886 (2005)
[Non Patent Literature 18] Oncogene, 13, 2328 (2006)
[Non Patent LiteratureL 19] Virchows Arch., 448, 52 (2006)
[Non Patent Literature 20] J. Immunol., 135, 1530 (1985)
[Non Patent Literature 21] Cancer Res., 46, 6489 (1986)
[Non Patent Literature 22] Cancer Res., 56, 1118 (1996)
[Non Patent LiteratureL 23] Immunol., 85, 668 (1995)
[Non Patent Literature 24] Cancer Res., 56, 1118 (1996)
[Non Patent Literature 25] Immunol., 85, 668 (1995)
[Non Patent Literature 26] J. Immunol., 144, 1382 (1990)
[Non Patent Literature 27] Nature, 322, 323 (1988)
[Non Patent Literature 28] Science, 242, 423 (1988)
[Non Patent Literature 29] Nature BiotechnoL, 15, 629 (1997)
[Non Patent Literature 30] Molecular Immunol., 32, 249 (1995)
[Non Patent Literature 31] J. Biol. Chem., 271, 2966 (1996)
[Non Patent LiteratureL 32] Cancer Res., 52, 3402(1992)

### Summary of the Invention

An object of the present invention is to provide a monoclonal antibody which specifically recognizes and binds to IgA1 comprising an O-linked sugar chain to which galactose is not bound, or a method of using the same.
There is a need for a monoclonal antibody which specifically recognizes and binds to IgA1 comprising an O-linked sugar chain to which galactose is not bound, or a method for using the same.

### Solution to Problem

The present invention relates to (1) to (32).
(1) A monoclonal antibody or an antibody fragment thereof which specifically recognizes and binds to a hinge region of a polypeptide encoded by a heavy chain gene of immunoglobulin A1 (hereinbelow, referred to as an IgA1 heavy chain) comprising a serine/threonine-linked sugar chain (hereinbelow, referred to as an O-linked sugar chain) to which galactose is not bound.
(2) A monoclonal antibody or an antibody fragment thereof which does not recognize a hinge region of an IgA1 heavy chain comprising an O-linked sugar chain to which galactose is bound, but recognizes and binds to a hinge region of an IgA1 heavy chain comprising the O-linked sugar chain to which galactose is not bound, among hinge regions of a polypeptide encoded by an IgA1 heavy chain gene to which the O-linked sugar chain is bound.
(3) The monoclonal antibody or the antibody fragment thereof according to (1) or (2), wherein the O-linked sugar chain to which galactose is not bound is at least one O-linked sugar chain selected from α-N-acetylgalactosamine-serine/threonine (hereinbelow, referred to as a Tn antigen) and a sialyl Tn antigen.
(4) The monoclonal antibody or the antibody fragment thereof according to (3), wherein the O-linked sugar chain to which galactose is not bound is the Tn antigen.
(5) The monoclonal antibody or the antibody fragment thereof according to any one of (1) to (4), wherein the monoclonal antibody is an antibody which specifically recognizes and binds to the hinge region of the IgA1 heavy chain comprising the amino acid sequence represented by SEQ ID NO:1.
(6) The monoclonal antibody or the antibody fragment thereof according to any one of (1) to (4), wherein the monoclonal antibody is an antibody which specifically recognizes and binds to a polypeptide which is a hinge region polypeptide of the IgA1 heavy chain comprising the amino acid sequence represented by SEQ ID NO:1 and is a glycopeptide to which N-acetylgalactosamine not having galactose is bound at least one amino acid residue selected from threonine at position 3, threonine at position 6, serine at position 8, serine at position 10, and threonine at position 14 from an amino terminal of the polypeptide.
(7) The monoclonal antibody or the antibody fragment thereof according to any one of (1) to (4), wherein the monoclonal antibody is an antibody which does not show cross-reactivity to a complement Cl inhibitor comprising the O-linked sugar chain to which galactose is not bound.
(8) The monoclonal antibody or the antibody fragment thereof according to any one of (1) to (4), wherein the monoclonal antibody is an antibody which competes with at least one monoclonal antibody selected from monoclonal antibodies KM4137, KM4140, and KM4144 when binding to the hinge region of the IgA1 heavy chain comprising the O-linked sugar chain to which galactose is not bound.
(9) The monoclonal antibody or the antibody fragment thereof according to any one of Claims (1) to (4), wherein the monoclonal antibody is an antibody which binds to an epitope to which at least one monoclonal antibody selected from monoclonal antibodies binds and which presents in the hinge region of the IgA 1 heavy chain comprising the O-linked sugar chain to which galactose is not bound.
(10) The antibody or the antibody fragment thereof according to any one of Claims (1) to (9), wherein the monoclonal antibody is an antibody which is produced from at least one hybridoma selected from hybridomas KM4137 (FERM BP-11214), KM4140 (FERM BP-11215), and KM4144 (FERM BP-11216).
(11) The antibody or the antibody fragment thereof according to any one of (1) to (9), wherein the monoclonal antibody is a recombinant antibody.
(12) The recombinant antibody or an antibody fragment thereof according to (11), wherein the recombinant antibody is an antibody selected from a human chimeric antibody, a humanized antibody, and a human antibody.
(13) The antibody fragment according to any one of (1) to (12), which is selected from Fab, Fab', F(ab')₂, a single chain antibody (scFv), a dimerized V region (diabody), a disulfide stabilized V region (dsFv), and a CDR-containing peptide.
(14) A hybridoma producing the monoclonal antibody according to any one of (1) to (9).
(15) A DNA encoding the antibody or the antibody fragment thereof according to any one of (1) to (13).
(16) A recombinant vector comprising the DNA according to (15).
(17) A transformant obtained by introducing the recombinant vector according to (16) into a host cell.
(18) A method of producing the antibody or the antibody fragment thereof according to any one of (1) to (13), the method comprising:
   culturing the hybridoma according to (14) or the transformant according to (17) in a medium so as to form and accumulate the antibody or the antibody fragment thereof according to any one of (1) to (13) in the culture; and
   collecting the antibody or the antibody fragment thereof from the culture.
(19) A method of immunologically detecting or measuring IgA1 having a hinge region comprising an O-linked sugar chain to which galactose is not bound, which comprises using the antibody or the antibody fragment thereof according to any one of (1) to (13).
(20) A reagent for detecting IgA 1 having a hinge region comprising an O-linked sugar chain to which galactose is not bound, which is a reagent using the antibody or the antibody fragment thereof according to any one of (1) to (13).
(21) A diagnostic agent for a disease relating to IgA1 having a hinge region comprising an O-linked sugar chain to which galactose is not bound, wherein the diagnostic agent uses the antibody or the antibody fragment thereof according to any one of (1) to (13).
(22) The diagnostic agent according to (21), wherein the disease relating to the IgA1 having the hinge region comprising the O-linked sugar chain to which galactose is not bound is an autoimmune disease.
(23) The diagnostic agent according to (21), wherein the disease relating to the IgA1 having the hinge region comprising the O-linked sugar chain to which galactose is not bound is IgA nephropathy.
(24) A therapeutic agent for a disease relating to IgA1 having a hinge region comprising an O-linked sugar chain to which galactose is not bound, wherein the therapeutic agent contains the antibody or the antibody fragment thereof according to any one of (1) to (13) as an active ingredient.
(25) The therapeutic agent according to (24), wherein the disease relating to the IgA1 having the hinge region comprising the O-linked sugar chain to which galactose is not bound is an autoimmune disease.
(26) The therapeutic agent according to (24), wherein the disease relating to the IgA1 having the hinge region comprising the O-linked sugar chain to which galactose is not bound is IgA nephropathy.
(27) A diagnostic method for a disease relating to IgA1 having a hinge region comprising an O-linked sugar chain to which galactose is not bound, the method comprising:
   detecting and measuring the IgA1 having the hinge region comprising the O-linked sugar chain to which galactose is not bound, by using the antibody or the antibody fragment thereof according to any one of (1) to (13).
(28) The diagnostic method according to (27), wherein the disease relating to IgA1 having the hinge region comprising the O-linked sugar chain to which galactose is not bound is an autoimmune disease.
(29) The diagnostic method according to (27), wherein the disease relating to IgA1 having the hinge region comprising the O-linked sugar chain to which galactose is not bound is IgA nephropathy.
(30) Use of the antibody or the antibody fragment thereof according to any one of (1) to (13) for producing a therapeutic agent for a disease relating to IgA1 having a hinge region comprising an O-linked sugar chain to which galactose is not bound.
(31) The use of the antibody or the antibody fragment thereof according to (30), wherein the disease relating to IgA1 having the hinge region comprising the O-linked sugar chain to which galactose is not bound is an autoimmune disease.
(32) The use of the antibody or the antibody fragment thereof according to (30), wherein the disease relating to IgA1 having the hinge region comprising the O-linked sugar chain to which galactose is not bound is IgA nephropathy.

### Advantageous Effects of Invention

According to the present invention, it is possible to provide a monoclonal antibody which specifically recognizes and binds to a hinge region of a polypeptide encoded by a heavy chain gene of immunoglobulin A1 comprising an O-linked sugar chain to which galactose is not bound. According to the present invention, it is also possible to provide a therapeutic agent or a diagnostic agent of various diseases relating to a hinge region of a polypeptide encoded by a heavy chain gene of immunoglobulin A1 comprising an O-linked sugar chain to which galactose is not bound.

### Brief Description of Drawings

Fig. 1 shows a construction flow of a plasmid pCR2B8PVH.
Fig. 2 shows a construction flow of a plasmid pCRIgA.
Fig. 3 shows a construction flow of a plasmid pCRmIgA.
Fig. 4 shows a construction flow of a plasmid pCR2B8PmIgA.
Fig. 5 shows a construction flow of a plasmid pKAN932B8PVHmIgA.
Fig. 6 shows SDS polyacrylamide electrophoresis of mIgA1-Fc.
Fig. 7 shows an O-linked sugar chain structure of mIgA1-Fc analyzed by an ELISA method. The ordinate indicates an average fluorescence intensity (OD415-OD490) at a sample wavelength of 415 nm and at a reference wavelength of 490 nm, and the values in the explanatory note indicate a concentration (µg/ml) of competitive substances.
Fig. 8 shows binding specificity of an established monoclonal antibody analyzed by an ELISA method. The ordinate is the absorbance in ELISA, which indicates binding properties of the monoclonal antibody to the respective immobilized antigen shown in the margin.
Fig. 9 shows binding specificity of the established monoclonal antibody analyzed by a competitive ELISA method. In the upper portion, a Tn antigen type human IgA1 is a competitive substance, and in the lower portion, human plasma-derived IgA1 is a competitive substance. The ordinate indicates absorbance, and the abscissa indicates a concentration (µg/ml) of the competitive substances.
Fig. 10 shows binding specificity of the established monoclonal antibody analyzed by flow cytometry. The upper portion indicates the confirmation of binding of the monoclonal antibody to an mIgA1-expressing DG44 cell line, and the lower portion indicates the confirmation of binding of the monoclonal antibody to an mIgA1-expressing Lec8 cell line. The ordinate indicates number of cells, and the abscissa indicates fluorescence intensity.
Fig. 11 shows quantitation results of sugar chain-deficient IgA1, which are results obtained by a sandwich ELISA method constructed using the established monoclonal antibody. The ordinate indicates absorbance, and the abscissa indicates concentration (µg/ml) of the antigen.
Fig. 12 shows measurement results of the competitive inhibition activity of KM4137 (▲), KM4140 (◆), and KM4144 (●) with respect to binding of an anti-Tn antigen-added mIgA hinge peptide monoclonal antibody KM4137(a), KM4140(b), or KM4144(c) labeled with biotin to a Tn antigen type human IgA1 using flow cytometry (FCM). The ordinate indicates an average fluorescence intensity (OD415-OD490) at a sample wavelength of 415 nm and a reference wavelength of 490 nm, and the abscissa indicates a concentration (µg/ml) of competitive substances.

### Detailed Description of the Invention

The present invention relates to a monoclonal antibody which specifically recognizes and binds to a hinge region of a polypeptide encoded by heavy chain gene of immunoglobulin A1 comprising an O-linked sugar chain to which galactose is not bound. The heavy chain gene of immunoglobulin A1 may be any one, so long as it encodes a heavy chain of immunoglobulin A1. Examples include a gene comprising a nucleotide sequence (SEQ ID NO:3) which encodes an amino acid sequence of a constant region of a heavy chain of a secretory immunoglobulin A1 (SEQ ID NO:2). In addition, the IgA1 heavy chain gene of the present invention includes a gene which hybridizes with a DNA consisting of the nucleotide sequence represented by SEQ ID NO:3 under stringent conditions and also encodes a polypeptide having the function of the IgA1 heavy chain, and the like.

The DNA which hybridizes under stringent conditions refers to a DNA which is obtained by colony hybridization, plaque hybridization, Southern blot hybridization or the like using, a DNA consisting of the nucleotide sequence represented by SEQ ID NO:3 as a probe. A specific example of such DNA is a DNA which can be identified by performing hybridization at 65°C in the presence of 0.7 to 1.0 mol/l sodium chloride using a filter or a slide glass with colony- or plaque-derived DNA or PCR product or oligo DNA comprising the nucleotide sequence immobilized thereon, and then washing the filter or the slide glass at 65°C with a 0.1 to 2-fold concentration of SSC solution (1-fold concentration of SSC solution: 150 mmol/l sodium chloride and 15 mmol/l sodium citrate). Hybridization can be carried out according to the methods described in Molecular Cloning, A Laboratory Manual, Second Edition, Cold Spring Harbor Lab. Press (1989), Current Protocols in Molecular Biology, John Wiley & Sons (1987-1997); DNA Cloning 1: Core Techniques, A Practical Approach, Second Edition, Oxford University (1995); and the like. Specifically, the DNA capable of hybridization under stringent conditions includes DNA having at least 60% or more homology, preferably 80% or more homology, and most preferably 95% or more homology to the nucleotide sequence represented by SEQ ID NO:3.

In the nucleotide sequence of the gene encoding a protein of a eukaryote, genetic polymorphism is often recognized. The IgA1 heavy chain gene used in the present invention also includes a gene in which small modification is generated in the nucleotide sequence by such polymorphism.
The IgA1 heavy chain includes a polypeptide comprising the amino acid sequence represented by SEQ ID NO:2; a polypeptide comprising an amino acid sequence in which at least one amino acid is deleted, substituted or added in the amino acid sequence represented by SEQ ID NO:2 and having the function of the IgA1 heavy chain; a polypeptide comprising an amino acid sequence having at least 60% homology, preferably at least 80% homology, more preferably at least 90% homology, and most preferably at least 95% homology, to the amino acid sequence represented by SEQ ID NO:2 and having the function of the IgA1 heavy chain; and the like.

The polypeptide comprising an amino acid sequence in which one or more amino acid residue(s) is/are deleted, substituted and/or added in the amino acid sequence represented by SEQ ID NO:2 can be obtained, for example, by introducing a site-directed mutation into DNA encoding the polypeptide comprising the amino acid sequence represented by SEQ ID NO:2 by using method for site-directed mutagenesis described in Molecular Cloning, A Laboratory Manual, Second Edition (Cold Spring Harbor Laboratory Press, 1989), Current Protocols in Molecular Biology (John Wiley & Sons, 1987-1997), Nucleic Acids Research, 10, 6487 (1982), Proc. Natl. Acad Sci. USA, 79, 6409 (1982), Gene, 34, 315 (1985), Nucleic Acids Research, 13, 4431 (1985), Proc. Natl. Acad. Sci. USA, 82, 488 (1985), or the like. The number of amino acid residues which are deleted, substituted or added is not particularly limited, and the number is preferably, 1 to dozens, such as 1 to 20, and more preferably 1 to several, such as 1 to 5.

The number of the homology described in the present invention may be a number calculated by using a homology search program known by the skilled person, unless otherwise indicated. Regarding the nucleotide sequence, the number may be calculated by using a default parameter in BLAST [J. Mol. Biol., 215, 403 (1990)] or the like, and regarding the amino acid sequence, the number may be calculated by using a default parameter in BLAST2 *[*Nucleic Acids Res., 25, 3389 (1997); Genome Res., 7, 649 (1997); http://www.ncbi.nlm.nih.gov/Education/BLASTinfo/information3.html] or the like.

As the default parameter, G (cost to open gap) is 5 for the nucleotide sequence and 11 for the amino acid sequence; -E (cost to extend gap) is 2 for the nucleotide sequence and 1 for the amino acid sequence; -q (penalty for nucleotide mismatch) is -3; -r (reward for nucleotide match) is; -e (expect value) is 10; -W (wordsize) is 11 residues for the nucleotide sequence and 3 residues for the amino acid sequence; -y (Dropoff (X) for blast extensions in bits) is 20 for blastn and 7 for a program other than blastn; -X (X dropoff value for gapped alignment in bits) is 15; and -Z (final X dropoff value for gapped alignment in bits) is 50 for blastn and 25 for a program other than blastn (http://www.ncbi.nlm.nih.gov/blast/html/blastcgihelp.html).

The polypeptide comprising a partial sequence of the amino acid sequence represented by SEQ ID NO:2 can be prepared according to a method known by the skilled person. For example, it can be prepared by deleting a part of DNA encoding the amino acid sequence represented by SEQ ID NO:2 and culturing a transformant into which an expression vector containing the DNA is introduced. Also, based on the thus prepared polypeptide or DNA, a polypeptide comprising an amino acid sequence in which one or more amino acid(s) is/are deleted, substituted or added in a partial sequence of the amino acid sequence represented by SEQ ID NO:2 can be prepared in the same manner as described above.

In the present invention, the polypeptide encoded by IgA 1 heavy chain gene, comprising an O-linked sugar chain to which galactose is not bound may be any IgA1 heavy chain comprising an O-linked sugar chain to which galactose is not bound. Specifically, examples of the polypeptide include IgA1 heavy chain polypeptide comprising an O-linked sugar chain to which galactose is not bound and is encoded by the nucleotide sequence represented by SEQ ID NO:3.

As a hinge region of IgA1, for example, a region corresponding to position 223 to position 240 of an IgA1 heavy chain polypeptide specifically disclosed in a document [Biochemical and Biophysical Research Communication] is exemplified. In the present invention, a hinge region of a polypeptide encoded by an IgA1 heavy chain gene comprising an O-linked sugar chain to which galactose is not bound may be any region so long as this region is a hinge region of an IgA1 heavy chain polypeptide comprising an O-linked sugar chain to which galactose is not bound. Specific examples thereof include an amino acid sequence that is represented by SEQ ID NO:1 and contained in common in IgA1 heavy chain polypeptides comprising an O-linked sugar chain to which galactose is not bound and being encoded by a nucleotide sequence represented by SEQ ID NO:3.

The term "O-linked sugar chain" means a structure in which a sugar chain is bound via an -OH group contained in each amino acid side chain of an amino acid residue of serine (Ser) or threonine (Thr) of a protein. Among O-linked sugar chains, an O-linked sugar chain having N-acetylgalactosamine (GalNAc) bound to the -OH group of the amino acid side chain of Ser or Thr on the polypeptide is called "mucin-type sugar chain". Specific examples of the O-linked sugar chain include T antigen (TF antigen), sialyl T antigen, Tn antigen, sialyl-Tn antigen, and the like (Table 1).

**[Table 1]**

| Name of Sugar Chain Antigen | Sugar Chain Structure |
|---|---|
| Tn antigen | GalNAc1α→Ser/Thr |
| Sialyl Tn antigen | NeuNAcα2→6GalNAc1α→Ser/Thr |
| T antigen | Galβ1→3GalNAc1α→Ser/Thr |
| Sialyl T antigen | NeuNAcα2→3Galβ1→3GalNAc1α→Ser/Thr |

| | |
|---|---|
| (NeuNAc:N-acetylneuraminic acid) | |

In the present invention, the term "O-linked sugar chain to which galactose is not bound" means an O-linked sugar chain in which galactose (Gal) is not bound to N-acetylgalactosamine (GalNAc) bound via an -OH group of the amino acid residue of Ser or Thr in a protein. Specifically, examples include the above-mentioned Tn antigen and sialyl-Tn antigen. The O-linked sugar chain to which galactose is not bound is an intermediate in the synthetic pathway of a normal O-linked sugar chain, and generally rarely exists in glycoproteins of normal cells, and the expression thereof is found in specific diseases, such as cancer or nephropathy.

Hereinafter, in the present invention, the O-linked sugar chain to which galactose is not bound may be sometimes described as an abnormal sugar chain, a protein to which the abnormal sugar chain is bound may be sometimes described as a sugar chain-deficient protein, and IgA1 to which the abnormal sugar chain is bound may be sometimes described as a sugar chain-deficient IgA1.
Examples of the amino acid residue of a polypeptide to which an O-linked sugar chain is bound include an amino acid residue of serine (Ser) or threonine (Thr) in an amino acid sequence of the hinge region of the IgA1 heavy chain polypeptide.

In addition, the amino acid residue of a polypeptide to which the O-linked sugar chain is bound may be verified by a consensus sequence of O-linked sugar chain using a sequencer software, such as NetOGlyc 3.1 server (http://www.cbs.dtu.dk/services/NetOGlyc/). Alternatively, a specific sugar chain binding site may be identified by mass spectrometry (MS) analysis of a glycoprotein containing an O-linked sugar chain.

In the present invention, as the amino acid residue of the hinge region polypeptide to which the O-linked sugar chain on the IgA1 heavy chain polypeptide is bound, any of Ser or Thr residues in the amino acid sequence of the hinge region of IgA 1 heavy chain polypeptide is available. Examples of these preferably include a sugar chain binding site comprising at least one amino acid residue selected from the group consisting of threonine at position 225, threonine at position 228, serine at position 230, serine at position 232 and thereonine at position 236, in the amino acid sequence of human IgA1 heavy chain polypeptide.

The number of an O-linked sugar chain which binds to the heavy chain hinge region per one molecule of the IgA1 heavy chain polypeptide may be any number so long as an O-linked sugar chain binds to at least one Ser or Thr residue. The number of an O-linked sugar chain is not limited.
Examples of methods for obtaining a cell of the present invention, which expresses the IgA1 comprising an O-linked sugar chain to which galactose is not bound (hereinafter referred to as "sugar chain-deficient IgA1"), include a method for constructing a sugar chain-deficient IgA1-expressing cell by introducing DNA encoding IgA1 heavy chain and DNA encoding IgA1 light chain into a cell line in which the activity of an enzyme capable of adding Gal to N-acetylgalactosamine (GalNAc) bound to Ser/Thr on the polypeptide, of a protein involved in the activity of the above enzyme, or of a protein involved in the transportation of uridine 5'-diphospate-galactose (UDP-galactose) in the O-linked sugar chain synthesis process, is decreased or deleted. Alternatively, the cell which expresses IgA1 having an O-linked sugar chain to which galactose is not bound may also be constructed by treating the cell which expresses IgA1 having a normal O-linked sugar chain with a sugar chain cleavage enzyme, such as sialidase and galactosidase.

Specific examples of the enzyme capable of adding Gal to GalNAc bound to Ser or Thr on the polypeptide may include β1,3-galactosyltransferase [The Journal of Biological Chemistry, 277, 178-186 (2002)], and the like. Examples of the protein involved in the activity of the enzyme adding Gal to GalNAc bound to Ser or Thr on the polypeptide include Cosmc [Procedings of the National Academy of Sciences of the United States of America, 99, 16613-16618 (2002)], which is a chaperone involved in protein folding of the enzyme, and the like.

An IgA1-expressing cell derived from an IgA nephropathy patient can be used as a sugar chain deficient-IgA1-expressing cell, based on the fact that an enzymatic activity is decreased or deleted due to the occurrence of addition, deletion, substitution, or the like in a DNA, which encodes an enzyme capable of adding Gal to GalNAc bound to Ser/Thr on the polypeptide, a protein involved in the activity of the enzyme, a protein involved in the transportation of UDP-galactose, or the like.

Examples of the protein involved in the transportation of UDP-galactose include UDP-galactose transporter, and the like. Examples of the cell line in which the activity of the UDP-galactose transporter is decreased or deleted include Lec8 cells [Glycobiology, 1, 307-14 (1991)], and the like.
In the present invention, examples of the cell expressing the sugar chain-deficient IgA1 include a cell which is naturally present in the human body, a cell line established from the cell which is naturally present in the human body, a cell obtained by gene recombination techniques, and the like. Preferred are a cell line in which, in the O-linked sugar chain synthesis process, an activity of an enzyme capable of adding Gal to GalNAc bound to Ser/Thr on the polypeptide, a protein involved in the activity of the enzyme or a protein involved in the transportation of UDP-galactose, is decreased or deleted as described above, a cell having a similar property and naturally existing in the human body, and the like.

Examples of the cell naturally existing in the human body is preferably a cell line in which in the O-linked sugar chain synthesis process an activity of an enzyme capable of adding Gal to GalNAc bound to Ser/Thr on the polypeptide, a protein involved in the activity of the enzyme or a protein involved in the transportation of UDP-galactose, is decreased or deleted. Specific examples of such a cell include a cell which expresses the IgA1 heavy chain polypeptide in the bodies of patients suffering from IgA nephropathy or cancer, for example, a cell expressing the IgA1 heavy chain polypeptide among immune-related cells or tumor cells obtained by biopsy or the like.

Examples of the cell obtained by gene recombination techniques include a sugar chain-deficient IgA1-expressing cell obtained by constructing a host cell in which, in the O-linked sugar chain synthesis process, an activity of an enzyme adding Gal to GalNAc bound to Ser/Thr on the polypeptide, a protein involved in the activity of the enzyme or a protein involved in the transportation of UDP-galactose, is decreased or deleted and then introducing an expression vector containing cDNA encoding a desired polypeptide into the host cell.

Specific examples of the host cell include a Lec8 cell in which the activity of the UDP-galactose transporter is decreased, or an IgA1-expressing cell derived from IgA nephropathy patient in which the β 1,3-galactosyltransferase activity is decreased or deleted due to abnormality of the enzyme or a Cosmc chaperone protein involved in the activity of the enzyme.
In addition, the sugar chain-deficient IgA1 protein may be constructed by using the above IgA1-expressing cell to express and purify the sugar chain-deficient IgA1 protein.

The sugar chain-deficient IgA1 protein can be obtained by expressing the IgA1 protein as a fusion protein with another material, followed by purification. Examples of the material to be fused with the IgA1 protein include polypeptides such as antibody constant region, antibody Fc region, GST tag, histidine tag (also referred to as "His tag"), and Myc tag. The fusion protein may be separated and purified by using an affinity column, such as Protein A, nickel column, and specific antibody column.

The monoclonal antibody or the antibody fragment of the present invention has a binding activity to the thus obtained sugar chain-deficient IgA1 cell or sugar chain-deficient IgA1.
Binding of the antibody or antibody fragment of the present invention to the sugar chain-deficient IgA1 polypeptide can be confirmed by a method in which the binding ability of a cell expressing a specified antigen and an antibody for the specific antigen is confirmed, for example, by a conventionally known immunological detection method, preferably a fluorescent cell staining method or the like. In addition, it can also be confirmed by a combination of conventionally known immunological detection methods [Monoclonal Antibodies-Principles and Practice, Third edition, Academic Press (1996), Antibodies-A Laboratory Manual, Cold Spring Harbor Laboratory (1988), Monoclonal Antibody Experiment Manual, Kodansha Scientific (1987)] and the like.

The monoclonal antibody of the present invention includes an antibody produced by a hybridoma and a recombinant antibody produced by a transformant transformed with an expression vector containing a gene encoding an antibody.
The hybridoma can be prepared, for example, by preparing the above cell expressing the sugar chain-defeficient IgA1 as an antigen, inducing an antibody-producing cell having antigen specificity from an animal immunized with the antigen, and fusing the antigen-producing cell with a myeloma cell. The anti-sugar chain-defeficient IgA1 antibody can be obtained by culturing the hybridoma or administering the hybridoma cell into an animal to cause ascites tumor in the animal and separating and purifying the culture or the ascites.

The animal immunized with an antigen may be any animal, so long as a hybridoma can be prepared, and mouse, rat, hamster, rabbit or the like is suitably used. Also, the cell having antibody-producing activity can be obtained from such an animal, and the antibody of the present invention includes an antibody produced by a hybridoma obtained by fusion of the cell after *in vitro* immunization with a myeloma cell.

The monoclonal antibody is an antibody secreted by a single clone of antibody-producing cells, and recognizes only one epitope (also called antigen determinant) and has the uniformity in amino acid sequence (primary structure).
Examples of the epitope include a single amino acid sequence, a three-dimensional structure consisting of an amino acid sequence, an amino acid sequence having a sugar chain bound thereto, a three-dimensional structure consisting of an amino acid sequence having a sugar chain bound thereto, and the like, which a monoclonal antibody recognizes and binds to. Examples of the epitope of the monoclonal antibody of the present invention include a three-dimensional structure of the sugar chain-deficient IgA1 protein.

Examples of the monoclonal antibody of the present invention include any monoclonal antibody, so long as it recognizes and also binds to the heavy chain hinge region of the sugar chain-deficient IgA1. Specific examples of the monoclonal antibody include monoclonal antibodies KM4137, KM4138, KM4139, KM4140, KM4144, and the like.
More specifically, examples of the monoclonal antibody of the present invention include a monoclonal antibody KM4137 produced by hybridoma KM4137, a monoclonal antibody which competes with the monoclonal antibody KM4137 in the binding to the heavy chain hinge region of the sugar chain-deficient IgA1, and a monoclonal antibody that binds to an epitope present in the hinge region of the sugar chain-deficient IgA1 heavy chain to which the monoclonal antibody KM4137 binds.

Further, examples of the monoclonal antibody of the present invention include a monoclonal antibody KM4138 produced by hybridoma KM4138, a monoclonal antibody which competes with the monoclonal antibody KM4138 in the binding to the heavy chain hinge region of the sugar chain-deficient IgA1, and a monoclonal antibody that binds to an epitope present in the hinge region of the sugar chain-deficient IgA1 heavy chain to which the monoclonal antibody KM4138 binds.

Further, examples of the monoclonal antibody of the present invention include monoclonal antibody KM4139 produced by hybridoma KM4139, a monoclonal antibody which competes with the monoclonal antibody KM4139 in the binding to the heavy chain hinge region of the sugar chain-deficient IgA1, and a monoclonal antibody that binds to an epitope present in the hinge region of the sugar chain-deficient IgA1 heavy chain to which the monoclonal antibody KM4139 binds.

Further, examples of the monoclonal antibody of the present invention may include monoclonal antibody KM4140 produced by hybridoma KM4140, a monoclonal antibody which competes with the monoclonal antibody KM4140 in the binding to the heavy chain hinge region of the sugar chain-deficient IgA1, and a monoclonal antibody that binds to an epitope present in the hinge region of the sugar chain-deficient IgA1 heavy chain to which the monoclonal antibody KM4140 binds.

Further, examples of the monoclonal antibody of the present invention include monoclonal antibody KM4144 produced by hybridoma KM4144, a monoclonal antibody which competes with the monoclonal antibody KM4144 in the binding to the heavy chain hinge region of sugar chain-deficient IgA1, and a monoclonal antibody that binds to an epitope present in the hinge region of the sugar chain-deficient IgA1 heavy chain to which the monoclonal antibody KM4144 binds.

Examples of the monoclonal antibody which competes with the monoclonal antibody of the present invention include, specifically, a monoclonal antibody which has a competitive reaction for a variety of monoclonal antibodies and the epitope present in the heavy chain hinge region of the sugar chain-deficient IgA1, as described above.
An antibody which competes with a monoclonal antibody is an antibody whose epitope in an antigen is the same as or partially the same as that of monoclonal antibody of the presetn invention, and which binds to the epitope.

Further, examples of the monoclonal antibody that binds to an epitope to which the monoclonal antibody of the present invention binds include, specifically, a monoclonal antibody that binds to the epitope present in the hinge region of the sugar chain-deficient IgA1 which is recognized by a variety of monoclonal antibodies described above.
The hybridoma KM4137, KM4140 and KM4144 have been deposited to International Patent Organism Depositary, National Institute of Advanced Industrial Science and Technology (Central 6, 1-1, Higashi 1-chome, Tsukuba, Ibaraki 305-8566, Japan) under the Budapest Treaty as FERM BP-11214, FERM BP-11215 and FERM BP-11216 on December 18, 2009.

The recombinant antibody includes an antibody produced by gene recombination, such as a human chimeric antibody, a humanized antibody, a human antibody and an antibody fragment thereof. Among the recombinant antibodies, one having antigen binding activity, low immunogenecity and prolonged half-life in blood is preferable as a therapeutic agent.
The human chimeric antibody is an antibody comprising a heavy chain variable region (hereinafter referred to as "VH") and a light chain variable region (hereinafter referred to as "VL") of an antibody of a non-human animal and a heavy chain constant region (hereinafter referred to as "CH") and a light chain constant region (hereinafter referred to as "CL") of a human antibody.

The human chimeric antibody of the present invention can be produced as follows. Specifically, the human chimeric antibody can be produced by obtaining cDNAs encoding VH and VL from a hybridoma which produces a monoclonal antibody which specifically recognizes the sugar chain-deficient IgA1 and binds to the heavy chain hinge region or a monoclonal antibody which specifically recognizes sugar chain-deficient IgA1 and binds to the heavy chain hinge region, inserting each of them into an expression vector for animal cell comprising DNAs encoding CH and CL of human antibody to thereby construct a vector for expression of human chimeric antibody, and then introducing the vector into an animal cell to express the antibody.

As the CH of the human chimeric antibody, any CH can be used, so long as it belongs to human immunoglobulin (hereinafter referred to as "hIg"), and those belonging to the hIgG class are preferred, and any one of the subclasses belonging to the hIgG class, such as hIgG1, hIgG2, hIgG3 and hIgG4, can be used. As the CL of the human chimeric antibody, any CL can be used, so long as it belongs to the hIg class, and those belonging to κ class or λ class can be used.

A humanized antibody is an antibody in which amino acid sequences of CDRs of VH and VL of an antibody derived from a non-human animal are grafted into appropriate positions of VH and VL of a human antibody, and is also called a human CDR-grafted antibody or a reshaped-antibody.
The humanized antibody of the present invention can be produced by constructing cDNAs encoding an antibody variable region (hereinafter referred to as "V region") in which the amino acid sequences of CDRs of VH and VL of a non-human animal antibody produced by a hybridoma which produces a monoclonal antibody which specifically recognizes the sugar chain-deficient IgA1 protein and binds to the heavy chain hinge region in the present invention are grafted into frameworks (hereinafter referred to as "FR") of VH and VL of any human antibody, inserting each of them into an expression vector of animal cell comprising genes encoding CH and CL of a human antibody to thereby construct a vector for expression of humanized antibody, and introducing it into an animal cell to thereby express.

As the amino acid sequences of FRs of VH and VL of a human antibody, any amino acid sequences can be used, so long as they are amino acid sequences of VH and VL, respectively, derived from a human antibody. Examples include amino acid sequences of FRs of VH and VL of human antibodies registered in database such as Protein Data Bank, common amino acid sequences of each sub group of FRs of VH and VL of human antibodies described in, for example, Sequences of Proteins of Immunological Interest, US Dept. Health and Human Services (1991), and the like.

As the CH of the humanized antibody, any CH can be used, so long as it belongs to the hIg class, and those of the hIgG class are preferred and any one of the subclasses belonging to the hIgG class, such as hIgG1, hIgG2, hIgG3 and hIgG4 can be used. As the CL of the humanized antibody, any CL can be used, so long as it belongs to the hIg class, and those belonging to the κ class or λ class can be used.
A human antibody is originally an antibody naturally existing in the human body, and it also includes antibodies obtained from a human antibody phage library or a human antibody-producing transgenic animal, which is prepared based on the recent advance in genetic engineering, cell engineering and developmental engineering techniques.

The antibody exogenously existing in the human body can be prepared, for example by isolating a human peripheral blood lymphocyte, immortalizing it by infecting with EB virus or the like and then cloning it to thereby obtain lymphocytes capable of producing the antibody, culturing the lymphocytes thus obtained, and purifying the antibody from the supernatant of the culture.
The human antibody phage library is a library in which antibody fragments such as Fab and scFv are expressed on the phage surface by inserting a gene encoding an antibody prepared from a human B cell into a phage gene. A phage expressing an antibody fragment of the cell surface having the desired antigen binding activity can be recovered from the library, using its activity to bind to an antigen-immobilized substrate as the index. The antibody fragment can be converted further into a human antibody molecule consisting of two full H chains and two full L chains by genetic engineering techniques.

A human antibody-producing transgenic animal means an animal in which a human antibody gene is integrated into cells. Specifically, a human antibody-producing transgenic animal can be prepared by introducing a gene encoding a human antibody into a mouse ES cell, grafting the ES cell into an early stage embryo of other mouse and then developing it into a complete animal. A preparation method of a human antibody from a human antibody-producing transgenic animal can be performed by obtaining a human antibody-producing hybridoma using a hybridoma preparation method usually applied for non-human animals, culturing the obtained hybridoma and producing and accumulating the human antibody in the supernatant of the culture.

An antibody or antibody fragment thereof in which one or more amino acids are deleted, substituted, inserted or added in the amino acid sequence constituting the above antibody or antibody fragment, having activity similar to the above antibody or antibody fragment is also included in the antibody or antibody fragment of the present invention.
The number of amino acid residues which are deleted, substituted, inserted and/or added is one or more, and is not specifically limited, but it is within the range where deletion, substitution or addition is possible by known methods such as the site-directed mutagenesis described in Molecular Cloning, Second Edition,; *Current Protocols in Molecular Biology;* Nucleic Acids Research, 10, 6487 (1982), Proc. Natl. Acad Sci. USA, 79, 6409 (1982); Gene, 34, 315 (1985), Nucleic Acids Research, 13, 4431 (1985); Proc. Natl. Acad Sci. USA, 82, 488 (1985) or the like. For example, the number is 1 to dozens, preferably 1 to 20, more preferably 1 to 10, and most preferably 1 to 5.

Deleting, substituting, inserting or adding one or more amino acids in the amino acid sequence of the above antibody means the followings. That is, it means there is deletion, substitution, insertion and/or addition of one or plural amino acid residues at any positions in one or plural amino acid sequences of a single sequence. Also, the deletion, substitution, insertion and/or addition may exist at the same case and the amino acid which is substituted, inserted or added may be either a natural type or a non-natural type. The natural type amino acid includes L-alanine, L-asparagine, L-aspartic acid, L-glutamine, L-glutamic acid, glycine, L-histidine, L-isoleucine, L-leucine, L-lysine, L-methionine, L-phenylalanine, L-proline, L-serine, L-threonine, L-tryptophan, L-tyrosine, L-valine, L-cysteine and the like.

Preferable examples of mutually substitutable amino acids are shown below. The amino acids in the same group are mutually substitutable.
- Group A:: leucine, isoleucine, norleucine, valine, norvaline, alanine, 2-aminobutanoic acid, methionine, O-methylserine, t-butylglycine, t-butylalanine, cyclohexylalanine
- Group B:: aspartic acid, glutamic acid, isoaspartic acid, isoglutamic acid, 2-aminoadipic acid, 2-aminosuberic acid
- Group C:: asparagine, glutamine
- Group D:: lysine, arginine, ornithine, 2,4-diaminobutanoic acid, 2,3-diaminopropionic acid
- Group E:: proline, 3-hydroxyproline, 4-hydroxyproline
- Group F:: serine, threonine, homoserine
- Group G:: phenylalanine, tyrosine
Effector activity of the antibody includes ADCC activity, CDC activity, antibody-dependent cellular phagocytosis (ADCP) activity, opsonization effects, and the like. It may be controlled by a variety of methods.

Examples of the method for controlling the effector activity include a method for controlling a sugar chain bound to the Fc region of the antibody, a method for carrying out amino acid modification of amino acid residue(s) in the Fc region of the antibody, and the like.
Examples of the method for controlling a sugar chain bound to the Fc region of the antibody include a method for lowering ADCC or CDC activity by eliminating a sugar chain at position 297 of the IgG antibody [Molecular Immunology, 32, 1311, (1995), WO2008/030564], a method for lowering CDC activity by decreasing the binding of galactose to the Fc region of the antibody, and the like.

Further, examples of the method for controlling a sugar chain bound to the Fc region of the antibody include a method for producing an antibody containing a sugar chain having no fucose bound to N-acetylglucosamine (GlcNAc) of a base to which a sugar chain is bound, in the N-linked sugar chain bound to asparagine at position 297 of the Fc region of the IgG antibody (US7,214,775, and US6,946,292), a method for producing an antibody containing a sugar chain containing bisecting GlcNAc bound thereto [Nature Biotechnology, 17, 176, (1999)], a method for producing an antibody containing a sugar chain bound to galactose (Gal) in the non-reducing terminal [Hum. Antibod. Hybridomas, 5, 143-151. (1994)], and the like.

Examples of the method for carrying out amino acid modification of amino acid residue(s) in the Fc region of the antibody include a method for controlling the effector activity by amino acid modification of the Fc region of the antibody (J.B.C., 277, 26733-26740, 2002, US6,737,056, US7,297,775, US2007/0020260, and WO2005/070963), a method for controlling the effector activity by domain exchange between respective subclasses of the antibody Fc region (W02007/011041), and the like.

The antibody fragment of the present invention includes Fab, Fab', F(ab')₂, scFv, diabody, dsFv and the like.
The antibody fragment of the present invention includes Fab, Fab', F(ab')₂, scFv, diabody, dsFv, a peptide comprising CDR and the like.
An Fab is an antibody fragment having a molecular weight of about 50,000 and antigen binding activity, in which about a half of the N-terminal side of H chain and the entire L chain, among fragments obtained by treating an IgG antibody molecule with a protease, papain (cleaved at an amino acid residue at position 224 of the H chain), are bound together through a disulfide bond.

The Fab of the present invention can be obtained by treating a monoclonal antibody which specifically recognizes the sugar chain-deficient IgA1 protein of the present invention and binds to the heavy chain hinge region with a protease, papain. Also, the Fab can be produced by inserting DNA encoding Fab of the antibody into an expression vector for prokaryote or an expression vector for eukaryote, and introducing the vector into a prokaryote or an eukaryote to express the Fab.

An F(ab')₂ is an antibody fragment having a molecular weight of about 100,000 and antigen binding activity and comprising two Fab regions which are bound in the hinge portion obtained by digesting the lower part of two disulfide bonds in the hinge region of IgG, with an enzyme, pepsin.
The F(ab')₂ of the present invention can be obtained by treating a monoclonal antibody which specifically recognizes the sugar chain-deficient IgA1 protein of the present invention and binds to the heavy chain hinge region with a protease, pepsin. Also, the F(ab')₂ can be produced by binding Fab' described below via a thioether bond or a disulfide bond.

An Fab' is an antibody fragment having a molecular weight of about 50,000 and antigen binding activity, which is obtained by cleaving a disulfide bond at the hinge region of the above F(ab')₂.
The Fab' of the present invention can be obtained by treating F(ab')₂ which specifically recognizes the sugar chain-deficient IgA1 protein of the present invention and binds to the heavy chain hinge region, with a reducing agent, dithiothreitol. Also, the Fab' can be produced by inserting DNA encoding Fab' fragment of the antibody into an expression vector for prokaryote or an expression vector for eukaryote, and introducing the vector into a prokaryote or eukaryote to express the Fab'.

A scFv is a VH-P-VL or VL-P-VH polypeptide in which a VH chain and a VL chain are linked using an appropriate peptide linker (hereinafter referred to as "P") and is an antibody fragment having antigen binding activity.
The scFv of the present invention can be produced by obtaining cDNAs encoding VH and VL of a monoclonal antibody which specifically recognizes the sugar chain-deficient IgA1 protein and binds to the heavy chain hinge region, constructing DNA encoding the scFv, inserting the DNA into an expression vector for prokaryote or an expression vector for eukaryote, and then introducing the expression vector into a prokaryote or eukaryote to express the scFv.

A diabody is an antibody fragment in which scFv is dimerized, and has divalent antigen binding activity. In the divalent antigen binding activity, two antigens may be the same or different.
The diabody of the present invention can be produced by obtaining cDNAs encoding VH and VL of a monoclonal antibody which specifically recognizes the sugar chain-deficient IgA1 protein and binds to the heavy chain hinge region, constructing DNA encoding the scFv so that the length of the amino acid sequence of P is 8 or less residues, inserting the DNA into an expression vector for prokaryote or an expression vector for eukaryote, and then introducing the expression vector into a prokaryote or eukaryote to express the diabody.

A dsFv is obtained by binding polypeptides in which one amino acid residue of each of VH and VL is substituted with a cysteine residue via a disulfide bond between the cysteine residues. The amino acid residue to be substituted with a cysteine residue can be selected based on a three-dimensional structure prediction of the antibody in accordance with the method shown by Reiter et al. [Protein Engineering, 7, 697(1994)].
The dsFv of the present invention can be produced by obtaining cDNAs encoding VH and VL of a monoclonal antibody which specifically recognizes the sugar chain-deficient IgA1 protein and binds to the heavy chain hinge region, constructing DNA encoding dsFv, inserting the DNA into an expression vector for prokaryote or an expression vector for eukaryote, and then introducing the expression vector into a prokaryote or eukaryote to express the dsFv.

A peptide comprising CDR is constituted by including at least one region or more of CDRs of VH or VL. The peptide comprising plural CDRs can be produced by connecting CDRs directly or via an appropriate peptide linker.
The peptide comprising CDR of the present invention can be produced by constructing DNAs encoding CDRs of VH and VL of a monoclonal antibody which specifically recognizes the sugar chain-deficient IgA1 protein and binds to the heavy chain hinge region, inserting the DNAs into an expression vector for prokaryote or an expression vector for eukaryote, and then introducing the expression vector into a prokaryote or eukaryote to express the peptide.

The peptide comprising CDRs can also be produced by a chemical synthesis method such as Fmoc method (fluorenylmethoxycarbonyl method) or tBoc method (t-butyloxycarbonyl method).
The present invention includes an antibody conjugate in which a monoclonal antibody or an antibody fragment thereof which specifically recognizes the sugar chain-deficient IgA1 protein and binds to the heavy chain hinge region is chemically or genetically bound to an agent, a protein, a radioisotope or the like.

The conjugate of the present invention can be produced by chemically conjugating an agent, a protein, a radioisotope or the like to the N-terminal side or C-terminal side of an H chain or an L chain of the monoclonal antibody or the antibody fragment thereof which specifically recognizes the sugar chain-deficient IgA1 protein and binds to the heavy chain hinge region in the present invention, an appropriate substituent or side chain of the antibody or the antibody fragment, a sugar chain in the antibody or the antibody fragment or the like [Antibody Engineering Handbook, edited by Osamu Kanemitsu, published by Chijin Shokan (1994)].

Also, the conjugate of the present invention can be genetically produced by linking a DNA encoding the monoclonal antibody or the antibody fragment thereof which specifically recognizes the sugar chain-deficient IgA1 protein and binds to the heavy chain hinge region in the present invention to other DNA encoding a protein to be conjugated, inserting the DNA into a vector for expression, and introducing the expression vector into a host cell of a prokaryote or eukaryote.

The agent includes a chemotherapeutic agent, a therapeutic antibody, an immunostimulator, an agent having high molecular weight, and the like.
The protein includes cytokine, a growth factor, a toxic protein, and the like.
Furthermore, the agent to be conjugated to the antibody or the antibody fragment thereof may be in a form of a prodrug. The prodrug in the present invention is an agent which is subjected to chemical modification by an enzyme existing in the tumor environment and is converted to a substance having an activity of damaging the tumor cells.

The chemotherapeutic agent includes any chemotherapeutic agents such as an alkylating agent, a nitrosourea agent, a metabolism antagonist, an anticancer antibiotic substance, an alkaloid derived from a plant, a topoisomerase inhibitor, an agent for hormonotherapy, a hormone antagonist, an aromatase inhibitor, a P glycoprotein inhibitor, a platinum complex derivative, an M-phase inhibitor and a kinase inhibitor. Examples of the chemotherapeutic agent include amifostine (Ethyol), cisplatin, dacarbazine (DTIC), dactinomycin, mecloretamin (nitrogen mustard), streptozocin, cyclophosphamide, iphosphamide, carmustine (BCNU), lomustine (CCNU), doxorubicin (adriamycin), doxorubicin lipo (Doxyl), epirubicin, gemcitabine (Gemsal), daunorubicin, daunorubicin lipo (Daunozome), procarbazine, mitomycin, cytarabine, etoposide, methotrexate, 5-fluorouracil, fluorouracil, vinblastine, vincristine, bleomycin, daunomycin, peplomycin, estramustine, paclitaxel (Taxol), docetaxel (Taxotea), aldesleukin, asparaginase, busulfan, carboplatin, oxaliplatin, nedaplatin, cladribine, camptothecin, CPT-12, 10-hydroxy-7-ethylcamptothecin (SN38), floxuridine, fludarabine, hydroxyurea, iphosphamide, idarubicin, mesna, irinotecan, nogitecan, mitoxantrone, topotecan, leuprolide, megestrol, melfalan, mercaptopurine, hydroxycarbamide, plicamycin, mitotane, pegasparagase, pentostatin, pipobroman, streptozocin, tamoxifen, goserelin, leuprorelin, flutamide, teniposide, testolactone, thioguanine, thiotepa, uracil mustard, vinorelbine, chlorambucil, hydrocortisone, prednisolone, methylprednisolone, vindesine, nimustine, semustine, capecitabine, Tomudex, azacytidine, UFT, oxaliplatin, gefitinib (Iressa), imatinib (STI 571), elrotinib, Flt3 inhibitor, VEGFR inhibitor, FGFR inhibitor, radicicol, 17-allylamino-17-demethoxygeldanamycin, rapamycin, amsacrine, all-trans-retinoic acid, thalidomide, anastrozole, fadrozole, letrozole, exemestane, gold thiomalate, D-penicillamine, bucillamine, azathioprine, mizoribine, cyclosporine, rapamycin, hydrocortisone, bexarotene (Targretin), tamoxifen, dexamethasone, progestin substances, estrogen substances, anastrozole (Arimidex), Leuplin, aspirin, indomethacin, celecoxib, azathioprine, penicillamine, gold thiomalate, chlorpheniramine maleate, chlorpheniramine, clemastine, tretinoin, bexarotene, arsenic, voltezomib, allopurinol, gemtuzumab, ibritumomab tiuxetan, 131 tositumomab, Targretin, ONTAK, ozogamine, clarithromycin, leucovorin, ifosfamide, ketoconazole, aminoglutethimide, suramin, methotrexate, maytansinoid and derivatives thereof.

The method for conjugating the chemotherapeutic agent with the antibody includes a method in which the chemotherapeutic agent and an amino group of the antibody are conjugated via glutaraldehyde, a method in which an amino group of the chemotherapeutic agent and a carboxyl group of the antibody are conjugated via watersoluble carbodiimide, and the like.
The therapeutic antibody includes an antibody against an antigen in which apoptosis is induced by binding of the antibody, an antibody against an antigen participating in formation of morbid state of tumor, an antibody which regulates immunological function and an antibody relating to angiogenesis in the morbid part.

The antigen in which apoptosis is induced by binding of the antibody includes cluster of differentiation (hereinafter "CD") 19, CD20, CD21, CD22, CD23, CD24, CD37, CD53, CD72, CD73, CD74, CDw75, CDw76, CD77, CDw78, CD79a, CD79b, CD80 (B7.1), CD81, CD82, CD83, CDw84, CD85, CD86 (B7.2), human leukocyte antigen (HLA)-Class II, EGFR and the like.

The antigen which regulates immunological function includes CD4, CD40, CD40 ligand, B7 family molecule (CD80, CD86, CD274, B7-DC, B7-H2, B7-H3, B7-H4), ligand of B7 family molecule (CD28, CTLA-4, ICOS, PD-1, BTLA), OX-40, OX-40 ligand, CD137, tumor necrosis factor (TNF) receptor family molecule (DR4, DR5, TNFR1, TNFR2), TNF-related apoptosis-inducing ligand receptor (TRAIL) family molecule, receptor family of TRAIL family molecule (TRAIL-R1, TRAIL-R2, TRAIL-R3, TRAIL-R4), receptor activator of nuclear factor kappa B ligand (RANK), RANK ligand, CD25, folic acid receptor 4, cytokine [interleukin-1α (hereinafter interleukin is referred to as "IL"), IL-1β, IL-4, IL-5, IL-6, IL-10, IL-13, transforming growth factor (TGF) β, TNFα, etc.], receptors of these cytokines, chemokine (SLC, ELC, I-309, TARC, MDC, CTACK, *etc.*) and receptors of these chemokines.

The antigen for the antibody which inhibits angiogenesis in the morbid part includes vascular endothelial growth factor (VEGF), angiopoietin, fibroblast growth factor (FGF), EGF, platelet-derived growth factor (PDGF), insulin-like growth factor (IGF), erythropoietin (EPO), TGFβ, IL-8, ephilin, SDF-1 and the like and receptors thereof.
The immunostimulator may be any natural products known as immunoadjuvants. Examples of an agent enhancing immunogen include β-1,3-glucan (lentinan, schizophyllan), α-galactosylceramide (KRN7000), fungus powder (picibanil, BCG) and fungus extract (krestin).

The high-molecular-weight agent includes polyethylene glycol (hereinafter referred to as "PEG"), albumin, dextran, polyoxyethylene, styrene-maleic acid copolymer, polyvinylpyrrolidone, pyran copolymer, hydroxypropylmethacrylamide, and the like. By binding these high-molecular-weight compounds to the antibody or antibody fragment, the following effects are expected: (1) improvement of stability against various chemical, physical or biological factors, (2) remarkable prolongation of half life in blood, (3) depletion of immunogenicity or suppression of antibody production, and the like [Bioconjugate Drug, Hirokawa Shoten (1993)]. Examples of the method for conjugating PEG to the antibody include a method for reacting an antibody with a PEG-modifying reagent [Bioconjugate Drug, Hirokawa Shoten (1993)]. The PEG-modifying reagent includes a modifying agent for ε-amino group of lysine (Japanese Published Unexamined Patent Application No. 178926/86), a modifying agent for a carboxyl group of aspartic acid and glutamic acid (Japanese Published Unexamined Patent Application No. 23587/81), a modifying agent for a guanidino group of arginine (Japanese Published Unexamined Patent Application No. 117920/90) and the like.

The cytokine or the growth factor may be any cytokine or growth factor, so long as it enhances cells such as NK cells, macrophages and neutrophils. Examples include interferon (hereinafter referred to as "IFN")-α, INF-β, INF-γ, IL-2, IL-12, IL-15, IL-18, IL-21, IL-23, granulocyte-colony stimulating factor (G-CSF), granulocyte macrophage-colony stimulating factor (GM-CSF), macrophage-colony stimulating factor (M-CSF) and the like.

The toxic protein includes ricin, diphtheria toxin, ONTAK and the like, and also includes a toxic protein in which mutation is introduced into a protein in order to control the toxicity.
The radioisotope includes ¹³¹I, ¹²⁵I, ⁹⁰Y, ⁶⁴Cu, ¹⁹⁹Tc, ⁷⁷Lu, ²¹¹At, ¹⁸⁶Re, ¹⁸⁸Re, ¹¹¹In and the like. The radioisotope can directly be conjugated with the antibody by Chloramine-T method. Also, a substance chelating the radioisotope can be conjugated with the antibody. The chelating agent includes methylbenzyldiethylene-triaminepentaacetic acid (MX-DTPA) and the like.

In the present invention, the antibody used in the present invention can be administered in combination with one or more of other agents, or in combination with radiation irradiation. The other agent includes the above-described chemotherapeutic agent, therapeutic antibody, immunostimulator, cytokine, growth factor and the like.
The radiation irradiation includes photon (electromagnetic) irradiation such as X-ray or γ-ray, particle irradiation such as electron beam, proton beam or heavy particle beam, and the like.

In the method for combined administration, the agent may be simultaneously administered with the antibody used in the present invention, or the agent may be administered before or after the administration of the antibody used in the present invention.
The detection method, quantification method, detection reagent quantification reagent or diagnostic agent in the present invention includes a method in which a specified label is used for labeling the antibody of the present invention. The label includes a label which is used in the general immunological detection or measuring method, and examples include enzymes such as alkaline phosphatase, peroxidase and luciferase, luminescent materials such as acridinium ester and lophine, fluorescent materials such as fluorescein isothiocyanate (FITC) and trimethylrhodamine (RITC), and the like.

Hereinafter, the production process of the antibody of the present invention will be described in more detail.

### 1. Production process of monoclonal antibody

### (1) Preparation of antigen

In accordance with the following procedure, sugar chain-deficient IgA1 protein as an antigen or a cell expressing the sugar chain-deficient IgA1 can be obtained by introducing an expression vector comprising a cDNA encoding full-length or partial-length IgA 1 heavy chain into yeast, an insect cell, an animal cell or the like, in which an activity of an enzyme capable of adding Gal to GalNAc bound to Ser/Thr on the polypeptide, a protein involved in the activity of the enzyme or a protein involved in the transportation of UDP-galactose is decreased or deleted, in the O-linked sugar chain synthesis process. Also, the sugar chain-deficient IgA1 can be purified from a variety of human-derived cultured cells, human tissues and the like which express a large amount of the sugar chain-deficient IgA1 onto a cell membrane or into a culture medium, to thereby prepare antigens. Further, the sugar chain-deficient IgA1 can also be obtained by an in vitro addition of a sugar chain to the IgA1 protein that was expressed and purified using a prokaryote, such as *Escherichia coli,* which is deficient in a sugar chain-adding ability.

Similarly, a cell which expresses IgA1 heavy chain containing a normal O-linked sugar chain can be obtained by introducing an expression vector comprising a cDNA encoding full-length or partial-length IgA1 heavy chain into a host cell such as yeast, insect cell, or animal cell which has a normal O-linked sugar chain synthesis process, and purifying the IgA1 heavy chain protein containing a normal O-linked sugar chain from the thus obtained cell.

The sugar chain-deficient IgA1 protein, the IgA1 protein containing a normal O-linked sugar chain or the expression cell obtained as above can be used for screening the desired antibody, and confirming the reactivity of the obtained antibody for an antigen.
The polypeptide used in the present invention can be produced, for example, by expressing a DNA encoding the polypeptide in a host cell using a method described in Molecular Cloning, A Laboratory Manual, Second Edition, Cold Spring Harbor Laboratory Press (1989), Current Protocols in Molecular Biology, John Wiley & Sons (1987-1997) or the like according to the following method.

Firstly, a recombinant vector is prepared by introducing a full length cDNA into downstream of a promoter of an appropriate expression vector. At this time, if necessary, a DNA fragment having an appropriate length containing a region encoding the polypeptide, which is prepared based on the full length cDNA, may be used instead of the above full length cDNA. Next, a transformant producing the polypeptide can be obtained by introducing the recombinant vector into a host cell suitable for the expression vector.

The host cell may be any one, so long as it has the ability to add an O-linked sugar chain and can express the gene of interest, and includes *Escherichia coli,* an yeast, an insect cell, an animal cell and the like.
As the expression vectors, those which can replicate autonomously in the host cell to be used or can be integrated into a chromosome, and comprises an appropriate promoter at such a position that the DNA encoding the polypeptide can be transcribed, are used.

When a prokaryote such as *Escherichia coli* is used as the host cell, it is preferred that the recombinant vector is autonomously replicable in the prokaryote and contains a promoter, a ribosome binding sequence, the DNA used in the present invention and a transcription termination sequence. The recombinant vector may further comprise a gene regulating the promoter.

The expression vector includes, for example, pBTrp2, pBTac1, pBTac2 (all manufactured by Roche Diagnostics), pKK233-2 (manufactured by Pharmacia), pSE280 (manufactured by Invitrogen), pGEMEX-1 (manufactured by Promega), pQE-8 (manufactured by QIAGEN), pKYP10 (Japanese Published Unexamined Patent Application No. 110600/83), pKYP200 *[*Agricultural Biological Chemistry, 48, 669 (1984)], pLSA1 [Agric. Biol. Chem., 53, 277 (1989)], pGEL1 [Proc. Natl. Acad. Sci. USA, 82, 4306 (1985)], pBluescript II SK(-) (manufactured by Stratagene), pTrs30 [prepared from *Escherichia coli* JM109/pTrS30 (FERM BP-5407)], pTrs32 [prepared from *Escherichia coli* JM109/pTrS32 (FERM BP-5408)], pGHA2 [prepared from *Escherichia coli* IGHA2 (FERM BP-400), Japanese Published Unexamined Patent Application No. 221091/85], pGKA2 [prepared from *Escherichia coli* IGKA2 (FERM BP-6798), Japanese Published Unexamined Patent Application No. 221091/85], pTerm2 (US4686191, US4939094, US5160735), pSupex, pUB110, pTP5, pC194, pEG400 *[*J. Bacteriol., 172, 2392 (1990)], pGEX (manufactured by Pharmacia), pET system (manufactured by Novagen), pME18SFL3 and the like.

Any promoter can be used, so long as it can function in the host cell to be used. Examples include promoters derived from *Escherichia coli,* phage and the like, such as trp promoter (Ptrp), lac promoter, PL promoter, PR promoter and T7 promoter. Also, artificially designed and modified promoters, such as a promoter in which two Ptrp are linked in tandem, tac promoter, lacT7 promoter and letI promoter, can be used.

Also, the above recombinant vector is preferably a plasmid in which the space between Shine-Dalgarno sequence, which is the ribosome binding sequence, and the initiation codon is adjusted to an appropriate distance (for example, 6 to 18 nucleotides). In the nucleotide sequence of DNA encoding the polypeptide used in the present invention, nucleotides can be arranged so as to obtain a suitable codon for expression in the host so that the producing ratio of the polypeptide of interest can be improved. Furthermore, the transcription termination sequence is not essential to express a gene in the above recombinant vector, it is preferred to arrange a transcription terminating sequence immediately downstream of the structural gene.

The host cell includes microorganisms belonging to the genera *Escherichia,* and examples include *Escherichia coli* XLI-Blue, *Escherichia coli* XL2-Blue, *Escherichia coli* DH1, *Escherichia coli* MC1000, *Escherichia coli* KY3276, *Escherichia coli* W1485, *Escherichia coli* JM109, *Escherichia coli* HB101, *Escherichia coli* No. 49, *Escherichia coli* W3110, *Escherichia coli* NY49, *Escherichia coli* DH5α and the like.
Any introduction method for the recombinant vector can be used, so long as it is a method for introducing DNA into the above-described host cell, and examples include a method using a calcium ion described in Proc. Natl. Acad. Sci. USA, 69, 2110 (1972), methods described in Gene, 17, 107 (1982) and Molecular & General Genetics, 168, 111 (1979) and the like.

When an animal cell is used as the host cell, an expression vector includes, for example, pcDNAI, pcDM8 (available from Funakoshi), pAGE107 [Japanese Published Unexamined Patent Application No. 22979/91; Cytotechnology, 3, 133 (1990)], pAS3-3 (Japanese Published Unexamined Patent Application No. 227075/90), pCDM8 [Nature, 329, 840,(1987)], pcDNAI/Amp (manufactured by Invitrogen), pREP4 (manufactured by Invitrogen), pAGE103 [J. Biochemistry, 101, 1307 (1987)], pAGE210, pME18SFL3, pKANTEX93 (WO 97/10354) and the like.

Any promoter can be used, so long as it can function in an animal cell. Examples include a promoter of IE (immediate early) gene of cytomegalovirus (CMV), SV40 early promoter, a promoter of retrovirus, a metallothionein promoter, a heat shock promoter, SRα promoter and the like. Also, the enhancer of the IE gene of human CMV can be used together with the promoter.

The host cell may be any one, so long as it is a cell line in which an activity of an enzyme capable of adding Gal to N-acetylgalactosamine (GalNAc) bound to Ser/Thr on the polypeptide, a protein involved in the activity of the enzyme or a protein involved in the transportation of uridine 5'-diphospate-galactose (UDP-galactose) is decreased or deleted, in the sugar chain synthesis process. Specifically, the host cell may be a Lec8 mutant [ACS Symp. Ser. 128, 214 (1980)], which is a Chinese hamster ovary (CHO) cell devoid of a UDP-galactose transporter.

Further, even though the cell is not deficient in an activity of an enzyme involved in the sugar chain synthesis process, or an activity of the transporter protein, a cell line in which the function of an enzyme or a transporter protein such as UDP-galactose transporter (also referred to as UDP-galactose translocator, UGALT), or core 1 synthase, glycoprotein-n-acetylgalactosamine 3-beta-galactosyltransferase (C1GALT1, also referred to as core 1 beta-3-gal-t, t synthase) or C1GALT1-specific chaperone 1 (c1galt1c1, also referred to as core 1 beta-3-galactosyltransferase-specific molecular chaperone (COSMC), C1GALT2), is decreased or deleted may be used.

Examples of the cell in which an activity of an enzyme involved in the sugar chain synthesis process, or an activity of the transporter protein is not deleted include Namalwa cells, simian COS cells, Chinese hamster-derived (CHO) cells, HBT5637 (Japanese Published Unexamined Patent Application No. 299/88), and the like.
Examples of the method for suppressing the gene function include antisense method, ribozyme method [Proc. Natl. Acad. Sci. U.S.A., 96, 1886 (1999)], homologous recombination method [Manipulating the Mouse Embryo A Laboratory Manual, Second Edition, Cold Spring Harbor Laboratory Press (1994), Gene Targeting, A Practical Approach, IRL Press at Oxford University Press (1993)], RNA-DNA ologonucleotide (RDO) method, RNA interference (RNAi) method [Nature, 391, 806, (1998), Proc. Natl. Acad. Sci. USA 95, 15502, (1998), Nature, 395, 854, (1998), Proc. Natl. Acad. Sci. USA), 96, 5049, (1999), Cell, 95, 1017, (1998), Proc. Natl. Acad. Sci. USA, 96, 1451, (1999), Proc. Natl. Acad. Sci. USA, 95, 13959, (1998), Nature Cell Biol., 2, 70, (2000)], method using retrovirus, method using transposon [Nature Genetics, 25, 35, (2000)], and the like.

Any introduction method of the recombinant vector can be used, so long as it is a method for introducing DNA into an animal cell, and examples include electroporation *[*Cytotechnology, 3, 133 (1990)], the calcium phosphate method (Japanese Published Unexamined Patent Application No. 227075/90), the lipofection method *[*Proc. Natl. Acad. Sci. USA, 84, 7413 (1987)], and the like.
As the expression method of the gene, in addition to direct expression, secretory production, fusion protein expression and the like in accordance with the method described in Molecular Cloning, A Laboratory Manual, Second Edition, Cold Spring Harbor Laboratory Press (1989) can be carried out. When expression is carried out in an eukaryote-derived cell, a polypeptide to which a sugar or a sugar chain is added can be obtained.

The polypeptide used in the present invention can be produced by culturing the thus obtained transformant in a medium to form and accumulate the polypeptide in the culture, and recovering it from the culture. The method for culturing the transformant in the medium is carried out according to the common method used in culturing hosts.
When a microorganism transformed with a recombinant vector containing an inducible promoter as a promoter is cultured, an inducer can be added to the medium, if necessary. For example, isopropyl-β-D-thiogalactopyranoside or the like can be added to the medium when a microorganism transformed with a recombinant vector using *lac* promoter is cultured; or indoleacrylic acid or the like can be added thereto when a microorganism transformed with a recombinant vector using trp promoter is cultured.

The media for culturing a transformant obtained with an animal cell as the host cell include generally used RPMI 1640 medium [The Journal of the American Medical Association, 199, 519 (1967)], Eagle's MEM medium *[*Science, 122, 501 (1952)], Dulbecco's modified MEM medium [Virology, 8, 396 (1959)] and 199 medium *[*Proceeding of the Society for the Biological Medicine, 73, 1 (1950)], the media to which fetal calf serum, *etc.* is added, and the like. The culturing is carried out generally at a pH of 6 to 8 and 30 to 40°C for 1 to 7 days in the presence of 5% CO₂. If necessary, an antibiotic such as kanamycin or penicillin can be added to the medium during the culturing.

As described above, the polypeptide used in the present invention can be produced by culturing a transformant derived from a microorganism, an animal cell or the like which comprises a recombinant vector into which a DNA encoding the polypeptide used in the present invention is inserted, in accordance with a common culturing method, to thereby form and accumulate the polypeptide, and then recovering the polypeptide from the culture.
As the gene expression method, in addition to direct expression, secretory production, fusion protein expression and the like can be carried out according to the method described in Molecular Cloning, A Laboratory Manual, Second Edition, Cold Spring Harbor Laboratory Press (1989).

The process for producing the polypeptide includes a method of intracellular expression in a host cell, a method of extracellular secretion from a host cell, a method for producing on a host cell membrane outer envelope, and the like. The appropriate method can be selected by changing the host cell used and the structure of the polypeptide produced.
When the polypeptide is produced in a host cell or on a host cell membrane outer envelope, the gene product can be positively secreted extracellularly in accordance with the method of Paulson et al. [J. Biol. Chem., 264, 17619 (1989)], the method of Lowe et al. [Proc. Natl. Acad. Sci. USA, 86, 8227 (1989), Genes Develop., 4, 1288 (1990)], the methods described in Japanese Published Unexamined Patent Application No. 336963/93 and WO 94/23021, and the like.

Also, the production amount can be increased in accordance with the method described in Japanese Published Unexamined Patent Application No. 227075/90 utilizing a gene amplification system using a dihydrofolate reductase gene.
The polypeptide can be isolated and purified from the above culture, for example, as follows.
When the polypeptide is intracellularly expressed as a soluble form, the post-cultured cells are collected by centrifugation, suspended in an aqueous buffer and then homogenated using ultrasonicator, French press, Manton Gaulin homogenizer, dynomill or the like to obtain a cell-free extract. Using a supernatant derived by centrifugation of the cell-free extract, a purified preparation can be obtained by a general enzyme isolation and purification techniques such as solvent extraction; salting out with ammonium sulfate *etc.*; desalting; an organic solvent precipitation; anion exchange chromatography using a resin such as diethylaminoethyl (DEAE)-sepharose, DIAION HPA-75 (manufactured by Mitsubishi Chemical); cation exchange chromatography using a resin such as S-Sepharose FF (manufactured by Pharmacia); hydrophobic chromatography using a resin such as butyl-Sepharose or phenyl-Sepharose; gel filtration using a molecular sieve; affinity chromatography; chromatofocusing; electrophoresis such as isoelectric focusing; and the like which may be used alone or in combination.

When the polypeptide is expressed intracellularly by forming an inclusion body, the cells are collected, homogenated and centrifuged in the same manner, and the inclusion body of the polypeptide are collected as a precipitation fraction. The collected inclusion body of the protein is solubilized with a protein denaturing agent. The protein is turned back into a normal three-dimensional structure by diluting or dialyzing the solubilized solution, and then a purified product of the polypeptide is obtained by the same isolation purification method as above.

Also, the polypeptide used in the present invention can be produced by a chemical synthesis method, such as Fmoc (fluorenylmethyloxycarbonyl) method or tBoc (t-butyloxycarbonyl) method. Also, it can be chemically synthesized using a peptide synthesizer manufactured by Advanced ChemTech, Perkin-Elmer, Pharmacia, Protein Technology Instrument, Synthecell-Vega, PerSeptive, Shimadzu Corporation, or the like.

### (2) Immunization of animal and preparation of antibody-producing cell

A mouse, rat or hamster 3 to 20 weeks old is immunized with the antigen prepared above, and antibody-producing cells are collected from the spleen, lymph node or peripheral blood of the animal. Also, when the increase of a sufficient titer in the above animal is not found due to low immunogenicity, a CD27 knockout mouse may be used as an animal to be immunized.

The immunization is carried out by administering the antigen to the animal through subcutaneous, intravenous or intraperitoneal injection together with an appropriate adjuvant (for example, complete Freund's adjuvant, combination of aluminum hydroxide gel with pertussis vaccine, or the like). When a partial peptide is used as the antigen, a conjugate with a carrier protein such as BSA (bovine serum albumin), KLH (keyhole limpet hemocyanin) or the like is produced to use as an immunogen.

The administration of the antigen is carried out 5 to 10 times every one week or every two weeks after the first administration. On the 3rd to 7th day after each administration, a blood sample is collected from the eyeground venous plexus to determine an antibody titer of the serum by enzyme immunoassay *[Antibodies-A Laboratory Manual* (Cold Spring Harbor Laboratory, 1988)] or the like. A mouse, rat or hamster showing a sufficient antibody titer in their sera against the antigen used for the immunization is used as a donor of antibody-producing cells.

In fusion of the antibody-producing cells and myeloma cells, on the 3rd to 7th days after final administration of the antigen, tissue containing the antibody-producing cells such as the spleen from the immunized mouse, rat or hamster is excised to collect the antibody-producing cell. When spleen cells are used, the spleen is cut out in an MEM medium (Nissui Pharmaceutical) and loosened by tweezers and centrifuged (at 1200 rpm, for 5 minutes). Then, the supernatant is discarded and a Tris-ammonium chloride buffer (pH. 7.65) is applied for 1 to 2 minutes to remove erythrocytes. After washing 3 times with the MEM medium, antibody-producing cells for fusion are provided.

### (3) Preparation of myeloma cells

Cell lines established from a mouse are used as myeloma cells. Examples include 8-azaguanine-resistant mouse myeloma cell line (derived from BALB/c mouse) P3-X63Ag8-U1 (P3-U1) *[*Current Topics in Microbiology and Immunology, 18, 1-7 (1978)], P3-NS1/1-Ag41 (NS-1) *[*European J. Immunology, 6, 511-519 (1976)], SP2/0-Ag14 (SP-2) *[*Nature, 276, 269-270 (1978)], P3-X63-Ag8653 (653) [J. Immunology, 123, 1548-1550 (1979)], P3-X63-Ag8 (X63) *[*Nature, 256, 495-497 (1975)] and the like. These cell lines are subcultured in an 8-azaguanine medium [RPMI1640 medium containing glutamine (1.5 mM), 2-mercaptoethanol (5×10⁻⁵ M), gentamicin (10 µg/ml) and fetal calf serum (FCS) (hereinafter referred to as "normal medium"), followed by further addition of 8-azaguanine (15 µg/ml)] and then cultured in the normal medium 3 or 4 days before cell fusion to ensure the cell number of 2×10⁷ or more on the day for fusion.

### (4) Cell fusion

The above-described antibody-producing cells and myeloma cells were sufficiently washed with an MEM medium or PBS (1.83 g of disodium hydrogen phosphate, 0.21 g of potassium dihydrogen phosphate, 7.65 g of sodium chloride, 1 liter of distilled water, pH 7.2) and mixed to give a ratio of the antibody-producing cells : the myeloma cells = 5 to 10 : 1, followed by centrifugation (1200 rpm, 5 minutes). Then, the supernatant is discarded, and precipitated cell clumps are sufficiently loosened. To 10⁸ of the antibody-producing cells, 0.2 to 1 mL of a mixture solution of 2 g of polyethylene glycol-1000 (PEG-1000), 2 mL of MEM and 0.7 mL of dimethylsulfoxide is added under stirring at 37°C, and 1 to 2 mL of MEM medium is added several times every one or two minutes, and MEM medium is added to give a total amount of 50 mL. After centrifugation (900 rpm, 5 minutes), the supernatant is discarded, the cells are gently loosen, and the cells are gently suspended in 100 mL of HAT medium [a normal medium containing hypoxanthine (10⁻⁴ mol/l), thymidine (1.5×10⁻⁵ mol/l) and aminopterin (4×10⁻⁷ mol/l)] by suction and sucking out using a measuring pipette. The suspension is dispensed at 100 µl/well onto a 96-well culturing plate and cultured in a 5% CO₂ incubator at 37°C for 7 to 14 days.

After the culturing, a portion of the culture supernatant is sampled and a hybridoma which is reactive to an antigen containing the polypeptide used in the present invention and is not reactive to an antigen which does not contain the polypeptide is selected by binding assay or the like as described below.
Then, cloning is carried out twice by a limiting dilution method [in the first round, HT medium (HAT medium without aminopterin) is used, and in the second round, the normal medium is used], and a hybridoma which shows a stably high antibody titer is selected as the monoclonal antibody-producing hybridoma.

### (5) Preparation of monoclonal antibody

The hybridoma cells producing an anti-CD27 monoclonal antibody obtained in (4) are administered by intraperitoneal injection into 8- to 10-week-old mice or nude mice pre-treated with pristane (0.5 ml of 2,6,10,14-tetramethylpentadecane (Pristane) is intraperitoneally administered, followed by feeding for 2 weeks) at a dose of 2×10⁶ to 5×10⁷ cells/animal. The hybridoma forms ascites tumor in 10 to 21 days. The ascitic fluid is collected from the mice, centrifuged (at 3,000 rpm, for 5 minutes) to remove solids, subjected to salting out with 40 to 50% saturated ammonium sulfate and then precipitated by caprylic acid, passed through a DEAE-Sepharose column, a protein A column or a gel filtration column to collect IgG or IgM fractions as purified monoclonal antibodies.

The subclass of the antibody can be determined using a subclass typing kit by enzyme immunoassay. The amount of the protein can be calculated by the Lowry method or from the absorbance at 280 nm.

### (6) Binding assay

As the antigen, a gene-introduced cell or a recombinant protein obtained by introducing an expression vector containing a cDNA encoding CD27 polypeptide used in the present invention into *Escherichia coli,* yeast, an insect cell, an animal cell or the like, or a purified polypeptide or a partial peptide obtained from a human tissue is used. When a partial peptide is used as the antigen, a conjugate with carrier proteins such as BSA (bovine serum albumin), KLH (keyhole limpet hemocyanin) or the like is prepared and is used.

After immobilizing these antigens as a solid layer by dispensing in a 96-well plate, a serum of an animal to be immunized, a culture supernatant of a monoclonal antibody-producing hybridoma or a purified antibody is dispensed therein as the primary antibody and allowed to react. After thoroughly washing with PBS or PBS-0.05% Tween, an anti-immunoglobulin antibody labeled with biotin, an enzyme, a chemiluminescent material, a radiation compound or the like is dispensed therein as the secondary antibody and allowed to react. After thoroughly washing with PBS-Tween, the reaction depending on the label substance of the secondary antibody is carried out.

The antibody which competes with the thus obtained monoclonal antibody for its binding to the heavy chain hinge region of CD27 can be prepared by adding a test antibody to the above-mentioned binding assay system and carrying out reaction. That is, a monoclonal antibody which competes with the thus obtained monoclonal antibody for its binding to the heavy chain hinge region of the sugar chain-deficient IgA1 can be prepared by screening of an antibody which the binding of the monoclonal antibody is inhibited when the test antibody to be tested is added.

In addition, an antibody which binds to an epitope which is recognized by a monoclonal antibody that recognizes the sugar chain-deficient IgA1 and binds to the heavy chain hinge region thereof, may be obtained by identifying an epitope of the antibody obtained using the above-mentioned binding assay system, and constructing a partial sugar chain binding peptide of the identified epitope, or a sugar chain binding peptide mimicking a three-dimensional structure of the epitope or the like, followed by immunization.

### 2. Preparation of recombinant antibodies

As production examples of recombinant antibodies, the methods for producing a human chimeric antibody and a humanized antibody are shown below.

### (1) Construction of an expression vector for a recombinant antibody

A vector for expression of recombinant antibody is an expression vector for animal cell into which DNAs encoding CH and CL of a human antibody have been inserted, and is constructed by cloning each of DNAs encoding CH and CL of a human antibody into an expression vector for animal cell.

The C region of a human antibody may be CH and CL of any human antibody. Examples include CH belonging to γ1 subclass, CL belonging to κ class, and the like. As the DNAs encoding CH and CL of a human antibody, a chromosomal DNA comprising an exon and an intron or cDNA can be used. As the expression vector for animal cell, any expression vector can be used, so long as a gene encoding the C region of a human antibody can be inserted thereinto and expressed therein. Examples include pAGE107 [Cytotechnol., 3, 133 (1990)], pAGE103 *[*J Biochem., 101, 1307 (1987)], pHSG274 [Gene, 27, 223 (1984)], pKCR [Proc. Natl. Acad. Sci. USA, 78, 1527 (1981)], pSG1bd2-4 [Cytotechnol., 4, 173 (1990)], pSE1UK1Sed1-3 [Cytotechnol., 13, 79 (1993)] and the like. Examples of promoters and enhancers used for an expression vector for animal cells include an SV40 early promoter [J Biochem., 101, 1307 (1987)], a Moloney mouse leukemia virus LTR *[*Biochem. Biophys. Res. Commun., 149, 960 (1987)], an immunoglobulin H chain promoter *[*Cell, 41, 479 (1985)] and enhancer *[*Cell, 33, 717 (1983)] and the like.

The expression vector for a recombinant antibody may be either of a type in which a gene encoding an antibody H chain and a gene encoding an antibody L chain exist on separate vectors or of a type in which both genes exist on the same vector (tandem type). In respect of easiness of construction of an expression vector for a recombinant antibody, easiness of introduction into animal cells, and balance between the expression amounts of antibody H and L chains in animal cells, a tandem type of the expression vector for a recombinant antibody is more preferred [J. Immunol. Methods, 167, 271 (1994)]. Examples of the tandem type of the vector for expression of recombinant antibody include pKANTEX93 (WO 97/10354), pEE18 [Hybridoma, 17, 559 (1998)], and the like.

### (2) Obtaining of cDNAs encoding V regions of an antibody derived from a non-human animal and analysis of amino acid sequences

cDNAs encoding VH and VL of an antibody derived from a non-human animal are obtained as follows.
mRNA is extracted from hybridoma cells producing a non-human animal antibody to synthesize cDNA. The synthesized cDNA is cloned into a vector such as a phage or a plasmid, to prepare a cDNA library. Each of a recombinant phage or recombinant plasmid containing cDNA encoding VH or VL is isolated from the library using DNA encoding a part of the C region or V region of an antibody derived from a non-human animal as a probe. The full length of the nucleotide sequences of VH and VL of the antibody derived from a non-human animal of interest on the recombinant phage or recombinant plasmid are determined, and the full length of the amino acid sequences of VH and VL are deduced from the nucleotide sequences.

The non-human animal may be any one such as mouse, rat, hamster or rabbit, so long as a hybridoma cell can be produced therefrom.
For preparing total RNA from a hybridoma cell, a guanidine thiocyanate-cesium trifluoroacetate method [Methods in Enzymol., 154, 3 (1987)] or the like may be used. For preparing mRNA from total RNA, an oligo (dT) immobilized cellulose column method [Molecular Cloning, A Laboratory Manual, Second Edition, Cold Spring Harbor Laboratory Press (1989)] or the like may be used. Also, examples of a kit for preparing mRNA from a hybridoma cell include Fast Track mRNA Isolation Kit (manufactured by Invitrogen), Quick Prep mRNA Purification Kit (manufactured by Pharmacia) and the like.
For synthesizing cDNA and preparing a cDNA library, publically-known methods [Molecular Cloning, A Laboratory Manual, Cold Spring Harbor Lab. Press (1989); Current Protocols in Molecular Biology, Supplement 1-34]; a method using a commercially available kit such as Super Script™ Plasmid System for cDNA Synthesis and Plasmid Cloning (manufactured by GIBCO BRL), ZAP-cDNA Kit (manufactured by Stratagene), *etc.*; or the like may be used.

As the vector into which the synthesized cDNA using mRNA extracted from a hybridoma cell as the template is inserted for preparing a cDNA library, any vector can be used, so long as the cDNA can be inserted. Examples include ZAP Express [Strategies, 5, 58 (1992)], pBluescript II SK(+) [Nucleic Acids Research, 17, 9494 (1989)], λzapII (manufactured by Stratagene), λgt10 and λgt11 [DNA Cloning: A Practical Approach, I, 49 (1985)], Lambda BlueMid (manufactured by Clontech), λExCell and pT7T3 18U (manufactured by Pharmacia), pcD2 [Mol. Cell. Biol., 3, 280 (1983)], pUC18 [Gene, 33, 103 (1985)], and the like.

Any *Escherichia coli* for introducing the cDNA library constructed by a phage or plasmid vector may be used, so long as the cDNA library can be introduced, expressed and maintained. Examples include XL1-Blue MRF' [Strategies, 5, 81 (1992)], C600 *[*Genetics, 39, 440 (1954)], Y1088 and Y1090 *[*Science, 222: 778 (1983)], NM522 [J. Mol. Biol., 166, 1 (1983)], K802 *[*J. Mol. Biol., 16, 118 (1966)], JM105 [Gene, 38, 275 (1985)], and the like.

A colony hybridization or plaque hybridization method using an isotope- or fluorescence-labeled probe may be used for selecting cDNA clones encoding VH or VL of an antibody derived from a non-human animal from the cDNA library [Molecular Cloning, A Laboratory Manual, Second Edition, Cold Spring Harbor Laboratory Press (1989)]. Also, the cDNAs encoding VH and VL can be prepared through polymerase chain reaction (hereinafter referred to as "PCR"; Molecular Cloning, A Laboratory, Manual, Second Edition, Cold Spring Harbor Laboratory Press (1989); Current Protocols in Molecular Biology, Supplement 1-34) by preparing primers and using cDNA prepared from mRNA or a cDNA library as the template.

The nucleotide sequence of the cDNA can be determined by digesting the cDNA selected by the above method with appropriate restriction enzymes or the like, cloning the fragments into a plasmid such as pBluescript SK(-) (manufactured by Stratagene), carrying out the reaction by a generally-used method of nucleotide sequence analysis such as the dideoxy method of Sanger, F. et al. [Proc. Natl. Acad. Sci. USA, 74, 5463 (1977)], and then analyzing the sequence using an automatic nucleotide sequence analyzer such as A.L.F. DNA sequencer (manufactured by Pharmacia).

Whether the obtained cDNAs encode the complete amino acid sequences of VH and VL of the antibody containing a secretory signal sequence can be confirmed by estimating the whole amino acid sequences of VH and VL from the determined nucleotide sequence and comparing them with the whole amino acid sequences of VH and VL of known antibodies *[*Sequences of Proteins of Immunological Interest, US Dept. Health and Human Services (1991)]. The length of the secretory signal sequence and N-terminal amino acid sequence can be deduced by comparing the full length of the amino acid sequences of VH and VL of the antibody comprising a secretory signal sequence with the whole amino acid sequences of VH and VL of known antibodies *[*Sequences of Proteins of Immunological Interest, US Dept. Health and Human Services (1991)], and the subgroup to which they belong can also be known. Furthermore, the amino acid sequence of each of CDRs of VH and VL can be identified by comparing with amino acid sequences of VH and VL of known antibodies *[*Sequences of Proteins of Immunological Interest, US Dept. Health and Human Services (1991)].

Moreover, the novelty of the sequences to be used can be certified by carrying out a homology search such as the BLAST method [J. Mol. Biol., 215, 403 (1990)] or the like with the complete amino acid sequences of VH and VL in arbitrary database such as SWISS-PROT, PIR-Protein.

### (3) Construction of an expression vector for a human chimeric antibody

cDNAs encoding VH and VL of a non-human animal antibody are cloned in the upstream of genes encoding CH and CL of human antibody of an expression vector for the recombinant antibody mentioned in the above 2(1) to thereby construct an expression vector for a human chimeric antibody. In order to ligate a 3'-terminal of cDNA encoding VH or VL of non-human animal antibody to 5'-terminal of CH or CL of a human antibody, each cDNA encoding VH and VL is constructed so that a nucleotide sequence of a linkage portion would encode appropriate amino acids and have an appropriate recognition sequence of a restriction enzyme. An expression vector for human chimeric antibody is constructed by cloning the obtained cDNAs encoding VH and VL respectively, in upstream of gene encoding CH or CL of a human antibody of the expression vector of the humanized antibody mentioned in the above 2(1), so that each of them is expressed in an appropriate form. In addition, cDNA encoding VH and VL or non-human antibody is amplified respectively by PCR using a synthetic DNA having a recognition sequence of an appropriate restriction enzyme at both terminals and each of them is cloned to the expression vector for the recombinant antibody mentioned in the above 2(1).

### (4) Construction of cDNAs encoding V regions of a humanized antibody

A cDNA encoding VH or VL of a humanized antibody can be obtained as follows. First, amino acid sequences of framework regions (hereinafter referred to as "FR") in VH or VL of a human antibody to which amino acid sequences of CDRs in VH or VL of an antibody derived from a non-human antibody are grafted are selected. Any amino acid sequences of FRs in VH or VL of a human antibody can be used, so long as they are from human. Examples include amino acid sequences of FRs in VH or VL of human antibodies registered in database such as Protein Data Bank or the like, and amino acid sequences common to subgroups of FRs in VH or VL of human antibodies *[*Sequences of Proteins of Immunological Interest, US Dept. Health and Human Services (1991)], and the like. In order to reduce loss of the binding activity of the antibody, amino acid sequences having high homology (at least 60% or more) with the amino acid sequence of FRs in VH or VL of the original antibody is selected. Then, amino acid sequences of CDRs of VH or VL of the original antibody are grafted to the selected amino acid sequence of FRs in VH and VL of the human antibody, respectively, to design each amino acid sequence of VH and VL of a humanized antibody. The designed amino acid sequences are converted to DNA sequences by considering the frequency of codon usage found in nucleotide sequences of genes of antibodies [Sequence of Proteins of Immunological Interest, US Dept. Health and Human Services (1991)], and the DNA sequence encoding the amino acid sequences of VH and VL of a humanized antibody are designed respectively. Based on the designed nucleotide sequences, several synthetic DNAs consisting of a length of about 100 nucleotides are synthesized, and PCR is carried out using them. In this case, it is preferred in each of the H chain and the L chain that 6 synthetic DNAs are designed in view of the reaction efficiency of PCR and the lengths of DNAs which can be synthesized.

Furthermore, the cDNA encoding VH or VL of a humanized antibody can be easily cloned into the expression vector for the humanized antibody constructed in the (1) of this item 2 by introducing the recognition sequence of an appropriate restriction enzyme to each 5' terminal of the synthetic DNAs on the both ends. After the PCR, each amplified product is cloned into a plasmid such as pBluescript SK (-) (manufactured by Stratagene) or the like, and the nucleotide sequence is determined according to the method described in (2) of this item 2 to obtain a plasmid comprising a DNA sequence encoding the amino acid sequence of VH or VL of a desired humanized antibody.

### (5) Modification of amino acid sequence of the V region of a humanized antibody

It is known that by simply grafting only CDRs in VH and VL of a non-human antibody into FRs of VH and VL of a human antibody, the antigen binding activity decreases compared to the original non-human antibody [BIO/TECHNOLOGY, 9, 266 (1991)]. As the reason, it is considered that not only CDRs but also several amino acid residues of FRs directly or indirectly relate to antigen binding activity in VH and VL of the non-human original antibody, and that grafting of CDRs, which leads to replacement the amino acid residues of FRs of a non-human antibody to the amino acid residues of FRs of a human antibody, decreases the antigen binding activity. In order to solve the problem, in humanized antibodies, among the amino acid sequences of FRs in VH and VL of a human antibody, amino acid residues which directly relate to binding to an antigen, amino acid residues which interact with amino acid residues of CDRs, or amino acid residues which indirectly relate to binding to an antigen by maintaining the three-dimensional structure of an antibody is identified and modified to amino acid residues of the non-human original antibody to thereby increase the antigen binding activity which has been decreased [BIO/TECHNOLOGY, 2, 266 (1991)]. In the production of a humanized antibody, so as to efficiently identify the amino acid residues of FRs relating to the antigen binding activity, the three-dimensional structure of an antibody is constructed and analyzed by X-ray crystallography [J. Mol. Biol., 112, 535 (1977)], computer-modeling [Protein Engineering, 7, 1501 (1994)] or the like. Although these information of the three-dimensional structure of antibodies has provided much useful information in the production of a humanized antibody, no method for producing a humanized antibody which can be applied to any antibodies has been established yet. Therefore, various attempts must be currently be necessary, for example, several modified antibodies of each antibody are produced and the correlation between each of the modified antibodies and its antibody binding activity is examined.

The modification of the amino acid sequence of FR in VH and VL of a human antibody can be accomplished using various synthetic DNA for modification according to PCR as described in (4) of this item 2. With regard to the amplified product obtained by the PCR, the nucleotide sequence is determined according to the method as described in (2) of this item 2 so that whether the designed modification has been carried out is confirmed.

### (6) Construction of an expression vector for a humanized antibody

An expression vector for a humanized antibody can be constructed by cloning each cDNA encoding VH or VL of a constructed recombinant antibody into upstream of each gene encoding CH or CL of the human antibody in the expression vector for the humanized antibody as described in (1) of this item 2.

For example, by introducing recognition sequences of appropriate restriction enzymes onto the 5'-terminals of synthetic DNAs, which are selected to be positioned at both ends among synthetic DNAs used for the construction of VH or VL of the humanized antibody in (4) and (5) of this item 2, VH and VL can be cloned into the upstream of each gene encoding CH or CL of the human antibody in the expression vector for the humanized antibody as described in (1) of this item 2 so that they are expressed in an appropriate form.

### (7) Transient expression of a recombinant antibody

The antigen binding activity of various humanized antibodies produced can be efficiently evaluated by transiently expressing the recombinant antibodies using the expression vector for the humanized antibody as described in (3) and (6) of this item 2 or the modified expression vector thereof. Any cell can be used as a host cell, so long as the host cell can express a recombinant antibody. Examples include COS-7 cell (ATCC CRL1651) is used in view of its high expression amount *[*Methods in Nucleic Acids Res., CRC Press, 283 (1991)]. Examples of the method for introducing the expression vector into COS-7 cell include a DEAE-dextran method [Methods in Nucleic Acids Res., CRC Press, 283 (1991)], a lipofection method [Proc. Natl. Acad. Sci. USA, 84, 7413 (1987)], and the like.

After introduction of the expression vector, the expression amount and antigen binding activity of the recombinant antibody in the culture supernatant can be determined by the enzyme immunoassay [hereinafter referred to as "ELISA"; Monoclonal Antibodies-Principles and practice, Third edition, Academic Press (1996), Antibodies-A Laboratory Manual, Cold Spring Harbor Laboratory (1988), Monoclonal Antibody Experiment Manual, Kodansha Scientific (1987)] and the like.

### (8) Stable expression of recombinant antibody

A transformant which stably expresses a recombinant antibody can be obtained by introducing the expression vector for the recombinant antibody described in (3) and (6) of this item 2 into an appropriate host cell.
Examples of the methods for introducing the expression vector into a host cell include electroporation [Japanese Published Unexamined Patent Application No. 257891/90, Cytotechnology, 3, 133 (1990)] and the like.

As the animal cell into which a vector for expression of recombinant is introduced, any cell can be used, so long as it is an animal cell which can produce the recombinant antibody. Examples include mouse SP2/0-Ag14 cell (ATCC CRL1581), mouse P3X63-Ag8.653 cell (ATCC CRL1580), CHO/dhFr- cell (ATCC CRL9096) and CHO/DG44 cell [Somatic Cell and Molecular Genetics, 12,555(1986)], both of which are two kinds of chinese hamster ovary cells, lection resistance-acquired Lec13 cell *[*Somatic Cell and Molecular genetics, 12, 55 (1986)], CHO cell in which α1,6-fucosyltransaferse gene is defected (WO 05/35586), rat YB2/3HL.P2.G11.16Ag.20 cell (ATCC CRL1662), and the like.

In addition to the above host cells, host cells in which activity of a protein such as an enzyme relating to synthesis of an intracellular sugar nucleotide, GDP-fucose, a protein such as an enzyme relating to the modification of a sugar chain in which 1-position of fucose is bound to 6-position of N-acetylglucosamine in the reducing end through α-bond in a complex type N-glycoside-linked sugar chain, or a protein relating to transport of an intracellular sugar nucleotide, GDP-fucose, to the Golgi body are introduced is decreased or deleted, preferably CHO cell deficient in α1,6-fucosyltransferase gene as described in WO 05/35586, WO 02/31140 or the like, can also be used.

After introduction of the expression vector, transformants which stably express a recombinant antibody are selected in accordance with the method disclosed in Japanese Published Unexamined Patent Application No. 257891/90, by culturing in an medium for animal cell culture medium containing an agent such as G418 sulfate (hereinafter referred to as "G418", manufactured by Sigma) or the like. Examples of animal cell culture include RPMI1640 medium (manufactured by Invitrogen), GIT medium (manufactured by Nissui Pharmaceutical), EX-CELL301 medium (manufactured by JRH), IMDM medium (manufactured by Invitrogen), Hybridoma-SFM medium (manufactured by Invitrogen), media thereby containing various additives such as fetal calf serum (hereinafter referred to as "FCS"), and the like. The recombinant antibody can be produced and accumulated in a culture supernatant by culturing the obtained transformants in a medium. The expression amount and antigen binding activity of the recombinant antibody in the culture supernatant can be measured by ELISA or the like. Also, in the transformant, the expression amount of the recombinant antibody can be increased by using DHFR amplification system disclosed in Japanese Published Unexamined Patent Application No. 257891/90, or the like.

The recombinant antibody can be purified from the culture supernatant of the transformant by a protein A column [Monoclonal Antibodies-Principles and practise, Third edition, Academic Press (1996), Antibodies-A Laboratory Manual, Cold Spring Harbor Laboratory (1988)]. Any other conventional methods for protein purification can be used. For example, gel filtration, ion-exchange chromatography, ultrafiltration and the like can be combined for purification. The molecular weight of the H chain or the L chain of the purified recombinant antibody or the whole antibody molecule is determined by polyacrylamide gel electrophoresis (hereinafter referred to as "SDS-PAGE") *[*Nature, 227, 680 (1970)], Western blotting [Monoclonal Antibodies-Principles and practice, Third edition, Academic Press (1996), Antibodies-A Laboratory Manual, Cold Spring Harbor Laboratory (1988)], and the like.

### 3. Activity evaluation of the antibodies or antibody fragments of the present invention

Reaction specificity of the purified antibody or antibody fragment of the present invention can be evaluated in the following procedure.

Using a cell expressing a normal sugar chain, and a cell line in which an activity of an enzyme for addition of Gal to GalNAc bound to Ser/Thr on the polypeptide, a protein affecting the activity of the enzyme or a protein involved in the transportation of uridine 5'-diphospate-galactose (UDP-galactose) is decreased or deleted, in the O-linked sugar chain synthesis process, as a host, IgA1-expressing cells can be respectively produced which express nucleotide sequence encoding IgA1 heavy chain (3). In this manner, a cell expressing IgA1 having a normal O-linked sugar chain, and a cell expressing sugar chain-deficient IgA1 can be constructed, and the reactivity of the cell lines, expressing each of IgA1, with the purified antibody can be measured by ELISA, fluorescent antibody technique [Cancer Immunol. Immunother., 36, 373 (1993)], or the like.

Alternatively, the extracellular region of membrane-bound IgA1 can be expressed as a soluble form such as fusion protein in each of the above-mentioned host cells, and purified under appropriate conditions to prepare respective IgA1 soluble proteins retaining a three-dimensional structure. Examples of the fusion proteins may include a fusion of the IgA1 protein with another polypeptide such as antibody constant region (also referred to as Fc), GST tag, histidine tag (also referred to as His tag) or Myc tag. The fusion protein may be segregated and purified by an affinity column such as Protein A, nickel column, specific antibody column, or the like. The reactivity of the purified CD27 soluble protein with the purified antibody can be measured by surface plasmon resonance (SPR)-aided BIAcoreTM, ELISA, immunoprecipitation or the like method [Monoclonal Antibodies-Principles and Practice, Third edition, Academic Press (1996), Antibodies-A Laboratory Manual, Cold Spring Harbor Laboratory (1988)]. 4. Method for diagnosing a disease using a monoclonal antibody or an antibody fragment of the present invention which specifically recognizes the sugar chain-deficient IgA1 and also binds to the heavy chain hinge region thereof.
A disease relating to the sugar chain-deficient IgA1 can be diagnosed by detecting or quantifying sugar chain-deficient IgA1 or a cell expressing and accumulating the polypeptide, using the antibody or antibody fragment of the present invention.

Diseases relating to a sugar chain-deficient IgA1 protein or to a cell expressing sugar chain-deficient IgA1 include any disease as long as the disease produces a protein comprising the sugar chain-deficient IgA1 polypeptide or a cell expressing the sugar chain-deficient IgA1 polypeptide in the body. The disease is preferably a disease that further increases the amount of the polypeptide expressed in a patient with a disease relating to the sugar chain-deficient IgA1 protein or to the cell expressing sugar chain-deficient IgA1, compared to a healthy person. Specific examples of the disease include an autoimmune disease and cancer. Examples of the autoimmune diseases include chronic glomerulonephritis caused mainly by IgA nephropathy, systemic lupus erythematosus, Henoch-Schonlein purpura, and the like. Examples of the cancer include cancer derived from plasma cells, specifically, plasmocytoma, IgA type myeloma, mantle cell lymphoma, chronic lymphatic leukemia, small lymphocytic leukemia, Burkitt lymphoma, follicular lymphoma, MALT lymphoma, diffuse large-cell lymphoma, plasmacytoma, and the like.

The biological sample to be used for the detection or measurement of a sugar chain-deficient IgA1 polypeptide in the present invention is not particularly limited, so long as it has a possibility of containing the polypeptide, such as tissue cells, blood, blood plasma, serum, pancreatic juice, urine, fecal matter, tissue fluid or culture medium.
Among diseases relating to sugar chain-deficient IgA1, for example, diagnosis of IgA nephropathy can be carried out in the following manner.

With regard to the biological samples collected from the living bodies of multiple healthy subjects, the expression level of the polypeptide in the biological samples of healthy subjects is confirmed by carrying out detection or measurement of a sugar chain-deficient IgA1 polypeptide by the following immunological methods using the antibody or antibody fragment of the present invention or derivatives thereof. The expression level of the polypeptide in the biological samples of the test subjects is also examined in the same manner, to be compared with the expression level in healthy subjects. When the expression level of the polypeptide in the test subjects is increased in comparison with the healthy persons, it can be diagnosed that they have IgA nephropathy or have a greater chance of developing IgA nephropathy..

Among diseases relating to a sugar chain-deficient IgA1, for example, diagnosis of a cancer can be carried out in the following manner.
With regard to the biological samples collected from the living bodies of multiple healthy subjects, the expression level of the polypeptide in the biological samples of healthy subjects is confirmed by carrying out detection or measurement of the sugar chain-deficient IgA1 by the following immunological methods using the antibody or antibody fragment of the present invention or derivatives thereof. The expression level of the polypeptide in the biological samples of the test subjects is also examined in the same manner, to be compared with the expression level in healthy subjects. When the expression level of the polypeptide in the test subjects is increased in comparison with the healthy persons, it can be diagnosed that cancer is positive.

The diagnostic agent containing the antibody or antibody fragment of the present invention or derivatives thereof may further contain a reagent for carrying out an antigen-antibody reaction or a reagent for detection of the reaction depending on the desired diagnostic method. The reagent for carrying out the antigen-antibody reaction includes a buffer, a salt, and the like. The reagent for detection includes a reagent used for common immunological detection or measurement such as antibody or antibody fragment thereof, derivatives thereof, labeled secondary antibody for recognizing the derivatives thereof and substrate corresponding to the labeling.

As a method for detection or measurement of the amount of the sugar chain-deficient IgA1 in the present invention, any known method may be included. For example, an immunological detection method or measurement method may be exemplified.
An immunological detection or measurement means a method in which an antibody amount or an antigen amount is detected or determined using a labeled antigen or antibody. Examples of the immunological detection or measurement are radioactive substance-labeled immunoantibody method (RIA), enzyme immunoassay (EIA or ELISA), fluorescent immunoassay (FIA), luminescent immunoassay, Western blotting method, physico-chemical means (TIA, LAPIA and PCIA) and the like.

Examples of the radioactive substance-labeled immunoantibody method (RIA) include a method, in which the antibody or antibody fragment of the present invention is allowed to react with an antigen or an antigen-expressing cell, then radiolabeled anti-immunoglobulin antibody or a binding fragment thereof is allowed to react therewith, followed by determination using a scintillation counter or the like.
Examples of the enzyme immunoassays (EIA or ELISA) include a method, in which the antibody or antibody fragment of the present invention is allowed to react with an antigen or an antigen-expressing cell, then a labeled anti-immunoglobulin antibody or an binding fragment thereof is allowed to react therewith and the chromogenic pigment is measured by a spectrophotometer, and, for example, sandwich ELISA may be used. As a label used in the enzyme immunoassay, any known enzyme label (Enzyme Immunoassay edited by Eiji Ishikawa, et al., published by Igaku Shoin) can be used as described already. Examples include alkaline phosphatase labeling, peroxidase labeling, luciferase labeling, biotin labeling and the like.

Sandwich ELISA is a method in which an antibody is bound to a solid phase, a target antigen for detection or measurement is trapped and another antibody is allowed to react with the trapped antigen. In the ELISA, after two kinds of antibodies which recognize the target antigen for detection or measurement and have a difference in a recognizing site or the antibody fragments thereof are prepared and an antibody or an antibody fragment is previously fixed on a plate (such as a 96-well plate) and another antibody or antibody fragment is labeled with a fluorescent substance such as FITC, an enzyme such as peroxidase, a biotin or the like. The plate to which the above antibody is fixed is allowed to react with the cell segregated from a living body or homogenated solution thereof, tissue or homogenated solution thereof, cell culture supernatant, serum, pleural effusion, ascites, eye solution or the like, then allowed to react with labeled monoclonal antibody or antibody fragment and a detection reaction corresponding to the labeled substance is carried out. When an antigen concentration in the sample to be tested is measured by the method, antigen concentration in the sample to be tested can be calculated from a calibration curve prepared by a stepwise dilution of antigen of known concentration. As antibodies used for sandwich ELISA, either polyclonal antibodies or monoclonal antibodies may be used. Also, antibody fragments such as Fab, Fab' and F(ab)₂ may be used. As a combination of two kinds of antibodies used in sandwich ELISA, a combination of monoclonal antibodies or antibody fragments recognizing different epitopes may be used or a combination of a polyclonal antibody with a monoclonal antibody or a antibody fragment may be used.

Examples of fluorescent immunoassays (FIA) include a method described in the literatures [Monoclonal Antibodies - Principles and practice, Third Edition, Academic Press (1996); Manual for Monoclonal Antibody Experiments, Kodansha Scientific (1987)] and the like. As a label for the fluorescent immunoassay, any of known fluorescent labels (Fluorescent Immunoassay, by Akira Kawao, Soft Science) may be used as described already. Examples include FITC labeling, RITC labeling and the like.

The luminescent immunoassay can be carried out using the methods described in Monoclonal Antibodies - Principles and practice, Third Edition, Academic Press (1996); Manual for Monoclonal Antibody Experiments, Kodansha Scientific (1987) and the like. As a label for luminescent immunoassay, any of known luminescent labels [Bioluminescence and Chemical Luminescence, Hirokawa Shoten; Rinsho Kensa, 42 (1998)] can be included as described above. Examples include acridinium ester labeling, lophine labeling or the like may be used.

Western blotting can be carried out a method in which an antigen or an antigen-expressing cell is fractionated by SDS-polyacrylamide gel electrophoresis [Antibodies-A Laboratory Manual (Cold Spring Harbor Laboratory, 1988)], the gel is blotted onto PVDF membrane or nitrocellulose membrane, the membrane is allowed to react with antigen-recognizing antibody or antibody fragment, further allowed to react with an anti-mouse IgG antibody or antibody fragment which is labeled with a fluorescent substance such as FITC, an enzyme label such as peroxidase, a biotin labeling, and the label is visualized to confirm the reaction. An example of Western blotting is described below.

Cells or tissues expressing a polypeptide having the amino acid sequence represented by SEQ ID NO:2 are dissolved in a solution and, 0.1 to 30 µg of protein per lane is electrophoresed under reducing conditions by an SDS-PAGE method. The electrophoresed protein is transferred to a PVDF membrane and allowed to react with PBS containing 1% of BSA (hereinafter referred to as "BSA-PBS") at room temperature for 30 minutes for blocking. Here, the monoclonal antibody of the present invention is allowed to react therewith, washed with PBS containing 0.05% Tween 20 (hereinafter referred to as "Tween-PBS") and allowed to react with a peroxidase-labeled goat anti-mouse IgG at room temperature for 2 hours. After washing with Tween-PBS, a band bound by the monoclonal antibody is detected using ECL™ Western Blotting Detection Reagents (manufactured by Amersham) or the like to thereby detect a polypeptide having the amino acid sequence represented by SEQ ID NO:2. As an antibody used for the detection in Western blotting, an antibody which can be bound to a polypeptide having native three-dimensional structure is used.

The physicochemical method is specifically carried out using the antibody or antibody fragment of the present invention by reacting CD27 as the antigen with the antibody or antibody fragment of the present invention to form an aggregate, and detecting this aggregate. Other examples of the physicochemical methods include a capillary method, a one-dimensional immunodiffusion method, an immunoturbidimetry and a latex immunoturbidimetry [Handbook of Clinical Test Methods, Kanehara Shuppan, 499 (1988)].

For example, in a latex immunodiffusion method, a carrier such as polystyrene latex having a particle size of about of 0.1 to 1 µm sensitized with antibody or antigen may be used and when an antigen-antibody reaction is carried out using the corresponding antigen or antibody, scattered light in the reaction solution increases while transmitted light decreases. When such a change is detected as absorbance or integral sphere turbidity, it is now possible to measure antigen concentration, etc. in the sample to be tested.

Since the antibody or antibody fragment of the present invention is capable of binding to an heavy chain hinge region of the sugar chain-deficient IgA1 polypeptide, it is preferably used for detecting a cell expressing the polypeptide.
For the detection of the cell expressing the polypeptide, known immunological detection methods can be used, and an immunoprecipitation method, a fluorescent cell staining method, an immune tissue staining method and the like are preferably used. Also, an immunofluorescent staining method using FMAT 8100 HTS system (Applied Biosystem) and the like can be used.

An immunoprecipitation method can be carried out using a method in which a cell expressing the polypeptide is allowed to react with the monoclonal antibody or antibody fragment of the present invention and then a carrier having specific binding ability to immunoglobulin such as protein G-Sepharose is added so that an antigen-antibody complex is precipitated. Also, the following method can be carried out.
The above-described antibody or antibody fragment of the present invention is solid-phased on a 96-well plate for ELISA and then blocked with BSA-PBS. When the antibody is in a non-purified state such as a culture supernatant of hybridoma cell, anti-mouse immunoglobulin or anti-rat immunoglobulin or protein A or G or the like is previously immobilized to a 96-well plate for ELISA and blocked with BSA-PBS and a culture supernatant of hybridoma cell is dispensed thereto for binding. After BSA-PBS is discarded and the residue is sufficiently washed with PBS, reaction is carried out with a lysate of cells or tissues expressing polypeptide having the amino acid sequence represented by SEQ ID NO:2. An immune precipitate is extracted from the well-washed plate with a sample buffer for SDS-PAGE and detected by the above-described Western blotting.

An immune cell staining method and an immune tissue staining method are immunofluorescent staining methods (a flow cytometry) where antigen-expressing cells or tissues are treated, if necessary, with a surfactant or methanol to increase permeability of an antibody to the cells or tissues, then the antibody of the present invention is allowed to react therewith, then further allowed to react with an anti-immunoglobulin antibody or binding fragment thereof labeled by a fluorescent substrate such as FITC, an enzyme such as peroxidase, a biotin or the like and the label is visualized and observed under a microscope or cells are allowed to react with a fluorescence-labeled antibody and analyzed by a flow cytometer. That can be carried out by the methods described, for example, in the literatures [*Monoclonal Antibodies - Principles and practice,* Third Edition, Academic Press (1996), *Manual for Experiments of Monoclonal Antibodies,* Kodansha Scientific (1987)]. Particularly, since the antibody or antibody fragment of the present invention binds to three-dimensional structure of an heavy chain hinge region of the sugar chain-deficient IgA1, it can be preferably used for detection of a cell expressing the polypeptide maintaining a natural type three-dimensional structure by a flow cytometry.

In addition, by using FMAT8100HTS system (manufactured by Applied Biosystems) which utilizes the principle of fluorescent antibody staining, the antigen amount or antibody amount can be measured without separating the formed antibody-antigen complex from the free antibody or antigen which does not participate in the formation of the antibody-antigen complex.
5. Method for treating disease using the monoclonal antibody or antibody fragment of the present invention which reacts with a sugar chain-deficient IgA1 polypeptide
The monoclonal antibody or the antibody fragment of the present invention which specifically recognizes a sugar chain-deficient IgA1 polypeptide and binds to the heavy chain hinge region thereof can be used for treating a disease relating to a sugar chain-deficient IgA1 polypeptide.

The disease relating to the sugar chain-deficient IgGA1 polypeptide may be any one, so long as it is a disease in which a cell expressing the polypeptide is detected *in vivo.* For example, it may be IgA nephropathy, cancer, or the like.
Further, the disease may also encompass a disease manifesting with nephrose syndrome or renal failure resulting from the development of IgA nephropathy.

Examples of the cancer may include a hematopoietic organ-derived tumor (also referred to as blood cancer) or an epithelial cell-derived solid cancer.
Examples of the blood cancer include, specifically, leukemia, lymphoma (Hodgkin lymphoma, non-Hodgkin lymphoma), multiple myeloma, and the like. Specific examples of the non-Hodgkin lymphoma include mantle cell lymphoma, chronic lymphocytic leukemia, small lymphocytic leukemia, Burkitt's lymphoma, follicular lymphoma, MALT lymphoma, diffuse large B-cell lymphoma, plasmacytoma, and the like.

Specific examples of the solid cancer include breast cancer, uterine cancer, colorectal cancer, stomach cancer, ovarian cancer, lung cancer, renal cancer, rectal cancer, thyroid cancer, uterine cervix cancer, small intestinal cancer, prostate cancer, pancreatic cancer, and the like.
The therapeutic agent of the present invention includes a therapeutic agent for cancer comprising the antibody or antibody fragment of the present invention, as an active ingredient. The therapeutic agent of the present invention also includes a therapeutic agent for cancer having effector activity such as ADCC activity and CDC activity, a therapeutic agent for cancer by an apoptosis-inducing activity and the like.

Since the antibody or antibody fragment of the present invention can recognizes a sugar chain-deficient IgA1 polypeptide expressed on the cell membrane, it can recognize a cell expressing a sugar chain-deficient IgA1 polypeptide *in vivo.* Accordingly, among the antibodies or the antibody fragments of the present invention, the antibody or antibody fragment thereof having effector activity can injure the cell expressing a sugar chain-deficient IgA1 polypeptide *in vivo* and *in vitro.* Also, since the antibody or antibody fragment of the present invention can injure and thereby decrease cells expressing a sugar chain-deficient IgA1 polypeptide *in vivo,* it is particularly effective as a therapeutic agent.

The therapeutic agent comprising the antibody or antibody fragment of the present invention or derivatives thereof may comprise only the antibody or antibody fragment or derivatives thereof as an active ingredient, and is preferably supplied as a pharmaceutical preparation produced by an publically-known method in the technical field of pharmaceutics, by mixing it with one or more pharmaceutically acceptable carriers.
It is preferred to select a route of administration which is most effective in treatment. Examples include oral administration and parenteral administration, such as buccal, tracheal, rectal, subcutaneous, intramuscular or intravenous administration. In the case of an antibody or peptide formulation, intravenous administration is preferred. The dosage form includes sprays, capsules, tablets, granules, syrups, emulsions, suppositories, injections, ointments, tapes and the like.

The pharmaceutical preparation suitable for oral administration includes emulsions, syrups, capsules, tablets, powders, granules and the like. Liquid preparations such as emulsions and syrups can be produced using, as additives, water; sugars such as sucrose, sorbitol and fructose; glycols such as polyethylene glycol and propylene glycol; oils such as sesame oil, olive oil and soybean oil; antiseptics such as p-hydroxybenzoic acid esters; flavors such as strawberry flavor and peppermint; and the like. Capsules, tablets, powders, granules and the like can be produced using, as additives, excipients such as lactose, glucose, sucrose and mannitol; disintegrating agents such as starch and sodium alginate; lubricants such as magnesium stearate and talc; binders such as polyvinyl alcohol, hydroxypropylcellulose and gelatin; surfactants such as fatty acid ester; plasticizers such as glycerin; and the like.

The pharmaceutical preparation suitable for parenteral administration includes injections, suppositories, sprays and the like. Injections can be prepared using a carrier such as a salt solution, a glucose solution or a mixture of both thereof. Suppositories can be prepared using a carrier such as cacao butter, hydrogenated fat or carboxylic acid. Sprays can be prepared using the antibody of the present invention or antibody fragment thereof as such or using it together with a carrier which does not stimulate the buccal or airway mucous membrane of the patient and can facilitate absorption of the compound by dispersing it as fine particles. The carrier includes lactose, glycerol and the like. Depending on the properties of the antibody and the carrier, it is possible to produce pharmaceutical preparations such as aerosols and dry powders. In addition, the components exemplified as additives for oral preparations can also be added to the parenteral preparations.

Although the dose or the frequency of administration varies depending on the objective therapeutic effect, administration method, treating period, age, body weight and the like, it is usually 10 µg/kg to 8 mg/kg per day and per adult.
The present invention is described below by Examples; however, the present invention is not limited to the following Examples.

### Example 1

Establishment of CHO cell line highly expressing sugar chain-deficient IgA1 on cell membrane

### (1) Construction of plasmid pKAN932B8PVHmIgA expressing membrane-bound IgA

In the following procedure, a vector pKAN932B8PVHmIgA for expressing membrane-bound immunoglobulin A on a cell membrane was prepared. This plasmid is a plasmid vector expressing a protein which is obtained by ligating an Fb portion of a heavy chain of anti-CD20 antibody 2B8P disclosed in WO 03/085107 to a constant region of membrane-bound immunoglobulin.

### 1. Preparation of pCR2B8PVH

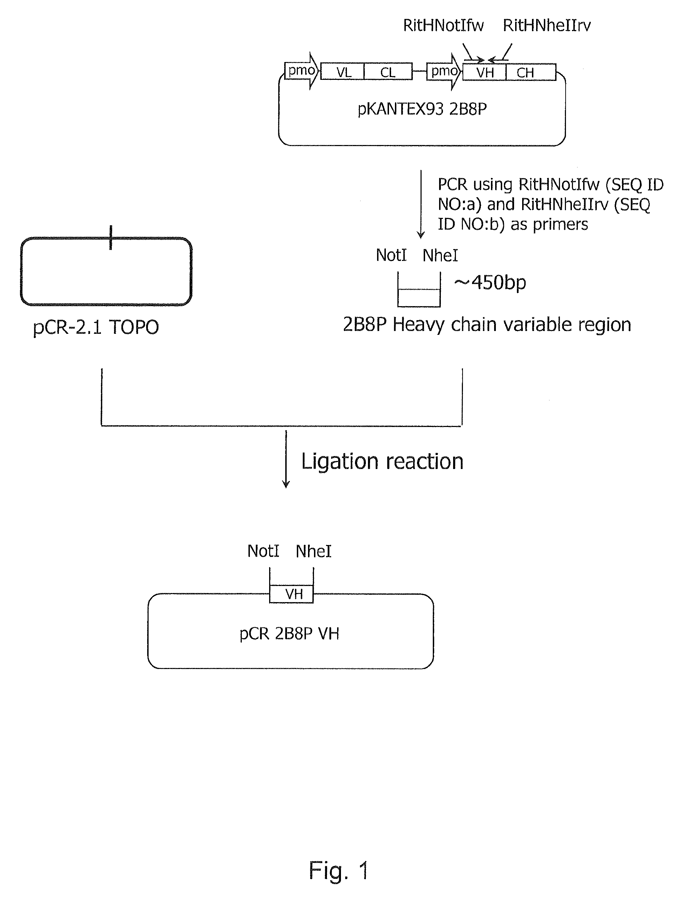

A gene fragment including a heavy chain variable region of an anti-CD20 antibody 2B8P was amplified by the following PCR using a plasmid vector pKANTEX932B8P disclosed in WO 03/085107 as a template. PCR was performed using a reaction solution in total containing 0.2 mmol/L dNTPs and 1 mmol/L magnesium chloride, 1 ng of pKANTEX932B8P, 1 µmol/L RitNotNheIfw (SEQ ID NO:4), 1 µmol/L RitNotNheIrv (SEQ ID NO:5), and 2.5 units of KODpolymerase (manufactured by TOYOBO CO., LTD.) to adjust the volume to 50 µL. The reaction was performed by the reaction condition of 25 cycles consisting of 98°C for 15 seconds, 65°C for 2 seconds, and 74°C for 30 seconds. The reaction solution was separated by 2% agarose gel electrophoresis, and then a PCR product of about 450 bp was inserted into a pCR-Blunt vector using ZeroBlunt PCR Cloning Kit (manufactured by Invitrogen) in accordance with the instructions attached to the kit. In this manner, pCR2B8PVH having the DNA sequence described in SEQ ID NO:6 was obtained (Fig. 1).

### 2. Preparation of pCRIgA

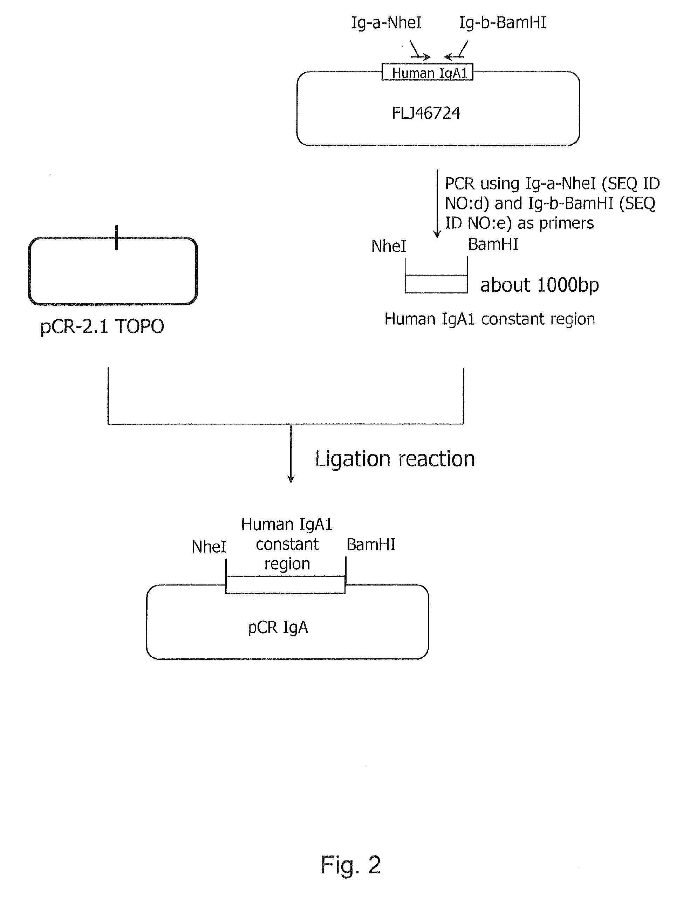

Thereafter, a DNA fragment comprising a constant region of immunoglobulin A was amplified by the following PCR using a plasmid enrolled in Genebank as a Homo sapiens cDNA clone FLJ46724 (distributed by NEDO human cDNA sequencing project) as a template. PCR was performed using a reaction solution in total containing 0.2 mmol/L dNTPs and 1 mmol/L magnesium chloride, a plasmid containing 1 ng of FLJ46724, 1 µmol/L Ig-a-NheI (SEQ ID NO:7), 1 µmol/L Ig-b-BamHI (SEQ ID NO:8), and 2.5 units of KODpolymerase (manufactured by TOYOBO CO., LTD.) to adjust the volume to 50 µL. The reaction was performed by the reaction condition of 25 cycles consisting of 98°C for 15 seconds and 68°C for 30 seconds. The reaction solution was separated by 1% agarose gel electrophoresis, and then a PCR product of about 1000 bp was inserted into a pCR-Blunt vector using ZeroBlunt PCR Cloning Kit (manufactured by Invitrogen) in accordance with the instructions attached to the kit. In this manner, pCRIgA having the DNA sequence described in SEQ ID NO:9 was obtained (Fig. 2).

### 3. Preparation of pCRmIgA

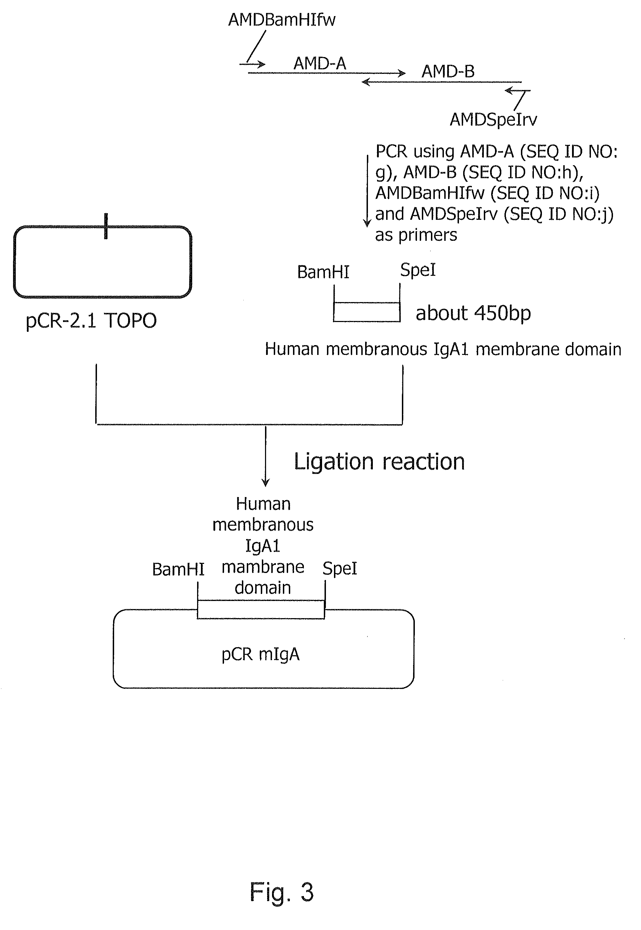

Subsequently, PCR was performed using 50 µL of a reaction solution in total containing 0.2 mmol/L dNTPs, 1 mmol/L magnesium chloride, and using 0.02 µmol/L AMD-A (SEQ ID NO:10), 0.02 µmol/L AMD-B (SEQ ID NO:11), 1 µmol/L AMDBamHIfw (SEQ ID NO:12), 1 µmol/L AMDSpeIrv (SEQ ID NO:13), and 2.5 units of KODpolymerase (manufactured by TOYOBO CO., LTD.). The reaction was performed by the reaction condition of 25 cycles consisting of 98°C for 15 seconds, 65°C for 2 seconds, and 74°C for 30 seconds. The reaction solution was separated by 2% agarose gel electrophoresis, and then a PCR product of about 450 bp was inserted into a pCR-Blunt vector using ZeroBlunt PCR Cloning Kit (manufactured by Invitrogen) in accordance with the instructions attached to the kit. In this manner, pCRmIgA having the DNA sequence described in SEQ ID NO:14 was obtained (Fig. 3).

### 4. Preparation of pCR 2B8P mIgA

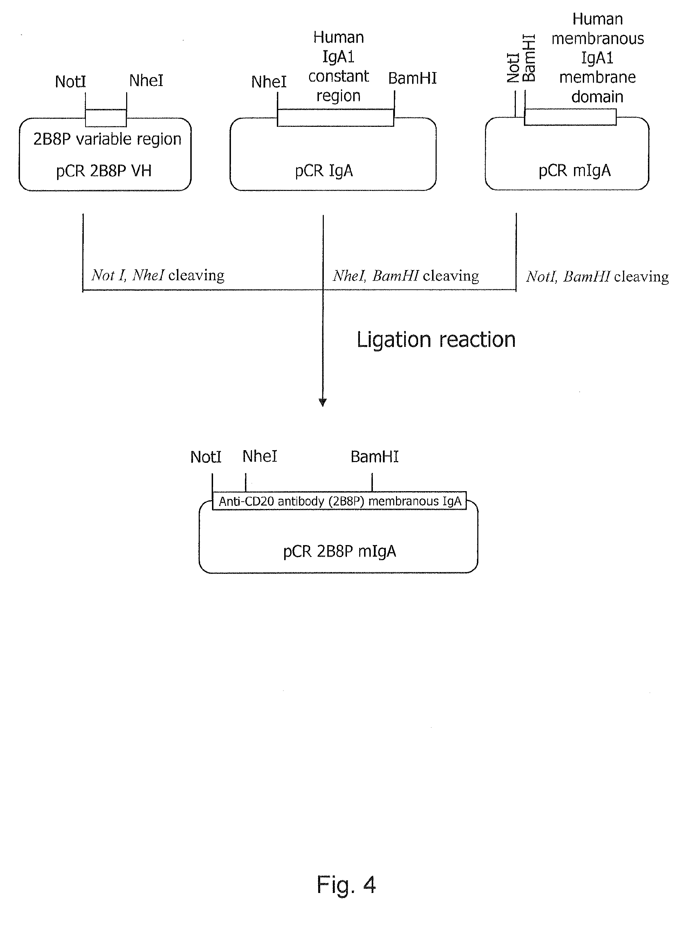

A DNA fragment of about 450 bp that was obtained by digestion of the pCR2BP8PVH prepared in the above section 1 with restriction enzymes *NotI* and *NheI,* and a DNA fragment of about 1000 bp that was obtained by digestion of pCRIgA prepared in the above section 2 with restriction enzymes *NheI* and *BamHI* were ligated to a plasmid DNA of about 3.5 Kbp that was obtained by digestion of the pCRmIgA prepared in the above section 3 with restriction enzymes *NotI* and *BamHI.* In this manner, a plasmid pCR 2B8P mIgA encoding a DNA in which a heavy chain variable region of the anti-CD20 antibody 2B8P was ligated to a heavy chain constant region of a membrane-bound immunoglobulin was constructed (Fig. 4). A membrane-bound IgA1 sequence (heavy chain) encoded with this plasmid are shown in SEQ ID NO:15.

### 5. Construction of pKAN932B8PVHmIgA

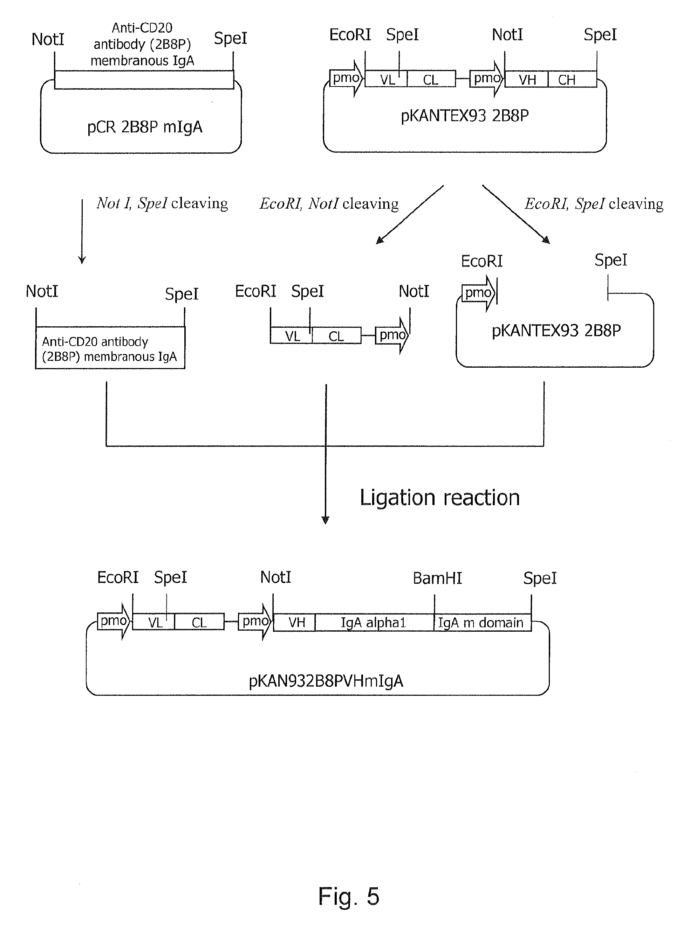

A DNA fragment of 1580 bp that was obtained by digestion of pCR 2B8P mIgA prepared in the above section 4 with restriction enzymes *NotI* and *SpeI* and a DNA fragment of about 2845 bp that was obtained by digestion of KANTEX932B8P with restriction enzymes *EcoRI* and *NotI* were ligated to a DNA fragment of about 13.5 Kbp that was obtained by digestion of pKANTEX932B8P with restriction enzymes *EcoRl* and *SpeI.* In this manner, a plasmid pKAN932B8PVHmIgA for expressing a protein in which a constant region of anti-CD20 antibody 2B8P is membrane-bound immunoglobulin A was prepared (Fig. 5). *Escherichia coli* transformed by this expression vector was seeded in 100 mL of an LB medium and cultured overnight, the bacteria bodies were then collected, and the plasmid was purified using a Qiafilter Plasmid Midi Kit (manufactured by QIAGEN) according to the protocols attached to the kit. After purification, 30 µg of the plasmid vector was linearized by digestion with a restriction enzyme *AatII.* After linearization, phenol/chloroform extraction and ethanol precipitation were performed, followed by dissolution in a TE buffer of a concentration of 1/10 (1 mM TrisHCl, 0.1 mM EDTA). Thereafter, the concentration was measured, and the plasmid was provided to gene introduction.

### (2) Introduction of plasmid pKAN932B8PVHmIgA expressing membrane-bound immunoglobulin A

By introducing pKAN932B8PVHmIgA into Lec8 cells as mutant CHO cells that mainly express a Tn-type sugar chain and into CHO/DG44 cells that mainly express a normal sugar chain, the Lec8 cells and the DG44 cells expressing membrane-bound immunoglobulin A were established. The introduction of the plasmid pKAN932B8PVHmIgA expressing the membrane-bound immunoglobulin A into the CHO/DG44 cells (hereinbelow, described as DG44) or the Lec8 cells was performed in the following procedure based on electroporation [Cytotechnology, 3, 133 (1990)]. First, the DG44 cells subcultured in a basic medium [Iscove's Modified Dulbecco's Medium (Invitrogen) supplemented with 10% dialyzed fetal bovine serum (Invitrogen), 50 µg/mL of Gentamycin (Nacalai Tesque, Inc.), and 1×HT supplement (manufactured by Invitrogen)], were suspended at a density of 8×10⁶ cells/mL in a K-PBS buffer [137 mmol/L KCl, 2.7 mmol/L NaCl, 8.1 mmol/L Na₂HPO₄, 1.5 mmol/L KH₂PO₄, 4.0 mmol/L MgCl₂], thereby preparing a cell suspension. Then, 200 µL (1.8×10⁶ cells) of the prepared cell suspension was mixed with 10 µg of the linearized pKAN932B8PVHmIgA prepared in the above section (1). Here, the Lec8 cells were subcultured in a basic medium (hereinbelow, described as a HT-medium) without 1×HT supplement. The cell-DNA mixture was transferred to Gene Pulser Cuvette (interelectrode distance: 2 mm) (Bio-Rad Laboratories, Inc.), and then gene introduction was performed using a gene introduction device GenePulser (Bio-Rad Laboratories, Inc.) under conditions of a pulse voltage of 0.35 KV and an electric capacity of 250 µF. The cell suspension was mixed with a 30 mL of HT-medium [Iscove's Modified Dulbecco's Medium (manufactured by Invitrogen) supplemented with 10% dialyzed fetal bovine serum (manufactured by Invitrogen) and 50 µg/mL of Gentamycin (Nacalai Tesque, Inc.)] and seeded at 100 µL/well in 3 sheets of 96 well plates. Two days after seeding, the medium was replaced with a subculture medium containing 500 µg/mL G418, followed by culturing for 10 days. Ten days later, the medium was replaced with an HT-medium containing 50 nM MTX (manufactured by Sigma-Aldrich Co. LLC.), thereby obtaining an MTX-resistant line. The membrane-bound immunoglobulin-expressing line derived from the Lec8 line was named mIgA/Lec8, and the membrane-bound immunoglobulin A-expressing cell derived from the DG44 cell was named mIgA/DG44.

### Example 2

### Preparation of sugar chain-deficient IgA1-Fc fusion protein

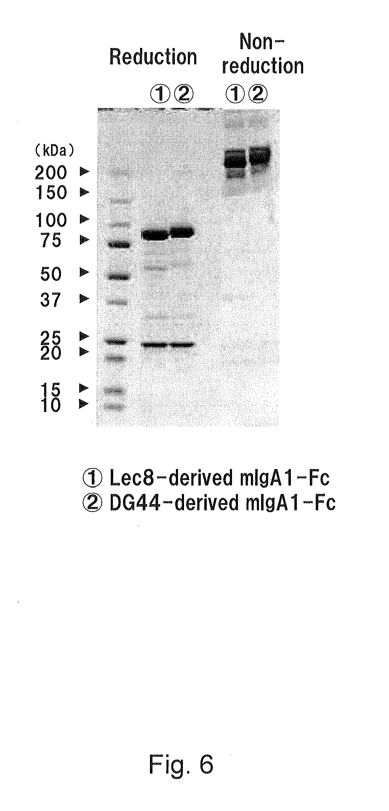

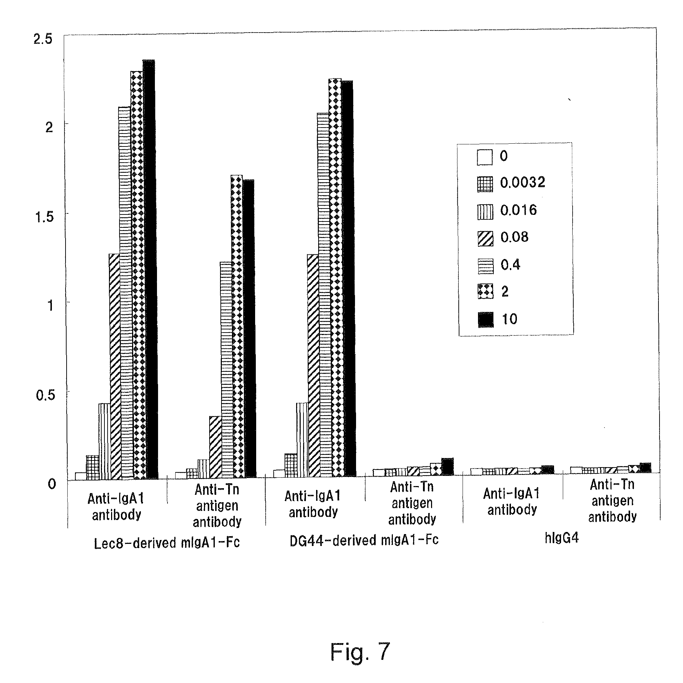

In order to obtain a soluble mIgA1 protein, an Fc fusion protein mIgA1-Fc designed by ligating the extracellular region of mIgA1 to human IgG4Fc was designed. Specifically, a gene fragment obtained by ligating a portion of the extracellular region of mIgA1 to human IgG4Fc was prepared by PCR, and this fragment was inserted into pKAN932B8PVHmIgA obtained in Example 1, thereby preparing an Fc fusion mIgA1 expression vector pKANTEX-mIgA1-Fc. This expression vector was introduced into CHO/DG44 cell line and Lec8 cell line, and 500 µg/mL of G418 was added to the medium to select gene-introduced cells. The selected gene-introduced cells were cultured for a week in a serum-free medium Excell-302 (SAFC), thereby obtaining culture supernatant containing mIgA1-Fc. About 1 L of the culture supernatant was refined using a Mabselect (GE Healthcare) column in accordance with the instructions attached to the column, thereby obtaining about 5 mg of each of DG44-derived mIgA1-Fc and Lec8-derived mIgA1-Fc. Each of the obtained mIgA1-Fcs was analyzed with SDS-PAGE so as to investigate the molecular weight and the purification level (Fig. 6). In addition, for the purpose of confirming modified sugar chains of the purified protein, the following enzyme immunoassay was performed. Into a 96-well EIA plate (Greiner Bio-One), 1 µg/mL of the DG44-derived or Lec8-derived mIgA1-Fc or human IgG4 (Sigma-Aldrich Co. LLC.) was dispensed at 50 µL/well and left to stand overnight at 4°C for adsorption. After the plate was washed, 1% BSA-PBS was added thereto at 100 µL/well and reacted at room temperature for an hour, thereby blocking the remaining active groups. Subsequently, 1% BSA-PBS was discarded, and as a primary antibody, mouse anti-human mIgA1 monoclonal antibody B3506B4 (Beckman Coulter, Inc.) or mouse anti-Tn antigen antibody 22-1-1 (MBL International) diluted with PBS was dispensed at 50 µL/well, followed by reaction for two hours. The plate was washed with 0.05% tween-PBS, and then as a secondary antibody, diluted peroxidase-labeled anti-mouse immunoglobulin (DAKO) was added thereto at 50 µL/well, followed by reaction at room temperature for an hour. Thereafter, the plate was washed with 0.05% tween-PBS, and color development was performed using an ABTS [2,2-azinobis(3-ethylbenzothiazole-6-sulfonic acid)ammonium] substrate solution [1 mmol/L ABTS, 0.1 mol/L citric acid buffer (pH 4.2), 0.1% H₂O₂]. Subsequently, an absorbance (OD415-OD490) at a sample wavelength of 415 nm and a reference wavelength of 490 nm was measured using a plate reader (MULTISKAN SPECTRUM; Thermo Inc.). As a result, it was confirmed that while the anti-IgA1 antibody bound to the DG44-derived and the Lec8-derived mIgA1-Fc, the anti-Tn antigen antibody bound only to the Lec8-derived mIgA1-Fc (Fig. 7). From this result, it was confirmed that the Lec8-derived mIgA1-Fc had the Tn antigen type O-linked sugar chain.

### Example 3

### Preparation of monoclonal antibody against hinge region of Tn antigen-bound IgA1

### (1) Preparation of glycopeptides as immunogen

A peptide in which N-acetylgalactosamine (GalNAc) was added to serine (Ser) at positions 230 and 232 and to threonine (Thr) at positions 225, 228, and 236 of a human IgA1 (IgA1) hinge region amino acid sequence (amino acids from position 223 to position 240 counted from the amino terminal) by α-binding, and to which cysteine (Cys) was further added to the amino terminal so as to bind to a carrier protein was synthesized using an automatic synthesizer (Shimadzu Corporation) (Tn-bound IgA1 hinge peptide, SEQ ID NO:16). In order to enhance immunogenicity, a conjugate with KLH (Wako Pure Chemical Industries, Ltd.) was prepared in the following manner, thereby obtaining an immunogen. That is, KLH was dissolved in PBS such that the amount was adjusted to 10 mg/mL, and 25 mg/mL MBS [N-(m-Maleimidobenzoyloxy)-succinimide] (Nacalai Tesque, Inc.) with a 1/10 volume was added dropwise thereto, followed by a reaction for 30 minutes under stirring. Then, 2.5 mg of KLH-MB, which was obtained excluding free MBS by using a gel filtration column such as a Sephadex G-25 column pre-equilibrated in with PBS, and was mixed with 1 mg of the peptide dissolved in 0.1 M sodium phosphate buffer (pH 7.0), followed by reaction at room temperature for 3 hours under stirring. After the reaction, the resultant was dialyzed with PBS, thereby obtaining an immunogen.

### (2) Immunization of animal and preparation of antibody-producing cell

To a 4-week-old female SD rat (Japan SLC, Inc), 100 µg of the Tn antigen-bound IgA1 hinge peptide-KLH conjugate prepared in the manner described in the section (1), 2 mg of an aluminum gel, and 1×10⁹ cells of a pertussis vaccine (manufactured by Chiba Serum Institute) were administered. After 2 weeks, 100 µg of the conjugate was administered once a week so as to be administered 4 times in total. Blood was collected from the caudal vein, and the reactivity to the Tn antigen-bound human plasma-derived IgA1 was investigated by the following enzyme immunoassay. Three days after the final immunization, a spleen was extracted from the rat showing a sufficient antibody titer. The spleen was minced into small pieces in a MEM medium (NISSUI PHARMACEUTICAL CO., LTD.), loosened with a pair of tweezers, and centrifuged (1200 rpm, 5 minutes). Thereafter, the supernatant was discarded. The resultant was treated with tris-ammonium chloride buffer (pH 7.65) for 1 to 2 minutes, thereby removing erythrocytes. The resultant was washed 3 times with a MEM medium and used for cell fusion.

### (3) Enzyme immunoassay

As a negative control antigen for assay, human plasma-derived IgA1 (BIOPUR) was used, and as a positive control antigen, Tn antigen type human IgA was used. In order to obtain the Tn antigen type human IgA1, 5 U/mL of β-Galactosidase (GKX-5013, ProZyme, Inc.) and 1 U/mL of Neuraminidase (24229-61, Nacalai Tesque, Inc.) were allowed to act on the human plasma-derived IgA1 at 37°C over night, and normal sugar chains were converted into Tn antigens. In addition, for the purpose of removing antibodies which recognize only Tn antigens, a Tn antigen type C 1 inhibitor that was obtained by treating a human plasma-derived C 1 inhibitor protein (ZLB Behring, product name Berinert) with the enzymes similarly to IgA1 was also used as a negative control antigen. Into a 96-well EIA plate (Greiner), 2.5 µg/mL each of the antigens were dispensed at 50 µL/well and left to stand overnight at 4°C for adsorption. After the plate was washed, 1% BSA-PBS was added thereto at 100 µL/well and reacted at room temperature for an hour, thereby blocking the remaining active groups. Subsequently, 1% BSA-PBS was discarded, and as a primary antibody, culture supernatant of a hybridoma or immunized rat antiserum was dispensed at 50 µL/well, followed by reaction for two hours. The plate was washed with 0.05% tween-PBS, and then as a secondary antibody, diluted peroxidase-labeled anti-rat immunoglobulin (DAKO) was added thereto at 50 µL/well, followed by a reaction at room temperature for an hour. Thereafter, the plate was washed with 0.05% tween-PBS, and then color development was performed using an ABTS [2,2-azinobis(3-ethylbenzothiazole-6-sulfonic acid) ammonium] substrate solution [1 mmol/L ABTS, 0.1 mol/L citric acid buffer (pH 4.2), 0.1% H₂O₂]. Subsequently, an absorbance (OD415-OD490) at a sample wavelength of 415 nm and a reference wavelength of 490 nm was measured using a plate reader (Emax; Molecular Devices, LLC.).

### (4) Preparation of mouse myeloma cell

An 8-azaguanine-resistant mouse myeloma cell line P3-U1 was cultured in a normal medium, and 2×10⁷ or more of cells were secured at the time of cell fusion and subjected to cell fusion as a parent line.

### (5) Preparation of hybridoma

The rat spleen cells obtained in the section (2) were mixed with the myeloma cells obtained in the section (4) at a mixing ratio of 10:1, followed by centrifugation (1200 rpm for 5 minutes). The supernatant was then discarded, a cell clumps precipitated were thoroughly loosened, and then a mixed solution containing 1 g of polyethylene glycol 1000 (PEG-1000), 1 mL of a MEM medium, and 0.35 mL of dimethyl sulfoxide was added thereto at 37 °C under stirring, in an amount of 0.5 mL/10⁸ mouse spleen cells. To the suspension, 1 mL of the MEM medium was added several times at every 1 to 2 minutes, and then the MEM medium was added thereto so as to yield a total amount of 50 mL. After centrifugation (900 rpm for 5 minutes), the supernatant was discarded, and then the cells were loosened gently. Subsequently, the cells were gently suspended in 100 ml of a HAT medium [a medium prepared by adding HAT Media Supplement (Invitrogen) to 10% fetal bovine serum-added RPMI medium] by being drawn up into and discharged from a measuring pipette. The suspension was dispensed at 200 µl/well into a 96-well culture plate and cultured in a 5% CO₂ incubator at 37°C for 10 to 14 days under a level of 5% CO₂. The culture supernatant was analyzed with enzyme immunoassay described in the section (3), wells reactive specifically to the Tn antigen type human IgA1 were selected, and cloning was repeated twice. In this manner, hybridoma lines KM4137, 4138, 4139, 4140, and 4144 producing anti-Tn antigen-bound mIgA hinge peptide monoclonal antibodies were established. Antibody classes were analyzed by enzyme immunoassay using subclass-specific secondary antibodies, whereby KM4137, KM4138, KM4139, and KM4144 were identified to be rat IgG2a, and KM4140 was identified to be rat IgG2b. These antibody classes are shown in Table. 2.

**[Table 2]**

| KM No. | Animal spices | Antibody class |
|---|---|---|
| KM4137 | Rat | IgG2a |
| KM4138 | Rat | IgG2a |
| KM4139 | Rat | IgG2a |
| KM4140 | Rat | IgG2b |
| KM4144 | Rat | IgG2a |

An experiment of immunizing a Balb/c mouse and a BxSB lupus mouse with the glycopeptide-KLH fusion protein prepared in the section (1) was performed a plurality of times. However, a monoclonal antibody specific to the Tn antigen type hinge region failed to be obtained. In addition, an experiment of immunizing an SD rat and a Balb/c mouse with the CHO cell line (mIgA/Lec8) that was established in Example 1 and highly expressed the Tn antigen type IgA1 on the cell membrane was performed a plurality of times. However, a monoclonal antibody specific to the Tn antigen type hinge region failed to be obtained. Moreover, an experiment of immunizing an SD rat and a Balb/c mouse with the Tn antigen type IgA1-Fc fusion protein prepared in Example 2 was also performed a plurality of times. However, a monoclonal antibody specific to the Tn antigen type hinge region failed to be obtained.

### (6) Purification of monoclonal antibody

Each of the hybridoma lines obtained in Section (5) was administered by intraperitoneal injection into 8-week-old female hairless mice (Balb/c) treated with pristine, at a dose of 5 to 20×10⁶ cells/mouse. Ten to 21 days thereafter, the hybridoma developed into ascites cancer. Acites was collected (1 to 8 mL/mouse) from the mice with ascites buildup and centrifuged (3000 rpm for 5 minutes) to remove solid contents. Thereafter, the ascites was refined by caprylic acid precipitation (Antibodies-A Laboratory Manual, Cold Spring Harbor Laboratory (1988)), thereby obtaining a purified monoclonal antibody.

### Example 4

Reactivity of anti-Tn antigen-bound mIgA hinge peptide monoclonal antibody

### (1) Binding ELISA

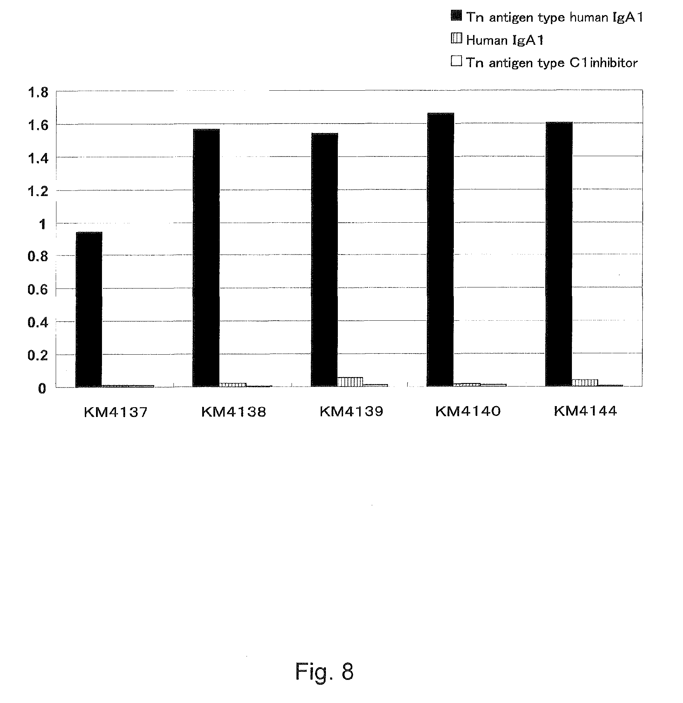

Reaction specificity of the obtained monoclonal antibody was investigated by the enzyme immunoassay described in the section (3) of Example 3. KM4137 to 4140 and KM4144 all showed reactivity specifically to the Tn antigen type human IgA1 but did not react with the human IgA1 or the Tn antigen type C1 inhibitor (Fig. 8).

### (2) Competitive enzyme immunoassay

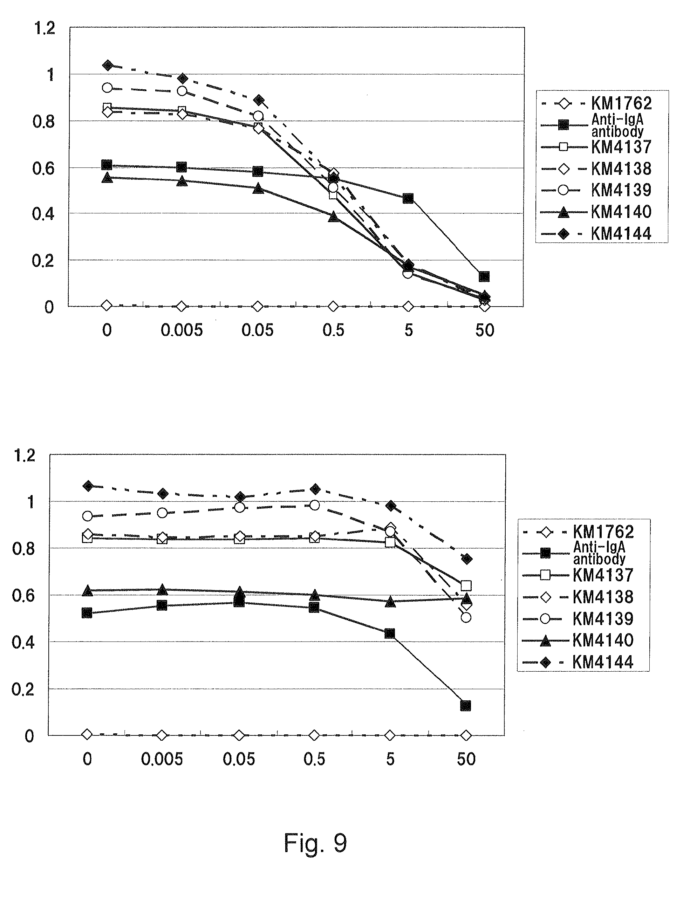

In order to evaluate binding specificity of the established monoclonal antibody, the following competitive enzyme immunoassay was performed. Into a 96-well EIA plate (Greiner), 2.5 µg/mL of the Tn antigen type human IgA1 prepared in the section (3) of Example 3 was dispensed at 50 µL/well and left to stand overnight at 4°C for adsorption. After the plate was washed, 1% BSA-PBS was added thereto at 100 µL/well and reacted at room temperature for an hour, thereby blocking the remaining active groups, Subsequently, 1% BSA-PBS was discarded, and as a competitive substance, the Tn antigen type human IgA1 or the human plasma-derived IgA1 was dispensed at 25 µL/well. In addition, as a primary antibody, the culture supernatant of the respective hybridomas established in Example 3 was prepared in an antibody concentration of about 1 µg/mL, dispensed at 25 µL/well, and allowed to react for 2 hours. The plate was washed with 0.05% tween-PBS, and then as a secondary antibody, diluted peroxidase-labeled anti-rat immunoglobulin (DAKO) was added thereto at 50 µL/well, followed by reaction at room temperature for an hour. Thereafter, the plate was washed with 0.05% tween-PBS, and color development was performed using an ABTS [2,2-azinobis(3-ethylbenzothiazole-6-sulfonic acid)ammonium] substrate solution [1 mmol/L ABTS, 0.1 mol/L citric acid buffer (pH 4.2), 0.1% hydrogen peroxide solution]. Subsequently, an absorbance (OD415-OD490) at a sample wavelength of 415 nm and a reference wavelength of 490 nm was measured using a plate reader (MULTISKAN SPECTRUM; Thermo Inc.). As a negative control antibody, a rat anti-Avermectin antibody KM1762 was used, and as a positive control, a rat anti-human IgA monoclonal antibody LO-HA-9 (P.A.R.I.S Anticorps) was used. As a result, it was clarified that when the Tn antigen type human IgA1 was used as a competitive substance, the established monoclonal antibody was absorbed depending on the concentration of the competitive substance and also absorbed at a concentration of the competitive substance that was 100 to 1000-fold lower, compared to a case where the human plasma-derived IgA1 was used as a competitive substance (Fig. 9). This result showed that all of the established monoclonal antibodies showed high binding specificity with respect to the Tn antigen type human IgA1.

### (3) Measurement of binding activity with respect to Tn antigen type human IgA1 or to human plasma-derived IgA1

Binding activity was measured with surface plasmon resonance. All of the following operations were performed using BiacoreT-100 (Biacore). An anti-mouse immunoglobulin antibody (Biacore) was immobilized on a CM5 sensor chip (Biacore) by amine coupling, and the culture supernatant of the respective hybridomas of which the antibody concentration was known was caused to flow on the chip so as to cause the respective antibodies to be captured. Thereafter, the Tn antigen type human IgA1 or the human plasma-derived IgA1 that was diluted with HBS-EP+buffer (Biacore) by 6 stages from 2µg/mL was caused to flow on the chip at a rate of 30 µL/ min, and a sensorgram in each concentration was analyzed. In this manner, an association rate constant and a dissociation rate constant of the respective antibodies with respect to the Tn antigen type human IgA1 were calculated using kinetics analysis (1:1 association model). The internal temperature of the Biacore-T100 was set to 25°C, and for each binding of the antibody, the chip was restored with a glycine buffer (Biacore) having pH of 1.5. As a result, all of the monoclonal antibodies showed affinity with a dissociation constant of about 10⁻⁸[M] with respect to the Tn antigen type human IgA1, but the binding of the antibodies to the human plasma-derived IgA1 were not detected (Table 3). This result indicated that all of the established monoclonal antibodies showed high binding specificity and affinity with the Tn antigen type human IgA1.

**[Table 3]**

| | Kₐ[1/Ms] | K_{d}[1/s] | K_{D}[M] |
|---|---|---|---|
| KM4137 | 4.07×10⁴ | 7.13×10⁻⁴ | 1.75×10⁻⁸ |
| KM4138 | 3.83×10³ | 7.65×10⁻⁴ | 2.00×10⁻⁷ |
| KM4139 | 4.21×10⁴ | 6.21×10⁻⁴ | 1.47×10⁻⁸ |
| KM4140 | 1.68×10⁴ | 1.24×10⁻³ | 7.40×10⁻⁸ |
| KM4144 | 4.36×10⁴ | 8.87×10⁻⁴ | 2.03×10⁻⁸ |

### (4) Evaluation of reactivity with mIgA1-expressing transfectant

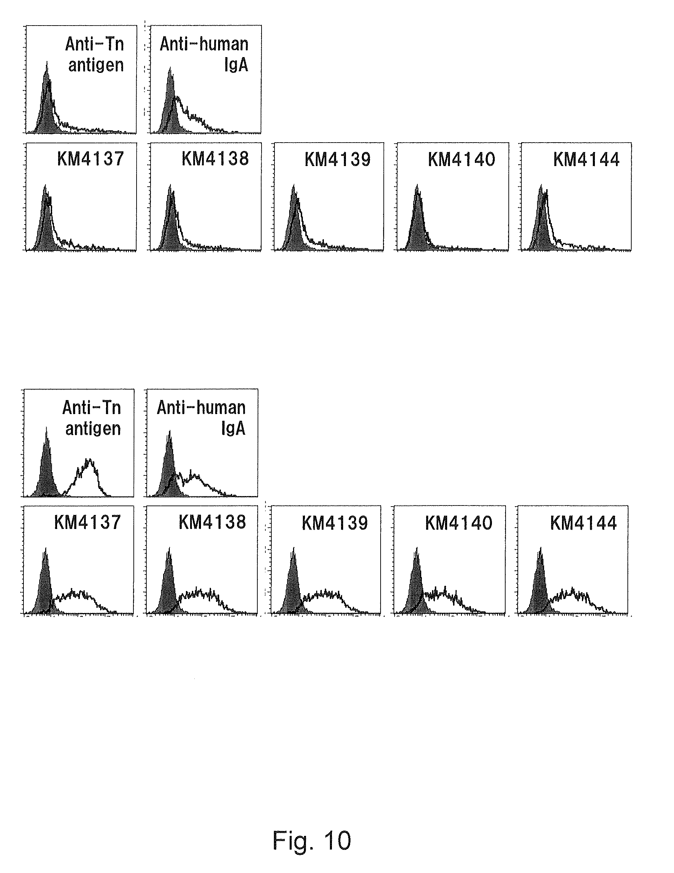

In 50 µL of culture supernatant of the established respective hybridomas, 5×10⁵ cells of the mIgA-expressing DG44 cell line and the mIgA1-expressing Lec8 cell line established in Example (1) were suspended and allowed to react at 4°C for an hour. After the reaction, the cells were washed by centrifugation three times by using PBS containing 0.05% NaN₃, and then a solution prepared by diluting an Alexa488-labeled goat anti-rat IgG (H+L) antibody (Invitrogen) or an Alexa488-labeled goat anti-mouse IgG (H+L) antibody (Invitrogen) with 1% BSA-PBS at 300-fold was added thereto to suspend the cells, followed by reaction at 4°C for an hour. After the reaction, the cells were washed by being centrifuged three times by using PBS containing 0.05% NaN₃, suspended in PBS containing 500 µL of 0.05% NaN₃, and analyzed using a flow cytometer FACS Calibur (BD Biosciences). As a negative control antibody, a mouse anti-ND28 monoclonal antibody KM511 (JP-A-08-165300) or a rat anti-Avermectin monoclonal antibody KM1762 (Clin Cancer Res. 2005 May 1; 11(9); 3494-502) was used, and as a positive control, a mouse anti-Tn antigen monoclonal antibody 22-1-1 (MBL) or a rat anti-human IgA monoclonal antibody LO-HA-9 (P.A.R.I.S) was used. As a result, it was confirmed that all of the established monoclonal antibodies did not bind to the mIgA1-expressing DG44 cell but bound specifically to the mIgA1-expressing Lec8 cell (Fig. 10). The mIgA1-expressing Lec8 cell expressed the Tn antigen type mIgA1, suggesting that the established monoclonal antibody could recognize the Tn antigen type mIgA1 existing on the cell surface.

### (5) Quantitation of Tn antigen type IgA1 in human serum

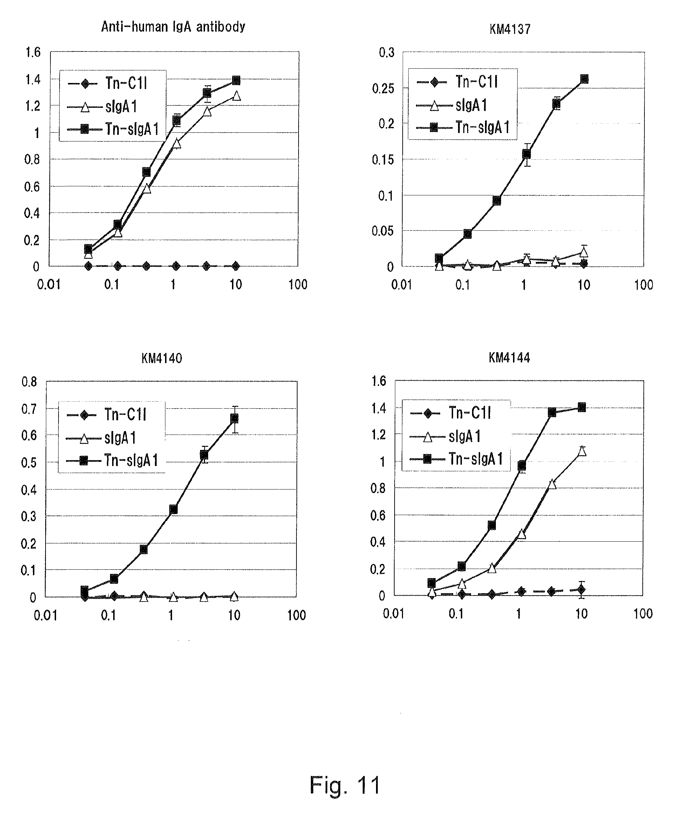

In order to construct a detection system for sugar chain-deficient IgA1 in human serum, the following enzyme immunoassay was performed. As a test substance, a substance obtained by diluting the Tn antigen type human IgA1 or the human plasma-derived IgA1 with human serum (Sigma-Aldrich Co. LLC.) was used. x µg/mL of KM4137, KM4140, and KM 4144 purified in Example 3 and a rat anti-human IgA monoclonal antibody LO-HA-9 (P.A.R.I.S) were dispensed at 50 µL/well and left to stand overnight at 4°C for adsorption. After the plate was washed, 1% BSA-PBS was added thereto at 100 µL/well and reacted at room temperature for an hour, thereby blocking the remaining active groups. Subsequently, 1% BSA-PBS was discarded, and the test substance diluted with PBS was dispensed at 50 µL/well and reacted for an hour. The plate was washed with 0.05% tween-PBS, and then as a secondary antibody, a diluted peroxidase-labeled mouse anti-human IgA1 monoclonal antibody B3506B4 (Beckman Coulter, Inc.) was added thereto at 50 µL/well, followed by a reaction at room temperature for an hour. Thereafter, the plate was washed with 0.05% tween-PBS, and color development was performed using an ABTS [2,2-azinobis(3-ethylbenzothiazole-6-sulfonic acid)ammonium] substrate solution [1 mmol/L ABTS, 0.1 mol/L citric acid buffer (pH 4.2), 0.1% hydrogen peroxide solution]. Subsequently, an absorbance (OD415-OD490) at a sample wavelength of 415 nm and a reference wavelength of 490 nm was measured using a plate reader (MULTISKAN SPECTRUM; Thermo Inc.). As a result, in the plate onto which the anti-human IgA monoclonal antibody was absorbed, both the Tn antigen type human IgA1 and the human plasma-derived IgA1 were detected. On the other hand, in the plate onto which the established KM4137, KM4140, and KM4144 were adsorbed, only the Tn antigen type human IgA1 was detected depending on the concentration of the antigen added (Fig. 11). This result showed that it is possible to specifically detect and quantitate only the sugar chain-deficient IgA1 by enzyme immunoassay using KM4137, KM4140, and KM4144.

### EXAMPLE 5

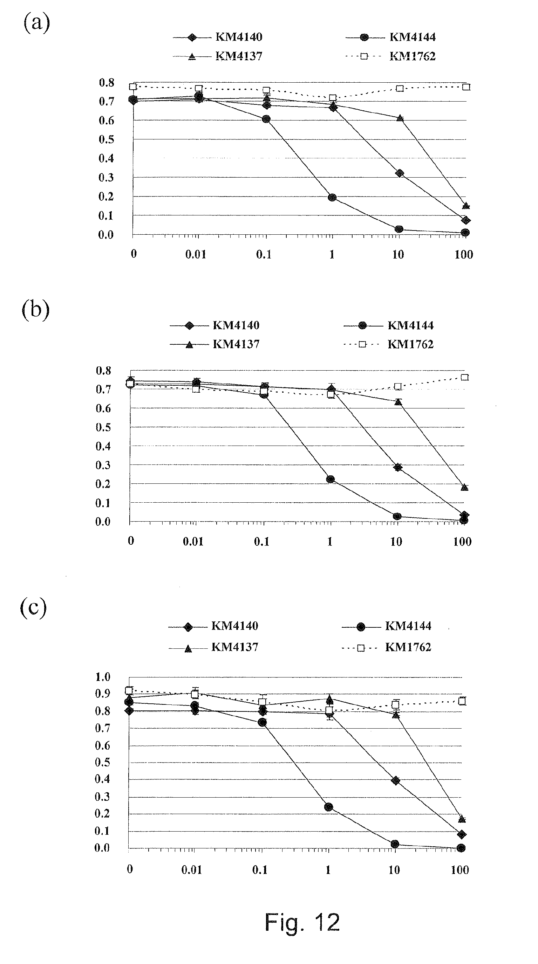

In order to evaluate the competitive inhibition activity of the established anti-Tn antigen-bound mIgA hinge peptide monoclonal antibodies KM4137, KM4140, and KM4144, the following competitive enzyme immunoassay was performed. In a 96-well Nunc Maxisorp plate (Nunc), 25 µg/mL of the Tn antigen type human IgA1 prepared in the section (3) of Example 3 was dispensed at 50 µL/well and left to stand overnight at 4°C for adsorption. After the plate was washed, 1% BSA-PBS was added thereto at 200 µL/well and reacted at room temperature for an hour, thereby blocking the remaining active groups. Subsequently, 1% BSA-PBS was discarded, and as a competitive substance, the anti-Tn antigen-bound mIgA hinge peptide monoclonal antibodies KM4137, KM4140, and KM4144 purified in Example 3 were dispensed at 25µL/well. In addition, as primary antibodies, biotin-labeled KM4137, biotin-labeled KM4140, and biotin-labeled KM4144, which were obtained by labeling the anti-Tn antigen-bound mIgA hinge peptide monoclonal antibodies purified in Example 3 with biotin, were prepared in amounts of 2 µg/mL, 0.16 µg/mL, and 10 µg/mL respectively, and dispensed at 25 µL/well and allowed to react for an hour. The plate was washed with 0.05% tween-PBS, and then as a secondary antibody, diluted peroxidase-labeled streptavidin (Sigma-Aldrich Co. LLC.) was added thereto at 50 µL/well and reacted at room temperature for an hour. Thereafter, the plate was washed with 0.05% tween-PBS, and color development was performed using an ABTS [2,2-azinobis(3-ethylbenzothiazole-6-sulfonic acid) ammonium] substrate solution [1 mmol/L ABTS, 0.1 mol/L citric acid buffer (pH 4.2), 0.1% hydrogen peroxide solution]. Subsequently, an absorbance (OD415-OD490) at a sample wavelength of 415 nm and a reference wavelength of 490 nm was measured using a plate reader (MULTISKAN SPECTRUM; Thermo Inc.). As a negative control antibody, a rat anti-Avermectin antibody KM1762 was used. As a result, it was found that the established anti-Tn antigen-added mIgA hinge peptide monoclonal antibodies KM4137, KM4140, and KM4144 competed with each other in the Tn antigen-bound mIgA hinge region (Fig. 12).

### Industrial Applicability

According to the present invention, it is possible to provide a monoclonal antibody or an antibody fragment thereof that specifically recognizes and binds to a hinge region of a polypeptide encoded by a heavy chain gene of immunoglobulin A1 comprising a serine/threonine-linked sugar chain to which galactose is not bound. It is also possible to provide a diagnostic agent using the antibody or the antibody fragment thereof and to provide a therapeutic agent containing the antibody or the antibody fragment thereof as an active ingredient.

### Deposit Number

IPOD FERM BP-11214
IPOD FERM BP-11215
IPOD FERM BP-11216

### Free Text of Sequence Listing

SEQ ID NO:1 human IgA1 hinge region amino acid sequence
SEQ ID NO:2 human IgA1 heavy chain amino acid sequence
SEQ ID NO:3 human IgA1 hinge region DNA sequence
SEQ ID NO:4 description of artificial sequence: RitNotNheIfw
SEQ ID NO:5 description of artificial sequence: RitNotNheIrv
SEQ ID NO:6 description of artificial sequence: pCR2B8PVH
SEQ ID NO:7 description of artificial sequence: Ig-a-NheI
SEQ ID NO:8 description of artificial sequence: Ig-b-BamHI
SEQ ID NO:9 description of artificial sequence: pCRIgA
SEQ ID NO:10 description of artificial sequence: AMD-A
SEQ ID NO:11 description of artificial sequence: AMD-A
SEQ ID NO:12 description of artificial sequence: AMDBamHIfw
SEQ ID NO:13 description of artificial sequence: AMDSpeIrv
SEQ ID NO:14 description of artificial sequence: pCRmIgA
SEQ ID NO:15 description of artificial sequence: membrane-bound IgA1 sequence
SEQ ID NO:16 Tn-added IgA1 hinge peptide amino acid sequence

## Claims

1. A monoclonal antibody or an antibody fragment thereof which specifically recognizes and binds to a hinge region of a polypeptide encoded by a heavy chain gene of immunoglobulin A1 (hereinbelow, referred to as an IgA1 heavy chain) comprising a serine/threonine-linked sugar chain (hereinbelow, referred to as an O-linked sugar chain) to which galactose is not bound.

2. A monoclonal antibody or an antibody fragment thereof which does not recognize a hinge region of an IgA1 heavy chain comprising an O-linked sugar chain to which galactose is bound, but recognizes and binds to a hinge region of an IgA1 heavy chain comprising the O-linked sugar chain to which galactose is not bound, among hinge regions of a polypeptide encoded by an IgA1 heavy chain gene to which the O-linked sugar chain is bound.

3. The monoclonal antibody or the antibody fragment thereof according to Claim 1 or 2, wherein the O-linked sugar chain to which galactose is not bound is at least one O-linked sugar chain selected from α-N-acetylgalactosamine-serine/threonine (hereinbelow, referred to as a Tn antigen) and a sialyl Tn antigen.

4. The monoclonal antibody or the antibody fragment thereof according to Claim 3, wherein the O-linked sugar chain to which galactose is not bound is the Tn antigen.

5. The monoclonal antibody or the antibody fragment thereof according to any one of Claims 1 to 4, wherein the monoclonal antibody is an antibody which specifically recognizes and binds to the hinge region of the IgA1 heavy chain comprising the amino acid sequence represented by SEQ ID NO:1.

6. The monoclonal antibody or the antibody fragment thereof according to any one of Claims 1 to 4, wherein the monoclonal antibody is an antibody which specifically recognizes and binds to a polypeptide which is a hinge region polypeptide of the IgA1 heavy chain comprising the amino acid sequence represented by SEQ ID NO:1 and is a glycopeptide to which N-acetylgalactosamine not having galactose is bound at least one amino acid residue selected from threonine at position 3, threonine at position 6, serine at position 8, serine at position 10, and threonine at position 14 from an amino terminal of the polypeptide.

7. The monoclonal antibody or the antibody fragment thereof according to any one of Claims 1 to 4, wherein the monoclonal antibody is an antibody which does not show cross-reactivity to a complement C1 inhibitor comprising the O-linked sugar chain to which galactose is not bound.

8. The monoclonal antibody or the antibody fragment thereof according to any one of Claims 1 to 4, wherein the monoclonal antibody is an antibody which competes with at least one monoclonal antibody selected from monoclonal antibodies KM4137, KM4140, and KM4144 when binding to the hinge region of the IgA1 heavy chain comprising the O-linked sugar chain to which galactose is not bound.

9. The monoclonal antibody or the antibody fragment thereof according to any one of Claims 1 to 4, wherein the monoclonal antibody is an antibody which binds to an epitope to which at least one monoclonal antibody selected from monoclonal antibodies KM4137, KM4140, and KM4144 binds and which presents in the hinge region of the IgA1 heavy chain comprising the O-linked sugar chain to which galactose is not bound.

10. The antibody or the antibody fragment thereof according to any one of Claims 1 to 9, wherein the monoclonal antibody is an antibody which produced from at least one hybridoma selected from hybridomas KM4137 (FERM BP-11214), KM4140 (FERM BP-11215), and KM4144 (FERM BP-11216).

11. The antibody or the antibody fragment thereof according to any one of Claims 1 to 9, wherein the monoclonal antibody is a recombinant antibody.

12. The recombinant antibody or an antibody fragment thereof according to Claim 11, wherein the recombinant antibody is an antibody selected from a human chimeric antibody, a humanized antibody, and a human antibody.

13. The antibody fragment according to any one of Claims 1 to 12, which is selected from Fab, Fab', F(ab')₂, a single chain antibody (scFv), a dimerized V region (diabody), a disulfide stabilized V region (dsFv), and a CDR-containing peptide.

14. A hybridoma producing the monoclonal antibody according to any one of Claims 1 to 9.

15. A DNA encoding the antibody or the antibody fragment thereof according to any one of Claims 1 to 13.

16. A recombinant vector comprising the DNA according to Claim 15.

17. A transformant obtained by introducing the recombinant vector according to Claim 16 into a host cell.

18. A method of producing the antibody or the antibody fragment thereof according to any one of Claims 1 to 13, the method comprising:
culturing the hybridoma according to Claim 14 or the transformant according to Claim 17 in a medium so as to form and accumulate the antibody or the antibody fragment thereof according to any one of Claims 1 to 13 in the culture; and
collecting the antibody or the antibody fragment thereof from the culture.

19. A method of immunologically detecting or measuring IgA1 having a hinge region comprising an O-linked sugar chain to which galactose is not bound, which comprises using the antibody or the antibody fragment thereof according to any one of Claims 1 to 13.

20. A reagent for detecting IgA1 having a hinge region comprising an O-linked sugar chain to which galactose is not bound, which is a reagent using the antibody or the antibody fragment thereof according to any one of Claims 1 to 13.

21. A diagnostic agent for a disease relating to IgA1 having a hinge region comprising an O-linked sugar chain to which galactose is not bound, wherein the diagnostic agent uses the antibody or the antibody fragment thereof according to any one of Claims 1 to 13.

22. The diagnostic agent according to Claim 21, wherein the disease relating to the IgA1 having the hinge region comprising the O-linked sugar chain to which galactose is not bound is an autoimmune disease.

23. The diagnostic agent according to Claim 21, wherein the disease relating to the IgA1 having the hinge region comprising the O-linked sugar chain to which galactose is not bound is IgA nephropathy.

24. A therapeutic agent for a disease relating to IgA1 having a hinge region comprising an O-linked sugar chain to which galactose is not bound, wherein the therapeutic agent contains the antibody or the antibody fragment thereof according to any one of Claims 1 to 13 as an active ingredient.

25. The therapeutic agent according to Claim 24, wherein the disease relating to the IgA1 having the hinge region comprising the O-linked sugar chain to which galactose is not bound is an autoimmune disease.

26. The therapeutic agent according to Claim 24, wherein the disease relating to the IgG1 having the hinge region comprising the O-linked sugar chain to which galactose is not bound is IgA nephropathy.

27. A diagnostic method for a disease relating to IgA1 having a hinge region comprising an O-linked sugar chain to which galactose is not bound, the method comprising:
detecting and measuring the IgA1 having the hinge region comprising the O-linked sugar chain to which galactose is not bound, by using the antibody or the antibody fragment thereof according to any one of Claims 1 to 13.

28. The diagnostic method according to Claim 27, wherein the disease relating to IgA1 having the hinge region comprising the O-linked sugar chain to which galactose is not bound is an autoimmune disease.

29. The diagnostic method according to Claim 27, wherein the disease relating to IgA1 having the hinge region comprising the O-linked sugar chain to which galactose is not bound is IgA nephropathy.

30. Use of the antibody or the antibody fragment thereof according to any one of Claims 1 to 13 for producing a therapeutic agent for a disease relating to IgA1 having a hinge region comprising an O-linked sugar chain to which galactose is not bound.

31. The use of the antibody or the antibody fragment thereof according to Claim 30, wherein the disease relating to IgA1 having the hinge region comprising the O-linked sugar chain to which galactose is not bound is an autoimmune disease.

32. The use of the antibody or the antibody fragment thereof according to Claim 30, wherein the disease relating to IgA1 having the hinge region comprising the O-linked sugar chain to which galactose is not bound is IgA nephropathy.


SEQUENCE LISTING

<110>  Kyowa Hakko Kirin Co., Ltd.

<120>  Anti-IgA1 antibody

<130>  1000P12120

<150>  JP 2009-296706
<151>  2009-12-28

<160>  16

<170>  PatentIn version 3.3

<210>  1
<211>  18
<212>  PRT
<213>  Homo sapiens

<400>  1

Pro Ser Thr Pro Pro Thr Pro Ser Pro Ser Thr Pro Pro Thr Pro Ser
1               5                   10                  15

Pro Ser

<210>  2
<211>  472
<212>  PRT
<213>  Homo sapiens

<400>  2

Glu Ser Gly Pro Gly Leu Val Lys Ser Ser Glu Thr Leu Ser Leu Thr
1               5                   10                  15

Cys Thr Val Ser Gly Gly Ser Ile Ser Ser Ser Ser Tyr Tyr Trp Gly
            20                  25                  30

Trp Ile Arg Gln Pro Pro Gly Lys Gly Leu Glu Trp Ile Ala Asn Thr
        35                  40                  45

Tyr Tyr Ser Gly Ile Thr Tyr Tyr Asn Pro Ser Leu Lys Ser Arg Val
    50                  55                  60

Thr Ile Ser Val Asp Thr Ser Lys Asn Gln Leu Ser Leu Lys Val Arg
65                  70                  75                  80

Ser Val Thr Ala Ala Asp Thr Ala Val Tyr Phe Cys Ala Arg His Gly
                85                  90                  95

Tyr Ser Arg Ser Gly Arg Thr Gly Ala Ile Asp Tyr Trp Gly Gln Gly
            100                 105                 110

Thr Leu Val Thr Val Ser Ser Ala Ser Pro Thr Ser Pro Lys Val Phe
        115                 120                 125

Pro Leu Ser Leu Cys Ser Thr Gln Pro Asp Gly Asn Val Val Ile Ala
    130                 135                 140

Cys Leu Val Gln Gly Phe Phe Pro Gln Glu Pro Leu Ser Val Thr Trp
145                 150                 155                 160

Ser Glu Ser Gly Gln Gly Val Thr Ala Arg Asn Phe Pro Pro Ser Gln
                165                 170                 175

Asp Ala Ser Gly Asp Leu Tyr Thr Thr Ser Ser Gln Leu Thr Leu Pro
            180                 185                 190

Ala Thr Gln Cys Leu Ala Gly Lys Ser Val Thr Cys His Val Lys His
        195                 200                 205

Tyr Thr Asn Pro Ser Gln Asp Val Thr Val Pro Cys Pro Val Pro Ser
    210                 215                 220

Thr Pro Pro Thr Pro Ser Pro Ser Thr Pro Pro Thr Pro Ser Pro Ser
225                 230                 235                 240

Cys Cys His Pro Arg Leu Ser Leu His Arg Pro Ala Leu Glu Asp Leu
                245                 250                 255

Leu Leu Gly Ser Glu Ala Asn Leu Thr Cys Thr Leu Thr Gly Leu Arg
            260                 265                 270

Asp Ala Ser Gly Val Thr Phe Thr Trp Thr Pro Ser Ser Gly Lys Ser
        275                 280                 285

Ala Val Gln Gly Pro Pro Glu Arg Asp Leu Cys Gly Cys Tyr Ser Val
    290                 295                 300

Ser Ser Val Leu Pro Gly Cys Ala Glu Pro Trp Asn His Gly Lys Thr
305                 310                 315                 320

Phe Thr Cys Thr Ala Ala Tyr Pro Glu Ser Lys Thr Pro Leu Thr Ala
                325                 330                 335

Thr Leu Ser Lys Ser Gly Asn Thr Phe Arg Pro Glu Val His Leu Leu
            340                 345                 350

Pro Pro Pro Ser Glu Glu Leu Ala Leu Asn Glu Leu Val Thr Leu Thr
        355                 360                 365

Cys Leu Ala Arg Gly Phe Ser Pro Lys Asp Val Leu Val Arg Trp Leu

370                 375                 380

Gln Gly Ser Gln Glu Leu Pro Arg Glu Lys Tyr Leu Thr Trp Ala Ser
385                 390                 395                 400

Arg Gln Glu Pro Ser Gln Gly Thr Thr Thr Phe Ala Val Thr Ser Ile
                405                 410                 415

Leu Arg Val Ala Ala Glu Asp Trp Lys Lys Gly Asp Thr Phe Ser Cys
            420                 425                 430

Met Val Gly His Glu Ala Leu Pro Leu Ala Phe Thr Gln Lys Thr Ile
        435                 440                 445

Asp Arg Leu Ala Gly Lys Pro Thr His Val Asn Val Ser Val Val Met
    450                 455                 460

Ala Glu Val Asp Gly Thr Cys Tyr
465                 470

<210>  3
<211>  1503
<212>  DNA
<213>  Homo sapiens

<400>  3
atgtgcaaga aaatgaagca cctgtggttc ttcctcctgc tggtggcggc tcccagatgg      60

gtcctgtccc agctgcagct gcaggagtcg ggcccaggac tggtgaagtc ttcggagacc     120

ctgtccctca cctgcactgt ctctggtggc tccatcagca gtagtagtta ctactggggc     180

tggatccgcc agcccccagg gaagggactg gagtggattg caaataccta ttatagtgga     240

atcacctact acaacccatc cctcaagagt cgcgtcacca tatccgtaga cacgtccaag     300

aaccagttgt ccctgaaggt gaggtctgtg accgccgcag acacggctgt gtatttctgt     360

gcgaggcatg gctatagcag gtcggggcga actggggcga ttgactactg gggccaggga     420

accctggtca ccgtctcctc agcatccccg accagcccca aggtcttccc gctgagcctc     480

tgcagcaccc agccagatgg gaacgtggtc atcgcctgcc tggtccaggg cttcttcccc     540

caggagccac tcagtgtgac ctggagcgaa agcggacagg gcgtgaccgc cagaaacttc     600

ccacccagcc aggatgcctc cggggacctg tacaccacga gcagccagct gaccctgccg     660

gccacacagt gcctagccgg caagtccgtg acatgccacg tgaagcacta cacgaatccc     720

agccaggatg tgactgtgcc ctgcccagtt ccctcaactc cacctacccc atctccctca     780

actccaccta ccccatctcc ctcatgctgc cacccccgac tgtcactgca ccgaccggcc     840

ctcgaggacc tgctcttagg ttcagaagcg aacctcacgt gcacactgac cggcctgaga     900

gatgcctcag gtgtcacctt cacctggacg ccctcaagtg ggaagagcgc tgttcaagga     960

ccacctgagc gtgacctctg tggctgctac agcgtgtcca gtgtcctgcc gggctgtgcc 1020

gagccatgga accatgggaa gaccttcact tgcactgctg cctaccccga gtccaagacc 1080

ccgctaaccg ccaccctctc aaaatccgga aacacattcc ggcccgaggt ccacctgctg 1140

ccgccgccgt cggaggagct ggccctgaac gagctggtga cgctgacgtg cctggcacgc 1200

ggcttcagcc ccaaggacgt gctggttcgc tggctgcagg ggtcacagga gctgccccgc 1260

gagaagtacc tgacttgggc atcccggcag gagcccagcc agggcaccac caccttcgct 1320

gtgaccagca tactgcgcgt ggcagccgag gactggaaga agggggacac cttctcctgc 1380

atggtgggcc acgaggccct gccgctggcc ttcacacaga agaccatcga ccgcttggcg 1440

ggtaaaccca cccatgtcaa tgtgtctgtt gtcatggcgg aggtggacgg cacctgctac 1500

tga 1503

<210> 4
<211> 24
<212> DNA
<213> Artificial

<220>
<223> Synthetic DNA

<400> 4
gggcggccgc gacccctcac catg 24

<210> 5
<211> 28
<212> DNA
<213> Artificial

<220>
<223> Synthetic DNA

<400> 5
ggctagctgc agagacggtg accgtggt 28

<210> 6
<211> 444
<212> DNA
<213> Artificial

<220>
<223> Synthetic DNA

<400> 6
cggccgcgac ccctcaccat gggttggagc ctcatcttgc tcttccttgt cgctgttgct 60

acgcgtgtcc tgtcccaggt acaactgcag cagcctgggg ctgagctggt gaagcctggg 120

gcctcagtga agatgtcctg caaggcttct ggctacacat ttaccagtta caatatgcac 180

tgggtaaaac agacacctgg tcggggcctg gaatggattg gagctattta tcccggaaat 240

ggtgatactt cctacaatca gaagttcaaa ggcaaggcca cattgactgc agacaaatcc 300

tccagcacag cctacatgca gctcagcagc ctgacatctg aggactctgc ggtctattac 360

tgtgcaagat cgacttacta cggcggtgac tggtacttca atgtctgggg cgcagggacc 420

acggtcaccg tctctgcagc tagc 444

<210> 7
<211> 29
<212> DNA
<213> Artificial

<220>
<223> Synthetic DNA

<400> 7
gggctagccc gaccagcccc aaggtcttc 29

<210> 8
<211> 29
<212> DNA
<213> Artificial

<220>
<223> Synthetic DNA

<400> 8
ggggatcccg ccaagcggtc gatggtctt 29

<210> 9
<211> 1005
<212> DNA
<213> Artificial

<220>
<223> Synthetic DNA

<400> 9
gctagcccga ccagccccaa ggtcttcccg ctgagcctct gcagcaccca gccagatggg 60

aacgtggtca tcgcctgcct ggtccagggc ttcttccccc aggagccact cagtgtgacc 120

tggagcgaaa gcggacaggg cgtgaccgcc agaaacttcc cacccagcca ggatgcctcc 180

ggggacctgt acaccacgag cagccagctg accctgccgg ccacacagtg cctagccggc 240

aagtccgtga catgccacgt gaagcactac acgaatccca gccaggatgt gactgtgccc 300

tgcccagttc cctcaactcc acctacccca tctccctcaa ctccacctac cccatctccc 360

tcatgctgcc acccccgact gtcactgcac cgaccggccc tcgaggacct gctcttaggt 420

tcagaagcga acctcacgtg cacactgacc ggcctgagag atgcctcagg tgtcaccttc 480

acctggacgc cctcaagtgg gaagagcgct gttcaaggac cacctgaccg tgacctctgt 540

ggctgctaca gcgtgtccag tgtcctgccg ggctgtgccg agccatggaa ccatgggaag 600

accttcactt gcactgctgc ctaccccgag tccaagaccc cgctaaccgc caccctctca 660

aaatccggaa acacattccg gcccgaggtc cacctgctgc cgccgccgtc ggaggagctg 720

gccctgaacg agctggtgac gctgacgtgc ctggcacgtg gcttcagccc caaggatgtg 780

ctggttcgct ggctgcaggg gtcacaggag ctgccccgcg agaagtacct gacttgggca 840

tcccggcagg agcccagcca gggcaccacc accttcgctg tgaccagcat actgcgcgtg 900

gcagccgagg actggaagaa gggggacacc ttctcctgca tggtgggcca cgaggccctg 960

ccgctggcct tcacacagaa gaccatcgac cgcttggcgg gatcc 1005


<210> 10
<211> 110
<212> DNA
<213> Artificial

<220>
<223> Synthetic DNA

<400> 10
ctgttgcaga ttggcagatg ccgcctccct atgtggtgct ggacttgccg caggagaccc 60

tggaggagga gacccccggc gccaacctgt ggcccaccac catcaccttc 110


<210> 11
<211> 112
<212> DNA
<213> Artificial

<220>
<223> Synthetic DNA

<400> 11
tactgggggc cctccctgtt gccagatggg ccccggacgc tggtcacggt cagtgctgtg 60

ctatagaaca ggctcagcag gaagagggtg aggaaggtga tggtggtggg cc 112


<210> 12
<211> 32
<212> DNA
<213> Artificial

<220>
<223> Synthetic DNA

<400> 12
ggggatcctg ctctgttgca gattggcaga tg 32


<210> 13
<211> 33
<212> DNA
<213> Artificial

<220>
<223> Synthetic DNA

<400> 13
ccactagttc agtactgggg gccctccctg ttg 33


<210> 14
<211> 159
<212> DNA
<213> Artificial

<220>
<223>  Synthetic DNA

<400>  14
ggatcctccc ccaaaccctg gatttatgcc acatccaacc tggcttctgg agtccctgtt      60

cgcttcagtg gcagtgggtc tgggacttct tactctctca ccatcagcag agtggaggct     120

gaagatgctg ccacttatta ctgccagcag tggactagt                            159


<210>  15
<211>  1632
<212>  DNA
<213>  Artificial

<220>
<223>  Synthetic DNA

<400>  15
atgggttgga gcctcatctt gctcttcctt gtcgctgttg ctacgcgtgt cctgtcccag      60

gtacaactgc agcagcctgg ggctgagctg gtgaagcctg gggcctcagt gaagatgtcc     120

tgcaaggctt ctggctacac atttaccagt tacaatatgc actgggtaaa acagacacct     180

ggtcggggcc tggaatggat tggagctatt tatcccggaa atggtgatac ttcctacaat     240

cagaagttca aaggcaaggc cacattgact gcagacaaat cctccagcac agcctacatg     300

cagctcagca gcctgacatc tgaggactct gcggtctatt actgtgcaag atcgacttac     360

tacggcggtg actggtactt caatgtctgg ggcgcaggga ccacggtcac cgtctctgca     420

gctagcccga ccagccccaa ggtcttcccg ctgagcctct gcagcaccca gccagatggg     480

aacgtggtca tcgcctgcct ggtccagggc ttcttccccc aggagccact cagtgtgacc     540

tggagcgaaa gcggacaggg cgtgaccgcc agaaacttcc cacccagcca ggatgcctcc     600

ggggacctgt acaccacgag cagccagctg accctgccgg ccacacagtg cctagccggc     660

aagtccgtga catgccacgt gaagcactac acgaatccca gccaggatgt gactgtgccc     720

tgcccagttc cctcaactcc acctacccca tctccctcaa ctccacctac cccatctccc     780

tcatgctgcc acccccgact gtcactgcac cgaccggccc tcgaggacct gctcttaggt     840

tcagaagcga acctcacgtg cacactgacc ggcctgagag atgcctcagg tgtcaccttc     900

acctggacgc cctcaagtgg gaagagcgct gttcaaggac cacctgaccg tgacctctgt     960

ggctgctaca gcgtgtccag tgtcctgccg ggctgtgccg agccatggaa ccatgggaag    1020

accttcactt gcactgctgc ctaccccgag tccaagaccc cgctaaccgc caccctctca    1080

aaatccggaa acacattccg gcccgaggtc cacctgctgc cgccgccgtc ggaggagctg    1140

gccctgaacg agctggtgac gctgacgtgc ctggcacgtg gcttcagccc caaggatgtg    1200

ctggttcgct ggctgcaggg gtcacaggag ctgccccgcg agaagtacct gacttgggca    1260

tcccggcagg agcccagcca gggcaccacc accttcgctg tgaccagcat actgcgcgtg    1320

gcagccgagg actggaagaa gggggacacc ttctcctgca tggtgggcca cgaggccctg   1380

ccgctggcct tcacacagaa gaccatcgac cgcttggcgg gatcctgctc tgttgcagat   1440

tggcagatgc cgcctcccta tgtggtgctg gacttgccgc aggagaccct ggaggaggag   1500

acccccggcg ccaacctgtg gcccaccacc atcaccttcc tcaccctctt cctgctgagc   1560

ctgttctata gcacagcact gaccgtgacc agcgtccggg gcccatctgg caacagggag   1620

ggcccccagt ac                                                       1632


<210>  16
<211>  19
<212>  PRT
<213>  Artificial

<220>
<223>  Synthetic peptide

<400>  16

Cys Pro Ser Thr Pro Pro Thr Pro Ser Pro Ser Thr Pro Pro Thr Pro
1               5                   10                  15


Ser Pro Ser